# EUROPEAN PATENT SPECIFICATION

(11) **EP 1 635 821 B1**
(45) Date of publication and mention of the grant of the patent: **08.07.2009**
(21) Application number: 04751178.7
(22) Date of filing: 30.04.2004
(51) Int. Cl.: A61K 31/415, A61K 31/4155, C07D 231/40, A61P 29/00

(54) **4-BROMO-5-(2-CHLORO-BENZOYLAMINO)-1H-PYRAZOLE-3-CARBOXYLIC ACID (PHENYL)AMIDE DERIVATIVES AND RELATED COMPOUNDS AS BRADYKININ B1 RECEPTOR ANTAGONISTS FOR THE TREATMENT OF INFLAMMATORY DISEASES**
4-BROM-5-(2-CHLOR-BENZOYLAMINO)-1H-PYRAZOL-3-CARBONSÄURE(PHENYL)AMID-DERIVATE UND VERWANDTE VERBINDUNGEN ALS BRADYKININ B1 RECEPTOR ANTAGONISTEN ZUR BEHANDLUNG VON ENTZÜNDLICHEN ERKRANKUNGEN
DERIVES PHENYLAMIDE DU 4-BROMO-5-(3-CHLORO-BENZOYLAMINO)-1H-PYRAZOLE-3-ACIDE CARBOXYLIQUE ET COMPOSES SIMILAIRES POUR L'UTILISATION COMME ANTAGONISTS DU RECEPTEUR BRADYKININ B1 POUR LE TRAITEMENT DE MALADIES INFLAMMATOIRES

(30) Priority: 02.05.2003 US 467695 P; 17.09.2003 US 503652 P
(43) Date of publication of application: 22.03.2006
(73) Proprietor: ELAN PHARMACEUTICALS, INC., South San Francisco, CA 94080 (US)
(72) Inventor: GAROFALO, Albert, W., South San Francisco, CA 94080 (US); TUNG, Jay, S., Belmont, CA 94002 (US); PLEISS, Michael, A., Sunnyvale, CA 94086 (US); WU, Jing, Redwood City, CA 94065 (US); WONE, David, W., G., Newark, CA 94560 (US); GUINN, Ashley C., Santa Monica, CA 90405 (US); DRESSEN, Darren, B., San Mateo, CA 94403 (US); MARUGG, Jennifer, San Jose, CA 95120 (US); NEITZEL, Martin, Pacifica, CA 94044 (US)
(74) Representative: Wytenburg, Wilhelmus Johannes
(86) International application number: PCT/US2004/013655
(87) International publication number: WO 2004/099155

(56) References cited:
- WO-A-00/50418
- WO-A-01/87888
- WO-A-02/02523
- WO-A-02/22655
- WO-A-96/05196
- WO-A-98/11101
- WO-A-99/27939
- WO-A-03/015774
- WO-A-03/095429
- DE-A- 3 421 386
- GB-A- 2 310 207
- BARALDI ET AL: "Benzoyl Nitrogen Mustard Derivatives of Benzoheterocyclic Analogues of Netropsin: Synthesis and Biological Activity" BIOORGANIC MEDICINAL CHEMISTRY, vol. 11, 10 April 2003 (2003-04-10), page 2381-2388, XP002301176
- BARALDI ET AL: "Synthesis and Antitumor Activity of New Benzoheterocyclic Derivatives of Distamycin A" JOURNAL OF MEDICINAL CHEMISTRY, vol. 43, 2000, pages 2675-2684, XP002301177
- BARALDI ET AL: "Novel Benzoyl Nitrogen Mustard Derivatives of Pyrazole Analogues of Distamycin A: Synthesis and Antileukemic Activity" BIOORGANIC AND MEDICINAL CHEMISTRY, vol. 7, 1999, pages 251-262, XP002301178
- BARALDI ET AL: "Structure-Activity Relationship of Novel Tallimustine Derivatives: Synthesis and Antitumor Activity" BIOORGANIC AND MEDICINAL CHEMISTRY LETTERS, vol. 6, no. 11, 1996, pages 1247-1252, XP002301179
- BARALDI ET AL: "Synthesis and Antitumor Activity of Novel Distamycin Derivatives" BIOORGANIC AND MEDICINAL CHEMISTRY LETTERS, vol. 6, no. 11, 1996, pages 1241-1246, XP002301246
- ATWELL ET AL: "5-Amino-1-(chloromethyl)-1,2-dihydro-3H-b enz[eindoles:" JOURNAL OF MEDICINAL CHEMISTRY, AMERICAN CHEMICAL SOCIETY. WASHINGTON, US, vol. 42, no. 17, 1999, pages 3400-3411, XP002294767 ISSN: 0022-2623
- DATABASE CA [Online] CHEMICAL ABSTRACTS SERVICE, COLUMBUS, OHIO, US; 1998, MARCHINI, S. ET AL: "Sequence-specific DNA alkylation of novel tallimustine derivatives" XP002301180 retrieved from STN Database accession no. 1998:302076 & ANTI-CANCER DRUG DESIGN, vol. 13, no. 3, 1998, pages 193-205,
- DATABASE CA [Online] CHEMICAL ABSTRACTS SERVICE, COLUMBUS, OHIO, US; 1992, LEE, H. H. ET AL: "Pyrazole analogs of the bispyrrolecarboxamide anti-tumor antibiotics: synthesis, DNA binding and anti-tumor properties" XP002301181 retrieved from STN Database accession no. 1992:173857 & ANTI-CANCER DRUG DESIGN, vol. 6, no. 5, 1991, pages 501-517,
- DATABASE CA [Online] CHEMICAL ABSTRACTS SERVICE, COLUMBUS, OHIO, US; 1998, ABDALLAH, MAGDA A. ET AL: "A convenient synthesis of 5-amino-4-(2-benzothiazolyl)pyrazoles" XP002301226 retrieved from STN Database accession no. 1998:233560 & INDIAN JOURNAL OF CHEMISTRY, SECTION B: ORGANIC CHEMISTRY INCLUDING MEDICINAL CHEMISTRY, vol. 36, no. 12, 1997, pages 1175-1177,

## Description

### BACKGROUND OF THE INVENTION

### Field of the Invention

This invention is directed to certain 3-amido-5-substituted pyrazole derivatives and related compounds. These compounds are useful as bradykinin B₁ receptor antagonists to relieve adverse symptoms in mammals mediated, at least in part, by bradykinin B₁ receptor including pain, inflammation, septic shock, the scarring process, etc.

### References

The following literature and patent publications are cited in this application as superscript numbers.
¹ J.G. Menke, et al.; J. Biol. Chem., 269(34):21583-21586 (1994).
² J. F. Hess, Biochem. Human B2 Receptor, Biophys. Res. Commun, 184:260-268 (1992).
³ R M. Burch, et al., "Bradykinin Receptor Antagonists", J. Med. Chem., 30:237-269 (1990).
⁴ Clark, W.G. "Kinins and the Peripheral Central Nervous Systems", Handbook of Experimental Pharmacology, Vol. XXV: "Bradykinin, Kallidin, and Kallikrein. Erdo, E.G. (Ed.), 311-322 (1979).
⁵ Ammons, W.S., et al., "Effects of Intracardiac Bradykinin on T2-T5 Medial Spinothalamic Cells", American Journal of Physiology, 249, R145-152 (1985).
⁶ Costello, A.H. et al., "Suppression of Carageenan-Induced Hyperalgesia, Hyperthermia and Edema by a Bradykinin Antagonist", European Journal of Pharmacology, 171:259-263 (1989).
⁷ Laneuville, et al., "Bradykinin Analogue Blocks Bradykinin-induced Inhibition of a Spinal Nociceptive Reflex in the Rat", European Journal of Pharmacology, 137:281-285 (1987).
⁸ Steranka, et al., "Antinociceptive Effects of Bradykinin Antagonists", European Journal of Pharmacology, 136:261-262 (1987).
⁹ Steranka, et al., "Bradykinin as a Pain Mediator: Receptors are Localized to Sensory Neurons, and Antagonists have Analgesic Actions", Neurobiology, 85:3245-3249 (1987).
¹⁰ Whalley, et al., in Naunyn Schmiederberg's Arch. Pharmacol., 336:652-655 (1987).
¹¹ Back, et al., "Determination of Components of the Kallikrein-Kinin System in the Cerebrospinal Fluid of Patients with Various Diseases", Res. Clin.Stud. Headaches, 3:219-226 (1972).
¹² Ness, et al., "Visceral pain: a Review of Experimental Studies", Pain, 41:167-234 (1990).
¹³ Aasen, et al., "Plasma kallikrein Activity and Prekallikrein Levels during Endotoxin Shock in Dogs", Eur. Surg., 10:5062(1977).
¹⁴ Aasen, et al., "Plasma Kallikrein-Kinin System in Septicemia", Arch. Surg., 118:343-346 (1983).
¹⁵ Katori, et al., "Evidence for the Involvement of a Plasma Kallikrein/Kinin System in the Immediate Hypotension Produced by Endotoxin in Anaesthetized Rats", Br. J. Pharmacol., 98:1383-1391 (1989).
¹⁶ Marceau, et al., "Pharmacology of Kinins: Their Relevance to Tissue Injury and Inflammation", Gen. Pharmacol., 14:209-229 (1982).
¹⁷ Weipert, et al., Brit J. Pharm., 94:282-284 (1988).
¹⁸ Haberland, "The Role of Kininogenases, Kinin Formation and Kininogenase Inhibitor in Post Traumatic Shock and Related Conditions", Klinische Woochen-Schrift, 56:325-331 (1978).
¹⁹ Ellis, et al., "Inhibition of Bradykinin-and Kallikrein-Induced Cerebral Arteriolar Dilation by Specific Bradykinin Antagonist", Stroke, 18:792-795 (1987).
²⁰ Kamitani, et al., "Evidence for a Possible Role of the Brain Kallikrein-Kinin System in the Modulation of the Cerebral Circulation", Circ. Res., 57:545-552 (1985).
²¹ Barnes, "Inflammatory Mediator Receptors and Asthma", Am. Rev. Respir. Dis., 135:S26-S31 (1987).
²³ Fuller, et al., "Bradykinin-induced Bronchoconstriction in Humans", Am. Rev. Respir. Dis., 135:176-180 (1987).
²⁴ Jin, et al., "Inhibition of Bradykinin-Induced Bronchoconstriction in the Guinea-Pig by a Synthetic B2 Receptor Antagonist", Br. J. Pharmacol., 97:598-602 (1989).
²⁵ Polosa, et al., "Contribution of Histamine and Prostanoids to Bronchoconstriction Provoked by Inhaled Bradykinin in Atopic Asthma", Allergy, 45:174-182 (1990).
²⁶ Baumgarten, et al., "Concentrations of Glandular Kallikrein in Human Nasal Secretions Increase During Experimentally Induced Allergic Rhinitis", J.Immunology, 137:1323-1328 (1986).
²⁷ Proud, et al., "Nasal Provocation with Bradykinin Induces Symptoms of Rhinitis and a Sore Throat", Am. Rev. Respir Dis.,137:613-616 (1988).
²⁸ Steward and Vavrek in "Chemistry of Peptide Bradykinin Antagonists" Basic and Chemical Research, R. M. Burch (Ed.), pages 51-96 (1991).
²⁹ Seabrook, et al., Expression of B1 and B2 Bradykinin Receptor mRNA and Their Functional Roles in Sympathetic Ganglia and Sensory Dorsal Root Ganglia Neurons from Wild-type and B2 Receptor Knockout Mice, Neuropharmacology, 36(7):1009-17 (1997)
³⁰ Elguero, et al., Nonconventional Analgesics: Bradykinin Antagonists, An. R. Acad Farm., 63(1):173-90 (Spa) (1997)
³¹ McManus, U.S. Patent No. 3,654,275, Quinoxalinecarboxamide Antiinflammatory Agents, issued April 4, 1972
³² Beyreuther, B.; *et al.,* International Patent application publication number WO 03/007958 A1 filed on July 4, 2002.
³³ Marceau, "Kinin B1 Receptors: A Review," Immunopharmacology, 30:1-26 (1995).
³⁴ Giese, et al., U.S. Patent No. 5,916,908, issued June 29, 1999
³⁵ Yoshida, *et al.,* Japanese Patent Application Serial No. 49100080
³⁶ Oxford Dictionary of Biochemistry and Molecular Biology. Oxford University Press, 2001.

### State of the Art

Bradykinin or kinin-9 (BK) is a vasoactive nonapeptide, H-Arg¹ -Pro² - Pro³ -Gly⁴-Phe⁵ - Ser⁶ -Pro⁷ -Phe⁸ -Arg⁹ -OH (SEQ. ID. NO.1), formed by the action of plasma kallikrein, which hydrolyses the sequence out of the plasma globulin kininogen. Plasma kallikrein circulates as an inactive zymogen, from which active kallikrein is released by Hageman factor. Tissue kallikrein appears to be located predominantly on the outer surface of epithelial cell membranes at sites thought to be involved in transcellular electrolyte transport.

Glandular kallikrein cleaves kininogen one residue earlier to give the decapeptide Lys-bradykinin (kallidin, Lys-BK) (SEQ. ID. NO. 2). Met-Lys-bradyldnin (SEQ. ID. NO. 3) is also formed, perhaps by the action of leukocyte kallikrein. Pharmacologically important analogues include des-Arg⁹ or BK₁₋₈ (Amino acids 1-8 of SEQ. ID. NO. 1)and Ile-Ser-bradykinin (or T-kinin) (SEQ. ID. NO. 4), [Hyp³]bradykinin (SEQ. ID. NO. 5), and [Hyp⁴]bradykinin (SEQ. ID. NO. 6).³⁶

Bradykinin (BK) (SEQ. ID. NO.1) is known to be one of the most potent naturally occurring stimulators of C-fiber afferents mediating pain. It also is a powerful blood-vessel dilator, increasing vascular permeability and causing a fall in blood pressure, edema-producing agent, and stimulator of various vascular and non-vascular smooth muscles in tissues such as uterus, gut and bronchiole. Bradykinin (SEQ. ID. NO. 1) is formed in a variety of inflammatory conditions and in experimental anaphylactic shock. The kinin/kininogen activation pathway has also been described as playing a pivotal role in a variety of physiologic and pathophysiologic processes, being one of the first systems to be activated in the inflammatory response and one of the most potent simulators of: (i) phospholipase A₂ and, hence, the generation of prostaglandins and leukotrienes; and (ii) phospholipase C and thus, the release of inositol phosphates and diacylgylcerol. These effects are mediated predominantly via activation of BK receptors of the BK₂ type.

Bradykinin receptor is any membrane protein that binds bradykinin (BK) (SEQ. ID. NO. 1) and mediates its intracellular effects. Two types of receptors are recognized: B₁, on which order of potency is des-Arg⁹-bradykinin (BK₁₋₈) (Amino acids 1-8 of SEQ. ID. NO.1)=kallidin (Lys-BK) (SEQ. ID. NO. 2)>BK (SEQ. ID. NO.1); and B₂, with order of potency kallidin (SEQ. ID. NO. 2)>BK (SEQ. ID. NO. 1)>>BK₁₋₈ (Amino acids 1-8 of SEQ. ID. NO. 1). Hence, BK₁₋₈ (Amino acids 1-8 of SEQ. ID. NO. 1) is a powerful discriminator.³⁶ B₁ receptors are considerably less common than B₂ receptors, which are present in most tissues. The rat B₂ receptor is a seven-transmembrane-domain protein which has been shown on activation to stimulate phosphoinositide turnover. The B₁ subtype is induced by inflammatory processes.³³ The distribution of receptor B₁ is very limited since this receptor is only expressed during states of inflammation. Bradykinin receptors have been cloned for different species, notably the human B₁ receptor (see J.G. Menke *et al.*¹, and human B2 receptor J.F. Hess²). Examples: B₁, database code BRB1_HUMAN, 353 amino acids (40.00 kDa); B₂, database code BRB2_HUMAN, 364 amino acids (41.44 kDa).³⁶

Two major kinin precursor proteins, high molecular weight and low molecular weight kininogen are synthesized in the liver, circulate in plasma, and are found in secretions such as urine and nasal fluid. High molecular weight kininogen is cleaved by plasma kallikrein, yielding BK (SEQ. ID. NO. 1), or by tissue kallikrein, yielding kallidin. Low molecular weight kininogen, however, is a substrate only for tissue kallikrein. In addition, some conversion of kallidin to BK (SEQ. ID. NO. 1) may occur inasmuch as the amino terminal lysine residue of kallidin (SEQ. ID. NO. 2) is removed by plasma aminopeptidases. Plasma half-lives for kinins are approximately 15 seconds, with a single passage through the pulmonary vascular bed resulting in 80-90% destruction. The principle catabolic enzyme in vascular beds is the dipeptidyl carboxypeptidase kininase II or angiotensin-converting enzyme (ACE). A slower acting enzyme, kininase I, or carboxypeptidase N, which removes the carboxyl terminal Arg, circulates in plasma in great abundance. This suggests that it may be the more important catabolic enzyme physiologically. Des-Arg⁹ -bradykinin (Amino acids 1-8 of SEQ. ID. NO. 1) as well as des-Arg¹⁰ -kallidin (Amino acids 1-9 of SEQ. ID. NO. 2) formed by kininase I acting on BK (SEQ. ID. NO. 1) or kallidin (SEQ. ID. NO. 2), respectively, are acting BK₁ receptor agonists, but are relatively inactive at the more abundant BK₂ receptor at which both BK (SEQ. ID. NO. 1) and kallidin (SEQ. ID. NO. 2) are potent agonists.

Direct application of bradykinin (SEQ. ID. NO.1) to denuded skin or intra-arterial or visceral injection results in the sensation of pain in mammals including humans. Kinin-like materials have been isolated from inflammatory sites produced by a variety of stimuli. In addition, bradykinin receptors have been localized to nociceptive peripheral nerve pathways and BK (SEQ. ID. NO. 1) has been demonstrated to stimulate central fibers mediating pain sensation. Bradykinin (SEQ. ID. NO. 1) has also been shown to be capable of causing hyperalgesia in animal models of pain. See, Burch, et al,³ and Clark, W.G.⁴

These observations have led to considerable attention being focused on the use of BK antagonists as analgesics. A number of studies have demonstrated that bradykinin antagonists are capable of blocking or ameliorating both pain as well as hyperalgesia in mammals including humans. See, Ammons, W. S., *et al.*⁵*,* Clark, W.G.⁴, Costello, A.H., *et al.* ⁶, Laneuville, *et al.* ⁷, Steranka, *et al.*⁸ and Steranka, *et al.*⁹

Currently accepted therapeutic approaches to analgesia have significant limitations. While mild to moderate pain can be alleviated with the use of non-steroidal anti-inflammatory drugs and other mild analgesics, severe pain such as that accompanying surgical procedures, burns and severe trauma requires the use of narcotic analgesics. These drugs carry the limitations of abuse potential, physical and psychological dependence, altered mental status and respiratory depression which significantly limit their usefulness.

Prior efforts in the field of BK antagonists indicate that such antagonists can be useful in a variety of roles. These include use in the treatment of burns, perioperative pain, migraine and other forms of pain, shock, central nervous system injury, asthma, rhinitis, premature labor, inflammatory arthritis, inflammatory bowel disease, neuropathic pain, etc. For example, *Whalley, et al.* ¹⁰ has demonstrated that BK antagonists are capable of blocking BK-induced pain in a human blister base model. This suggests that topical application of such antagonists would be capable of inhibiting pain in burned skin, e.g., in severely burned patients that require large doses of narcotics over long periods of time and for the local treatment of relatively minor burns or other forms of local skin injury.

The management of perioperative pain requires the use of adequate doses of narcotic analgesics to alleviate pain while not inducing excessive respiratory depression. Post-operative narcotic-induced hypoventilation predisposes patients to collapse of segments of the lungs, a common cause of post-operative fever, and frequently delays discontinuation of mechanical ventilation. The availability of a potent non-narcotic parenteral analgesic could be a significant addition to the treatment of perioperative pain. While no currently available BK antagonist has the appropriate pharmacodynamic profile to be used for the management of chronic pain, frequent dosing and continuous infusions are already commonly used by anesthesiologists and surgeons in the management of perioperative pain.

Several lines of evidence suggest that the kallikrein/kinin pathway may be involved in the initiation or amplification of vascular reactivity and sterile inflammation in migraine. (See, *Back, et al.*¹¹)*.* Because of the limited success of both prophylactic and non-narcotic therapeutic regimens for migraine as well as the potential for narcotic dependence in these patients, the use of BK antagonists offers a highly desirable alternative approach to the therapy of migraine.

Bradykinin (SEQ. ID. NO. 1) is produced during tissue injury and can be found in coronary sinus blood after experimental occlusion of the coronary arteries. In addition, when directly injected into the peritoneal cavity, BK (SEQ. ID. NO. 1) produces a visceral type of pain. (See, Ness, *et al*.¹²). While multiple other mediators are also clearly involved in the production of pain and hyperalgesia in settings other than those described above, it is also believed that antagonists of BK (SEQ. ID. NO. 1) have a place in the alleviation of such forms of pain as well.

Shock related to bacterial infections is a major health problem. It is estimated that 400,000 cases of bacterial sepsis occur in the United States yearly; of those, 200,000 progress to shock, and 50% of these patients die. Current therapy is supportive, with some suggestion in recent studies that monoclonal antibodies to Gram-negative endotoxin may have a positive effect on disease outcome. Mortality is still high, even in the face of this specific therapy, and a significant percentage of patients with sepsis are infected with Gram-positive organisms that would not be amenable to anti-endotoxin therapy.

Multiple studies have suggested a role for the kallikrein/kinin system in the production of shock associated with endotoxin. See, Aasen, *et al.*¹³, Aasen, *et al.*¹⁴, Katori, *et al.*¹⁵ and Marceau, *et al.*¹⁶ Recent studies using newly available BK antagonists have demonstrated in animal models that these compounds can profoundly affect the progress of endotoxic shock. (See, Weipert, *et al.*¹⁷). Less data is available regarding the role of BK (SEQ. ID. NO. 1) and other mediators in the production of septic shock due to Gram-positive organisms. However, it appears likely that similar mechanisms are involved. Shock secondary to trauma, while frequently due to blood loss, is also accompanied by activation of the kallikrein/kinin system. (See, Haberland.¹⁸)

Numerous studies have also demonstrated significant levels of activity of the kallikrein/kinin system in the brain. Both kallikrein and BK (SEQ. ID. NO. 1) dilate cerebral vessels in animal models of central nervous system (CNS) injury. (See Ellis, *et al*.¹⁹ and Kamitani, *et al.*²⁰). Bradykinin antagonists have also been shown to reduce cerebral edema in animals after brain trauma. Based on the above, it is believed that BK antagonists should be useful in the management of stroke and head trauma.

Other studies have demonstrated that BK receptors are present in the lung, that BK (SEQ. ID. NO.1) can cause bronchoconstriction in both animals and man and that a heightened sensitivity to the bronchoconstrictive effect of BK (SEQ. ID. NO. 1) is present in asthmatics. Some studies have been able to demonstrate inhibition of both BK (SEQ.ID. NO. 1) and allergen-induced bronchoconstriction in animal models using BK antagonists. These studies indicate a potential role for the use of BK antagonists as clinical agents in the treatment of asthma. (See Barnes²¹, Burch, *et al.³,* Fuller, *et al.* ²³, Jin, *et al.²⁴* and Polosa, *et al.²⁵*.) Bradykinin has also been implicated in the production of histamine and prostanoids to bronchoconstriction provoked by inhaled bradykinin in atopic asthma.²⁵ BK (SEQ. ID. NO. 1) has also been implicated in the production of symptoms in both allergic and viral rhinitis. These studies include the demonstration of both kallikrein and BK (SEQ. ID. NO. 1) in nasal lavage fluids and that levels of these substances correlate well with symptoms of rhinitis. (See, Baumgarten, *et al*.²⁶, Jin, *et al.* ²⁴, and Proud, *et al.²⁷)*

In addition, studies have demonstrated that BK (SEQ. ID. NO. 1) itself can cause symptoms of rhinitis. Stewart and Vavrek²⁸ discuss peptide BK antagonists and their possible use against effects of BK (SEQ. ID. NO.1). A great deal of research effort has been expended towards developing such antagonists with improved properties. However, notwithstanding extensive efforts to find such improved BK antagonists, there remains a need for additional and more effective BK antagonists. Two of the major problems with presently available BK antagonists are their low levels of potency and their extremely short durations of activity. Thus there is a special need for BK antagonists having increased potency and for duration of action.

Two generations of peptidic antagonists of the B2 receptor have been developed. The second generation has compounds two orders of magnitude more potent as analgesics than first generation compounds and the most important derivative was icatibant. The first non-peptidic antagonist of the B2 receptor, described in 1993, has two phosphonium cations separated by a modified amino acid. Many derivatives of this di-cationic compound have been prepared. Another non-peptidic compound antagonist of B2 is the natural product Martinelline. See Elguero, *et al.,*³⁰ and Seabrook.²⁹

U.S. Patent 3,654,275³¹ teaches that certain 1,2,3,4-tetrahydro-1-acyl-3-oxo-2-quinoxalinecarboxamides have anti-inflammatory activity.

International Patent Application WO 03/007958 filed on July 2,2002 and published on January 30, 2003 discloses tetrahydroquinoxalines acting as bradykinin antagonists.³²

U.S. Patent 5,916,908³⁴ teaches the use of 3,5-disubstituted pyrazoles or 3,4,5-trisubstituted pyrazoles as kinase inhibitors.

Japanese Patent Application Serial No. 49100080³⁵ teaches 2-aminopyrazoles as anti-inflammatory agents.

Currently there is no marketed therapeutic agent for the inhibition of bradykinin B₁ receptor. In view of the above, compounds which are bradykinin B₁ receptor antagonists would be particularly advantageous in treating those diseases mediated by bradykinin B₁ receptor.
WO 99/27939 describes a method of using certain ureido derivatives of a poly-4-amino-2-carboxy-1-methyl pyrrole of the following formula to inhibit inflammation: 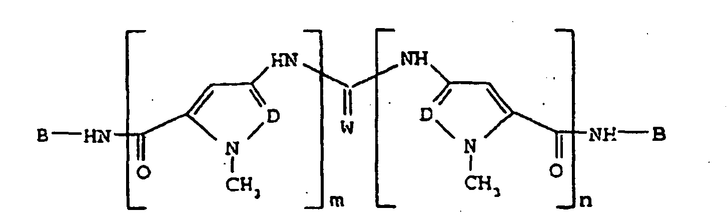 GB 2,310,207 describes certain ureido derivatives of substituted heterocyclic compounds of the following formula. Apparently, the compounds are angiogenesis inhibitors and are useful for treating, for example, chronic inflammation, diabetic retinopathy, psoriasis, rheumatoid arthritis and tumour growth.

B-CO-B (I)

wherein each of the B groups, which are the same, is a group (i) 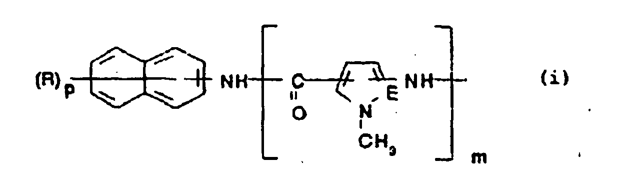 WO 02/02523 describes a process for preparing certain distamycin-guanidines of the following formula. Apparently, the compounds have antitumour activity. 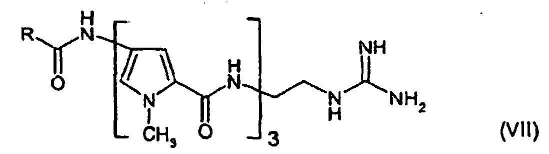 wherein
R is selected from the group consisting of: 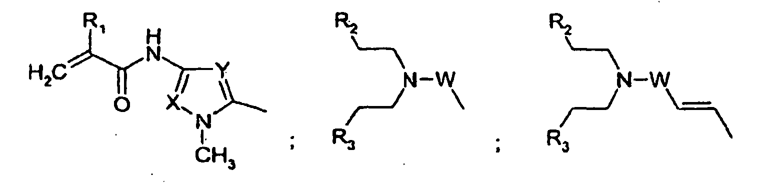 WO 96/05196 describes certain peptidic compounds of the following formula that are analgous to Distamycin A. Apparently, the compounds are useful as antitumour or antiviral agents. 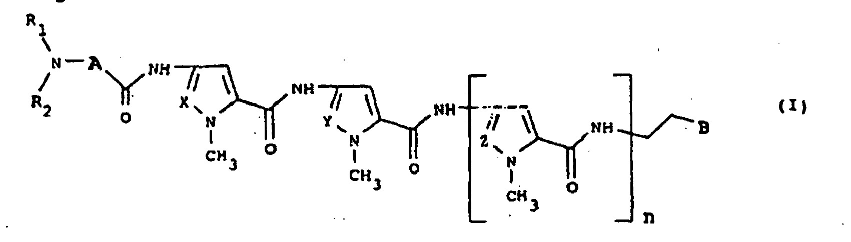 wherein n is 0 or 1;
each of X, Y, Z is independently N or CH;
A is a pentatomic heteromonocyclic ring unsubstituted or
substituted by a C₁-C₃ alkyl group;
WO 02/22655 describes certain distamycin derivatives of the following formula, in particular acryloyl derivatives of distamycin conjugated with glutathione. Apparently, the compounds have antitumoural activity. 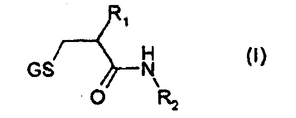 R₂ can be a distamycin or distamycin-like structure of the following formula where G can be phenylene. 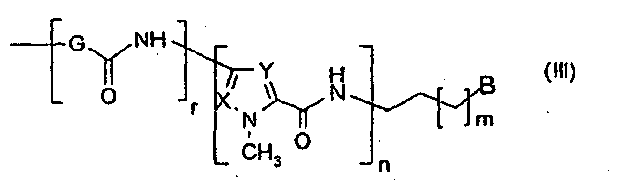 WO 03/015774 describes certain benzamide compounds of the following formula. Apparently, they are useful in the prevention of a disease or medical condition mediated through glucokinase (GLK), leading to a decreased glucose threshold for insulin secretion, e.g., type 2 diabetes. 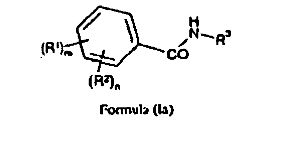 The following compound is shown in Example 5 on page 89 therein: 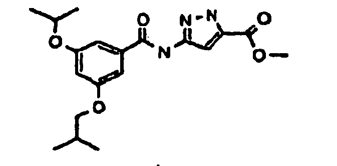 WO 98/11101 describes certain amino analogues of cyclopropylindoles. Apparently, these compounds are useful as prodrugs for antibody-directed enzyme-prodrug therapy (ADEPT) and gene-directed enzyme-prodrug therapy (GDEPT) for cancer. The following compound is shown as Compound D in Figure 5 therein: 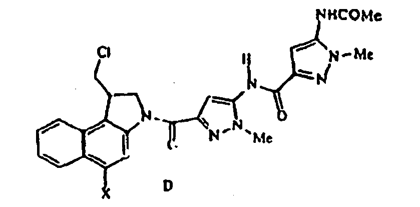 Baraldi et al., Bioorganic and Medicinal Chemistry, 2003, Vol. 11, pp. 2381-2388 , describes the synthesis and biological anti-cancer activity of certain benzoyl nitrogen mustard derivatives of benzoheterocyclic analogues of Netropsin of the following formula: 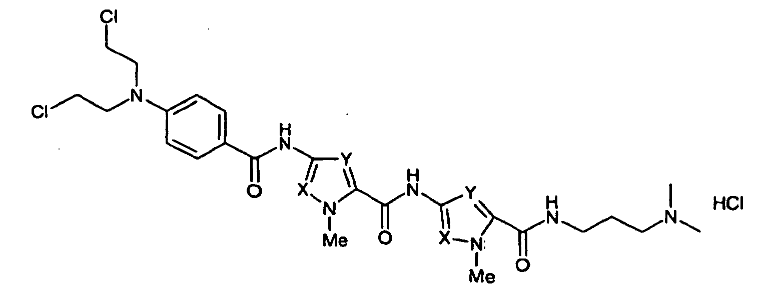 Baraldi et al., J. Med. Chem., 2000, Vol. 43, pp. 2675-2684 , describes the synthesis and antitumour activity of certain Benzoheterocyclic derivatives of Distamycin A, including those illustrated below:

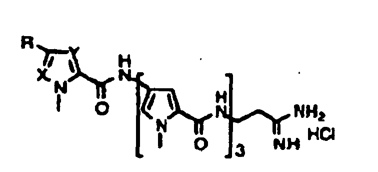

| **Compound** | **X** | **Y** | **R** |
|---|---|---|---|
| **9** | N | CH | 4-[(ClCH₂CH₂)₂N]C₆H₄CONH |
| **10** | N | CH | CH₂=CBrCONH |

Baraldi et al., Bioorganic and Medicinal Chemistry, 1999, Vol. 7, pp. 251-262 , describes the synthesis and antileukemic activity of benzoyl nitrogen mustard derivatives of certain pyrazole analogues of Distamycin A, exemplified by the following formulae: 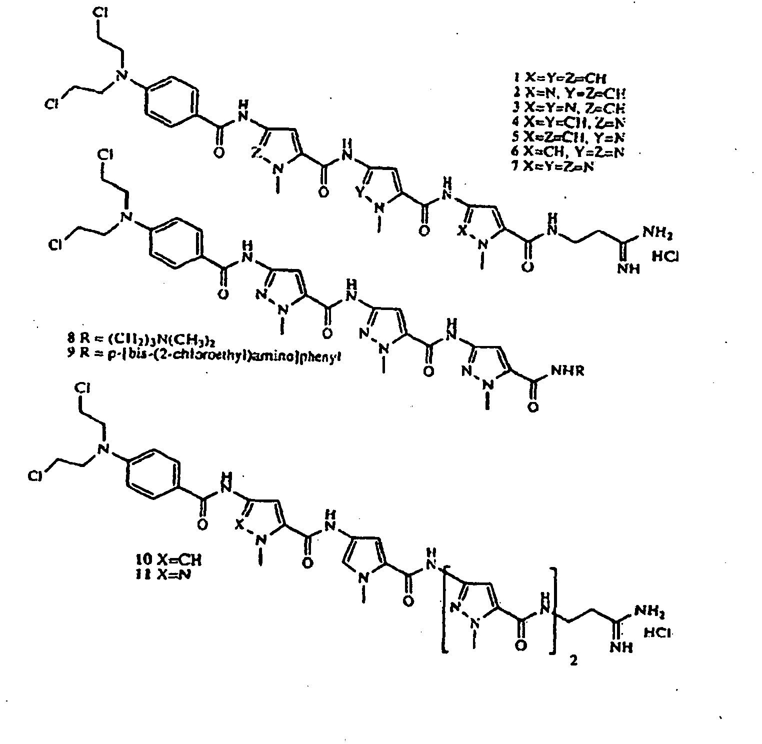 Baraldi et al., Bioorganic and Medicinal Chemistry Letters, 1996, Vol. 6, pp. 1247-1252 , describes the synthesis and antitumour activity of certain tallimustine derivatives, including the compound illustrated below: 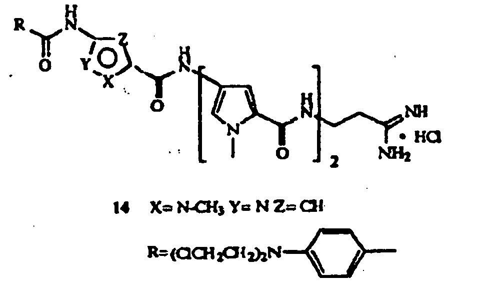 Baraldi et al., Bioorganic and Medicinal Chemistry Letters, 1996, Vol. 6, pp. 1241-1246 , describes the synthesis and antitumour activity of certain Distamycin derivatives, including the compound illustrated below: 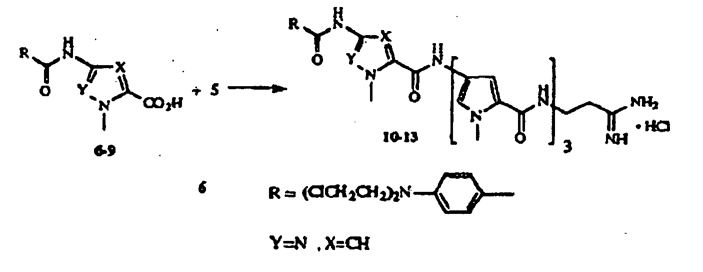 Atwell et al., J. Med. Chem., 1999, Vol. 42, No. 17, pp. 3400-3411 , describes a study of structure-activity relationships for the cytotoxicity of certain side-chain analogues of 5-amino-seco-CBI compounds. The following compound is shown as Compound D in Table 1 therein: 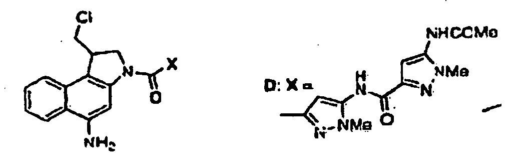 Marchini et al., Anti-Cancer Drug Design, 1998, Vol. 13, pp. 193-205 , describes certain analogues of the sequence-specific minor groove alkylator, tallimustine including those illustrated below. Apparently, the compounds are cytotoxic. 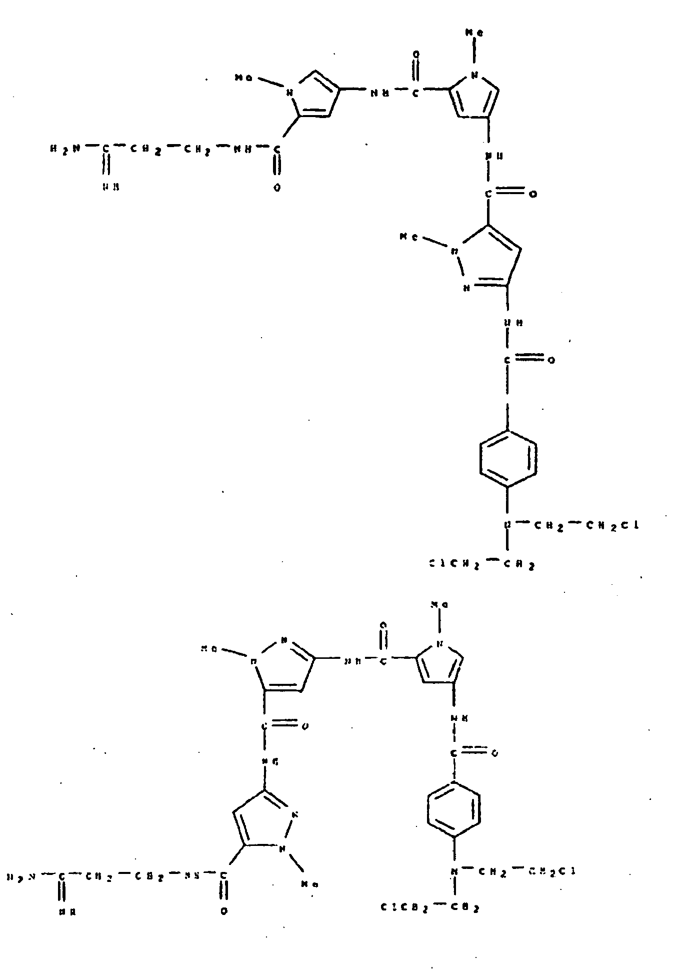 Lee et al., Anti-Cancer Drug Design, 1991, Vol. 6, pp. 501-517 , describes certain pyrazole analogues of bispyrrolecarboxamide anti-tumour antibiotics, including the following compound. 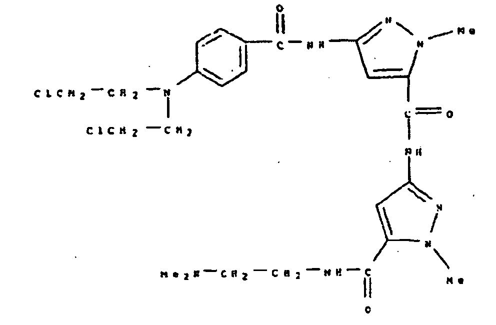 Abdallah et al., Indian Journal of Chemistry, Section B: Organic Chemistry Including Medicinal Chemistry, 1997, Vol. 36B, pp. 1175-1177 , describes the synthesis of certain 5-amino-4-(2-benzothiazolyl)pyrazoles, including the following compound. 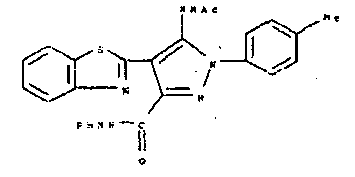 WO 03/095429 describes certain compounds that apparently are useful for the treatment, diagnosis and prophylaxis of diseases in which abnormal protein structures occur, e.g., Alzheimer's disease. The following compounds are shown on page 17 therein: 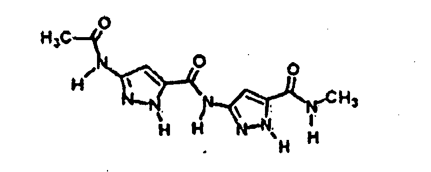 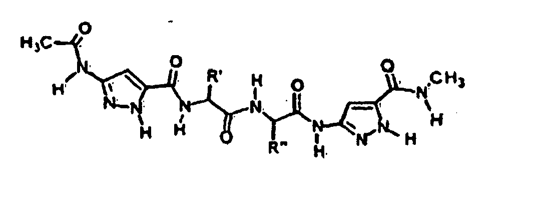 WO 00/50418 describes certain benzenesulfonamide compounds of the following formula as bradykinin antagonists. 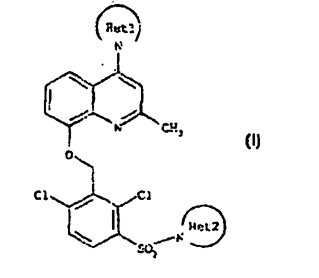 WO 01/87888 describes certain pyrazolopyrimidinone derivatives of the following formula as bradykinin antagonists. 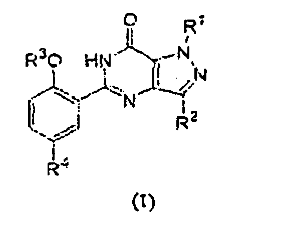 DE 3,421,386 describes certain anti-inflammatory compounds of the following formula:

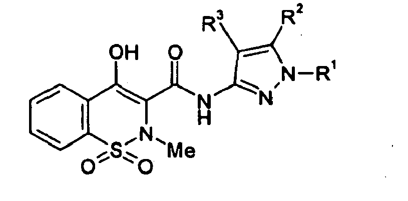

### SUMMARY OF THE INVENTION

This invention is directed, in part, to compounds that are bradykinin B₁ receptor antagonist. It is also directed to compounds that are useful for treating diseases or relieving adverse symptoms associated with disease conditions in mammals, where the disease is mediated at least in part by bradykinin B₁ receptor. For example, inhibition of the bradykinin B₁ receptor is believed to be useful for the moderation of pain, inflammation, septic shock, the scarring process, etc. These compounds are preferably selective for antagonism of the B₁ receptor over the B₂ receptor. Certain of the compounds exhibit increased potency and are expected to also exhibit an increased duration of action.

In one embodiment, this invention provides compounds of Formula (I) or Formula (II): 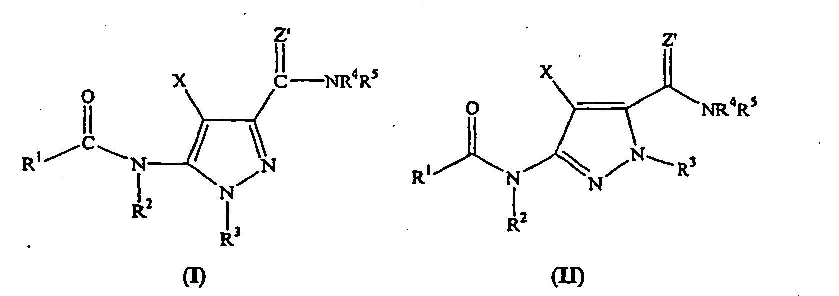 wherein
Z' is selected from O, S and NH;
R¹ is selected from 2-chlorophenyl and 2-fluorophenyl;
R² is selected from the group consisting of hydrogen, alkyl, and substituted alkyl;
R³ is selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic;
R⁴ is selected from the group consisting of aryl, substituted aryl, heteroaryl, and substituted heteroaryl;
R⁵ is selected from the group consisting of hydrogen, alkyl and substituted alkyl;
X is selected from the group consisting of hydrogen, alkyl, substituted alkyl, alkoxy, substituted alkoxy, aryl, substituted aryl, carboxyl, carboxyl esters, cyano, halo, heteroaryl, substituted heteroaryl, hydroxy, nitro, amino, substituted amino, acylamino, and aminoacyl;
or pharmaceutically acceptable salts thereof;
with the proviso that the compound of Formula (I) is not
A') 4-Bromo-5-(2-chloro-benzoylamino)-1H-pyrazole-3-carboxylic acid phenylamide.

In Formula (I) or Formula (II), Z' is preferably O. disubstituted phenyls and trisubstituted phenyls such as 5-dimethylaminonaphth-1-yl, 2-chlorophenyl, 2-fluorophenyl, 2-bromophenyl, 2-hydroxyphenyl, 2-nitrophenyl, 2-methylphenyl, 2-methoxyphenyl, 2-phenoxyphenyl, 2-trifluoromethylphenyl, 4-fluorophenyl, 4-chlorophenyl, 4-bromophenyl, 4-nitrophenyl, 4-methylphenyl, 4-hydroxyphenyl, 4-methoxyphenyl, 4-ethoxyphenyl, 4-butoxyphenyl, 4-*iso*-propylphenyl, 4-phenoxyphenyl, 4-trifluoromethylphenyl, 4-hydroxymethylphenyl, 3-methoxyphenyl, 3-hydroxyphenyl, 3-nitrophenyl, 3-fluorophenyl, 3-chlorophenyl, 3-bromophenyl, 3-phenoxyphenyl, 3-thiomethoxyphenyl, 3-methylphenyl, 3-trifluoromethylphenyl, 2,3-dichlorophenyl, 2,3-difluorophenyl, 2,4-dichlorophenyl, 2,5-dimethoxyphenyl, 3,4-dichlorophenyl, 3,4-difluorophenyl, 3,4-methylenedioxyphenyl, 3,4-dimethoxyphenyl, 3,5-difluorophenyl, 3,5-dichlorophenyl, 3,5-di-(trifluoromethyl)phenyl, 3,5-dimethoxyphenyl, 2,4-dichlorophenyl, 2,4-difluorophenyl, 2,6-difluorophenyl, 3,4,5-trifluorophenyl, 3,4,5-trimethoxyphenyl, 3,4,5-tri-(trifluoromethyl)phenyl, 2,4,6-trifluorophenyl, 2,4,6-trimethylphenyl, 2,4,6-tri-(trifluoromethyl)phenyl, 2,3,5-trifluorophenyl, 2,4,5-trifluorophenyl, 2,5-difluorophenyl, 2-fluoro-3-trifluoromethylphenyl, 4-fluoro-2-trifluoromethylphenyl, 2- fluoro4-trifluoromethylphenyl, 4-benzyloxyphenyl, 2-chloro-6-fluorophenyl, 2,3,4,5,6-pentafluorophenyl, 2,5-dimethylphenyl, 4-phenylphenyl and 2-fluoro-3-trifluoromethylphenyl, 2-(quinolin-8-yl)thiomethyl)phenyl, 2-((3-methylphen-1-ylthio)methyl)phenyl, and the like.

Preferred R¹ substituted aryl groups are alkaryl groups which include, by way of example, benzyl, 2-phenylethyl, 3-phenyl-*n*-propyl, and the like.

Preferred R¹ alkyl, substituted alkyl, alkenyl, cycloalkyl and cycloalkenyl groups in Formula (I) or Formula (II) include, by way of example, *iso*-propyl, *n*-propyl, *n*-butyl, *iso*-butyl, *sec*-butyl, *t*-butyl, -CH₂CH=CH₂, -CH₂CH=CH(CH₂)₄CH₃, cyclopropyl, cyclobutyl, cyclohexyl, cyclopentyl, cyclohex-1-enyl, -CH₂-cyclopropyl, -CH₂-cyclobutyl, -CH₂-cyclohexyl, -CH₂-cyclopentyl, -CH₂CH₂-cyclopropyl, -CH₂CH₂-cyclobutyl, -CH₂CH₂-cyclohexyl, -CH₂CH₂-cyclopentyl, and the like.

Preferred R¹ heteroaryls and substituted heteroaryls in Formula (I) or Formula (II) include, by way of example, pyrid-2-yl, pyrid-3-yl, pyrid-4-yl, fluoropyridyls (including 5-fluoropyrid-3-yl), chloropyridyls (including 5-chloropyrid-3-yl), thiophen-2-yl, thiophen-3-yl, benzothiazol-4-yl, 2-phenylbenzoxazol-5-yl, furan-2-yl, benzofuran-2-yl, thionaphthen-2-yl, 2-chlorothiophen-5-yl, 3-methylisoxazol-5-yl, 2-(thiophenyl)thiophen-5-yl, 6-methoxythionaphthen-2-yl, 3-phenyl-1,2,4-thiooxadiazol-5-yl, 2-phenyloxazol-4-yl, 5-chloro-1,3-dimethylpyrazol-4-yl; 2-methoxycarbonyl-thiophen-3-yl; 2,3-dimethylimidazol-5-yl; 2-methylcarbonylamino-4-methyl-thiazol-5-yl; quinolin-8-yl; thiophen-2-yl; 1-methylimidiazol-4-yl; 3,5-dimethylisoxazol-4-yl; and the like.

Particularly preferred R¹ groups include, by way of example only, 5-dimethylaminonaphth-1-yl, 2-chlorophenyl, 2-fluorophenyl, 2-bromophenyl, 2-hydroxyphenyl, 2-nitrophenyl, 2-methylphenyl, 2-methoxyphenyl,

R¹ may be also be sulfonated aminoalkyl such as Formula (V) below, wherein R²¹ is hydrogen or methyl, and R²⁰ is an amino acid side chain or where R²⁰ and R²¹ and the atoms to which they are attached form a heterocyclic or heteroaryl group of from 4 to 12 ring atoms, and R²² is alkyl, substituted alkyl, aryl or substituted aryl.

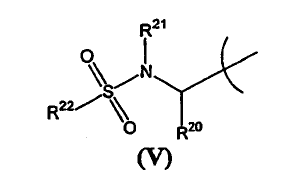

Preferred R² groups include hydrogen, methyl, ethyl, isopropyl, 2-methoxyeth-1-yl, pyrid-3-ylmethyl, benzyl, *t*-butoxycarbonyl-methyl and the like. The particularly preferred R² is hydrogen.

Preferred R³ groups include hydrogen, methyl, ethyl, isopropyl, 2-methoxyeth-1-yl, pyrid-3-ylmethyl, benzyl, t-butoxycarbonyl-methyl and the like. Particularly preferred R³ groups include hydrogen, C₁₋₄alkyl, optionally substituted monocyclic aryl, and optionally substituted monocyclic heteroaryl. Most preferred R³ groups are hydrogen, methyl and phenyl.

Preferred R⁴ groups include 4-(N,N-diethylamino)phenyl; 4-(N,N-dimethylamino)phenyl; 2-methylphenyl; phenyl; 1-naphthyl; 4-methylphenyl; 4-chlorophenyl; 3,4-dichlorophenyl; 4-methoxyphenyl; pyridin-3-yl; pyridin-4-yl; 2-chlorophenyl; 4-methoxy-3-hydroxyphenyl; 2-methoxypyridin-5-yl; 3,5-Dimethoxyphenyl; pyrazin-2-yl; 4-ethylphenyl; 4-(1-(RorS)-1-methylprop-1-yl); 1-methyl-1*H*-pyrazol-3-yl; 9H-fluoren-9-yl; isoquinolin-1-yl; isoquinolin-3-yl; 4-phenylthiazol-2-yl; 4-(4-pyridin-4-yl-piperazin-1-yl)phenyl; 4-[1,4'-bipiperidin]-1'-yl-phenyl; 1H-benzimidazol-2-yl; benzothiazol-2-yl; 4-(2-(4,5-dihydro-1H-imidazol-2-yl)ethyl)phenyl; 4-(3-aminopropyl)phenyl; 4-(2-aminoethyl)phenyl; 4-[1,4'-bipiperidin]-1'-yl-phenyl; 4-(2-(1,4,5,6-tetrahydropyrimidin-2-yl)ethyl)phenyl; 4-(4,5-dihydro-1H-imidazol-2-yl)phenyl; 4-fluoro-3-cyano-phenyl; and 4-(2-cyanoethyl)phenyl.

Additional preferred R⁴ groups include 2-ethoxyphenyl; 3-(2-methylthiazol-5-yl)-pyrazol-5-yl; 4-aminophenylamino; 4-(1H-imidazol-2-ylmethyl)-phenylamino; 4-[2-(1H-imidazol-2-yl)-ethyl]-phenylamino; 4-aminomethyl-phenylamino; 4-(1H-imidazol-2-yl)-phenylamino; 4-[N,N'-diethylamidino]-phenylamino; 4-[N,N'-dimethylamidino]-phenylamino; 4-[N,N'-diphenylamidino]-phenylamino; 4-(4,5-dihydro-1H-imidazol-2-ylmethyl)-phenylamino; 4-(1H-benzimidazol-2-yl)-phenylamino; 4-[N-(4,5-dihydro-1H-imidazol-2-yl)aminomethyl]-phenylamino; 4-(1H-benzimidazol-2-ylmethyl)-phenylamino; 4-(1,4,5,6-tetrahydro-pyrimidin-2-yl)-phenylamino; and 4-(1,4,5,6-tetrahydro-pyrimidin-2-ylmethyl)-phenylamino.

Preferred R⁵ groups include hydrogen, methyl, ethyl, *iso*-propyl, 2-methoxyethyl, and pyrid-3-yl-methyl.

Preferred X groups include hydrogen, bromine, chlorine, fluorine and methyl.

When R³ in Formula (I) or Formula (II) is other than hydrogen, two geometric isomers may exist. When R³ is hydrogen Formula (I) or Formula (II) are tautomers. In those cases where the compounds of Formula (I) or Formula (II) exist as tautomers, optical isomers or geometric isomers, the above formulas are intended to represent isomer mixtures as well as the individual isomeric bradykinin B₁ receptor antagonist or intermediate isomers, all of which are encompassed within the scope of this invention.

Further, references to the compounds of Formula (I) or Formula (II) with respect to pharmaceutical applications thereof are also intended to include pharmaceutically acceptable salts of the compounds of Formula (I) or Formula (II).

Compounds within the scope of this invention include those set forth in Table I as follows:

**TABLE I**

| | | | | | |
|---|---|---|---|---|---|
| 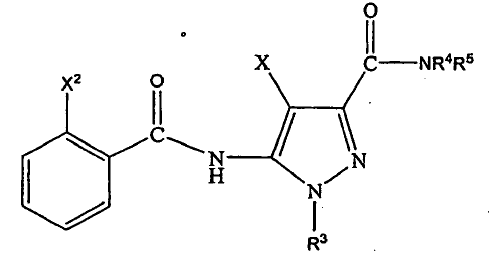 | | | | | |

| **Cpd #** | **X²** | **X** | **R³** | **R⁵** | **R⁴** |
|---|---|---|---|---|---|
| **201** | Cl | Br | H | H | 4-(N,N-diethylamino)phenyl |
| **202** | Cl | Br | H | H | 4-(N,N-dimethylamino)phenyl |
| **203** | Cl | Br | H | H | 2-methylphenyl |
| **204** | Cl | CH₃ | H | H | phenyl |
| **205** | Cl | ethyl | H | H | phenyl |
| **206** | Cl | *n*-propyl | H | H | phenyl |
| **207** | Cl | Br | H | H | phenyl |
| **209** | Cl | Br | H | Me | phenyl |
| **210** | Cl | Br | H | H | 1-naphthyl |
| **211** | Cl | Br | H | H | 4-methylphenyl |
| **212** | Cl | Br | H | H | 4-chlorophenyl |
| **213** | Cl | Br | H | H | 3,4-dichlorophenyl |
| **214** | Cl | Br | H | H | 4-methoxyphenyl |
| **215** | Cl | Br | H | H | pyridine-3-yl |
| **216** | Cl | Br | H | H | pyridine-4-yl |
| **217** | Cl | Br | H | H | 2-chlorophenyl |
| **218** | Cl | Cl | H | H | phenyl |
| **219** | Cl | Br | H | H | 4-methoxy-3-hydroxyphenyl |
| **220** | Cl | Br | H | H | 2-methoxypyridin-5-yl |
| **221** | Cl | Br | H | H | 3,5-Dimethoxyphenyl |
| **222** | Cl | Br | H | H | pyrazin-2-yl |
| **224** | Cl | Br | H | H | 4-ethylphenyl |
| **225** | Cl | Br | H | H | 4-(1-(R or S)-1-methylprop-1-yl)phenyl |
| **226** | Cl | Br | H | H | 1-methyl-1*H*-pyrazol-3-yl |
| **227** | Cl | Br | H | H | 9H-fluoren-9-yl |
| **228** | Cl | Br | H | H | isoquinolin-1-yl |
| **229** | Cl | Br | H | H | isoquinolin-3-yl |
| **230** | Cl | Br | H | H | 4-phenylthiazol-2-yl |
| **231** | Cl | Br | H | H | 4-(4-pyridin-4-yl-piperazin-1-yl)phenyl |
| **232** | Cl | Br | H | H | 4-[1,4'-bipiperidin]-1'-yl-phenyl |
| **233** | Cl | Br | H | H | 1H-benzimidazol-2-yl |
| **234** | Cl | Br | H | H | benzothiazol-2-yl |
| **235** | Cl | Br | H | H | 4-(2-(4,5-dihydro-1H-imidazol-2- yl)ethyl)phenyl |
| **236** | Cl | Br | H | H | 4-(3-aminopropyl)phenyl |
| **237** | Cl | Br | H | H | 4-(2-aminoethyl)phenyl |
| **238** | F | Br | H | H | 4-[1,4'-bipiperidin]-1'-yl-phenyl |
| **239** | Cl | Br | H | H | 4-(2-(1,4,5,6-tetrahydropyrimidin-2- yl)ethyl)phenyl |
| **240** | Cl | Br | H | H | 4-(4,5-dihydro-1H-imidazol-2-yl)phenyl |
| **241** | Cl | Br | H | H | 4-fluoro-3-cyano-phenyl |
| **242** | Cl | Br | Me | H | 4-[1,4'-bipiperidin]-1'-yl-phenyl |
| **243** | Cl | Br | Me | H | 4-(4-pyridin-4-yl-piperazin-1-yl)phenyl |
| **244** | F | Me | *t*-butyl | H | 4-(2-cyanoethyl)phenyl |
| **245** | F | Me | *t*-butyl | H | 4-(2-(4,5-dihydro-1H-imidazol-2-yl)ethyl)phenyl |
| **246** | F | Me | H | H | 4-(2-(4,5-dihydro-1H-imidazol-2-yl)ethyl)phenyl |
| **247** | F | Me | phenyl | H | 4-(2-(4,5-dihydro-1H-imidazol-2-yl)ethyl)phenyl |
| **248** | F | Me | phenyl | H | 4-(2-cyanoethyl)phenyl |
| **249** | Cl | Br | Me | H | 4-(3-aminopropyl)phenyl |
| **250** | Cl | Br | Me | H | 4-(2-(4,5-dihydro-1H-imidazol-2-yl)ethyl)phenyl |

Particularly preferred compounds of the present invention include the following:
4-bromo-5-(2-chlorobenzoylamino)-1H-pyrazole-3-carboxylic acid (4-diethylaminophenyl)amide;
4 -bromo-5-(2-chlorobenzoylamino)-1H-pyrazole-3-carboxylic acid (4-dimethylaminophenyl)amide;
4-bromo-5-(2-chlorobenzoylamino)-1H-pyrazole-3-carboxylic acid o-tolylamide;
5-(2-chlorobenzoylamino)-4-methyl-1H-pyrazole-3-carboxylic acid phenylamide;
5-(2-chlorobenzoylamino)-4-ethyl-1H-pyrazole-3-carboxylic acid phenylamide;
5-(2-chlorobenzoylamino)-4-propyl-1H-pyrazole-3-carboxylic acid phenylamide;
4-bromo-5-(2-chlorobenzoylamino)-1H-pyrazole-3-carboxylic acid phenylamide;
4-bromo-5-(2-chlorobenzoylamino)-1H-pyrazole-3-carboxylic acid methylphenyl-amide;
4-bromo-5-(2-chlorobenzoylamino)-1H-pyrazole-3-carboxylic acid naphthalen-1-ylamide;
4-bromo-5-(2-chlorobenzoylamino)-1H-pyrazole-3-carboxylic acid p-tolylamide;
4-bromo-5-(2-chlorobenzoylamino)-1H-pyrazole-3-carboxylic acid (4-chlorophenyl)amide;
4-bromo-5-(2-chlorobenzoylamino)-1H-pyrazole-3-carboxylic acid (3,4-dichlorophenyl)amide;
4-bromo-5-(2-chlorobenzoylamino)-1H-pyrazole-3-carboxylic acid (4-methoxyphenyl)amide;
4-bromo-5-(2-chlorobenzoylamino)-1H-pyrazole-3-carboxylic acid pyridin-3-ylamide
4-bromo-5-(2-chlorobenzoylamino)-1H-pyrazole-3-carboxylic acid pyridin-4-ylamide
4-bromo-5-(2-chlorobenzoylamino)-1H-pyrazole-3-carboxylic acid (2-chlorophenyl)amide;
4-chloro-5-(2-chlorobenzoylamino)-1H-pyrazole-3-carboxylic acid phenylamide;
4-bromo-5-(2-chlorobenzoylamino)-1H-pyrazole-3-carboxylic acid (3-hydroxy-4-methoxyphenyl)amide;
4-bromo-5-(2-chlorobenzoylamino)-1H-pyrazole-3-carboxylic acid (6-methoxypyridin-3-yl)amide;
4-bromo-5-(2-chlorobenzoylamino)-1H-pyrazole-3-carboxylic acid (3,5-dimethoxyphenyl)amide;
4-bromo-5-(2-chlorobenzoylamino)-1H-pyrazole-3-carboxylic acid pyrazin-2-ylamide;
4-bromo-5-(2-chlorobenzoylamino)-2H-pyrazole-3-carboxylic acid (4-ethylphenyl)amide;
4-bromo-5-(2-chlorobenzoylamino)-2H-pyrazole-3-carboxylic acid (4-sec-butylphenyl)amide;
4-bromo-5-(2-chlorobenzoylamino)-1H-pyrazole-3-carboxylic acid (1-methyl-1H-pyrazol-3-yl)amide;
4-bromo-5-(2-chlorobenzoylamino)-1 H-pyrazole-3-carboxylic acid (9H-fluoren-9-yl)amide;
4-bromo-5-(2-chlorobenzoylamino)-1H-pyrazole-3-carboxylic acid isoquinolin-1-ylamide;
4-bromo-5-(2-chlorobenzoylamino)-1H-pyrazole-3-carboxylic acid isoquinolin-3-ylamide;
4-bromo-5-(2-chlorobenzoylamino)-1H-pyrazole-3-carboxylic acid (4-phenylthiazol-2-yl)amide;
4-bromo-5-(2-chlorobenzoylamino)-1H-pyrazole-3-carboxylic acid [4-(4-pyridin-4-yl-piperazin-1-yl)phenyl]amide;
4-bromo-5-(2-chlorobenzoylamino)-1H-pyrazole-3-carboxylic acid (4-[1,4'-bipiperidin]-1'-yl-phenyl)amide;
4-bromo-5-(2-chlorobenzoylamino)-1H-pyrazole-3-carboxylic acid (1H-benzimidazol-2-yl)amide; and
4-bromo-5-(2-chlorobenzoylamino)-1H-pyrazole-3-carboxylic acid benzothiazol-2-ylamide;
4-bromo-5-(2-chlorobenzoylamino)-1H-pyrazole-3-carboxylic acid (4-(2-(4,5-dihydro-1 H-imidazol-2-yl)ethyl)phenyl)amide;
4-bromo-5-(2-chloroben2oylamino)-1H-pyrazole-3-carboxylic acid (4-(3-aminopropyl)phenyl)amide;
4-bromo-5-(2-chlorobenzoylamino)-1H-pyrazole-3-carboxylic acid (4-(2-aminoethyl)phenyl)amide;
4-bromo-5-(2-fluorobenzoylami.no)-1H-pyrazole-3-carboxylic acid (4-[1,4'-bipiperidin]-1'-yl-phenyl)amide;
4-bromo-5-(2-chlorobenzoylamino)-1H-pyrazole-3-carboxylic acid (4-(2-(1,4,5,6-tetrahydropyrimidin-2-yl)ethyl)phenyl)amide;
4-bromo-5-(2-chlorobenzoylamino)-1H-pyrazole-3-carboxylic acid (4-(4,5-dihydro-1H-imidazol-2-yl)phenyl)amide;
4-bromo-5-(2-chlorobenzoylamino)-1H-pyrazole-3-carboxylic acid (4-fluoro-3-cyanophenyl)amide;
4-bromo-5-(2-chlorobenzoylamino)-1-methyl-pyrazole-3-carboxylic acid (4-[1,4'-bipiperidin]-1'-yl-phenyl)amide;
4-bromo-5-(2-chlorobenzoylamino)-1-methyl-pyrazole-3-carboxylic acid [4-(4-pyridine-4-yl-piperazin-1-yl)phenyl]amide;
4-methyl-5-(2-fluorobenzoylamino)-1-t-butyl-pyrazole-3-carboxylic acid (4-(2-cyanoethyl)phenyl)amide;
4-methyl-5-(2-fluorobenzoylamino)-1-t-butyl-pyrazole-3-carboxylic acid (4-(2-(4,5-dihydro-1H-imidazol-2-yl)ethyl)phenyl)amide;
4-methyl-5-(2-fluorobenzoylamino)-1H-pyrazole-3-carboxylic acid (4-(2-(4,5-dihydro- 1H-imidazol-2-yl)ethyl)phenyl)amide;
4-methyl-5-(2-fluorobenzoylamino)-1-phenyl-pyrazole-3-carboxylic acid (4-(2-(4,5-dihydro-1H-imidazol-2-yl)ethyl)phenyl)amide;
4-methyl-5-(2-fluorobenzoylamino)-1-phenyl-pyrazole-3-carboxylic acid (4-(2-cyanoethyl)phenyl)amide;
4-bromo-5-(2-chlorobenzoylamino)-1-methyl-pyrazole-3-carboxylic acid (4-(3-aminopropyl)phenyl)amide;
4-bromo-5-(2-chlorobenzoylamino)-1-methyl-pyrazole-3-carboxylic acid (4-(2-(4,5-dihydro-1H-imidazol-2-yl)ethyl)phenyl)amide;
and pharmaceutically acceptable salts thereof.

The following compounds are not included in the present invention

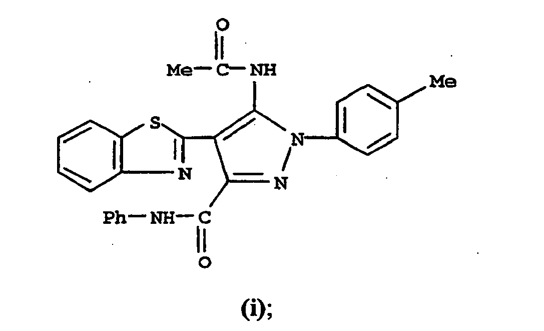

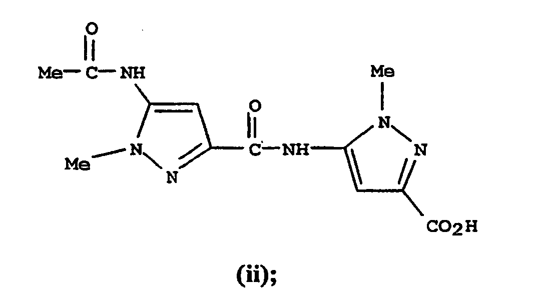

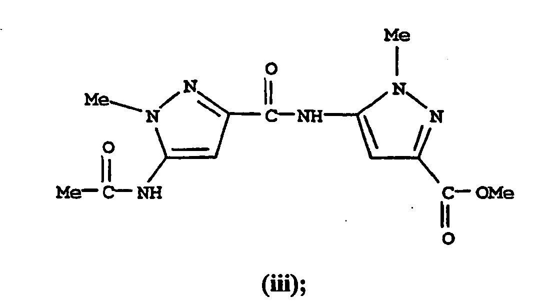

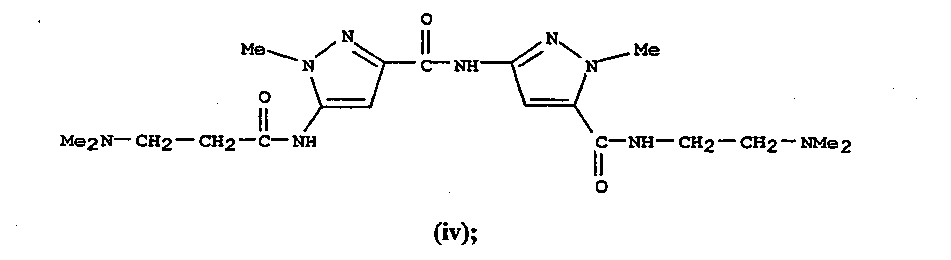

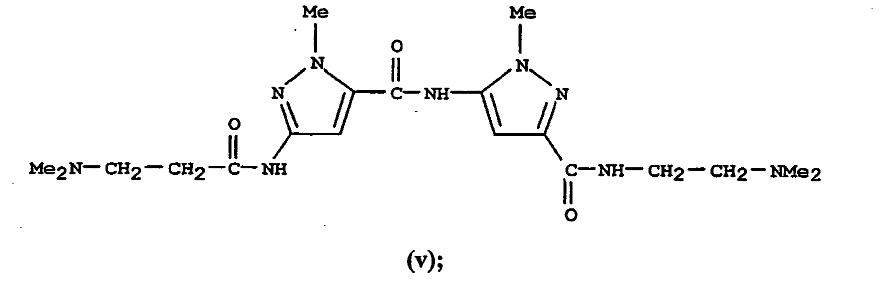

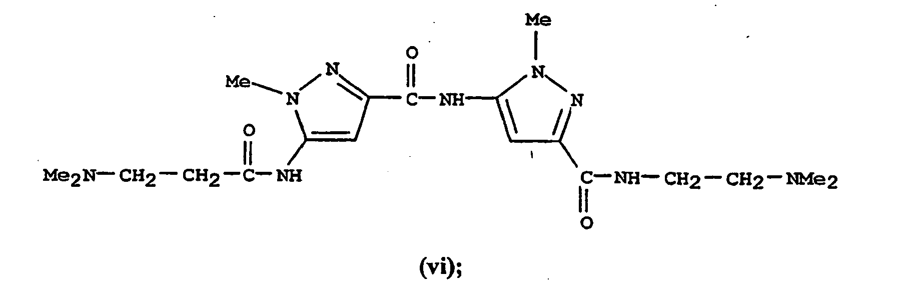

Compounds of Formula (I) and Formula (II) can be employed as selective antagonists of the bradykinin B₁ over the bradykinin B₂ receptor.

The present invention also provides a selective antagonist of bradykinin B₁ receptor over bradykinin B₂ receptor that is a compound of Formula (I) or Formula (II).

Described herein is a method for selectively inhibiting bradykinin B₁ receptor over bradykinin B₂ receptor in a biological sample comprising both the bradykinin B₁ and B₂ receptors which method comprises contacting an inhibiting effective amount of a compound of Formula (I) or Formula (II) or mixture thereof to the biological sample.

The present invention further provides a pharmaceutical composition comprising a pharmaceutically acceptable carrier and an amount of a compound of Formula (I) or Formula (II) or mixtures thereof effective to treat or palliate adverse symptoms in mammals mediated by bradykinin B₁ receptor.

Described herein is a method for treating or palliating adverse symptoms in a mammal mediated at least in part by bradykinin B₁ receptor which method comprises administering a therapeutically effective amount of a compound of Formula (I) or Formula (II) or mixtures thereof or as is more generally the case the pharmaceutical composition.

The present invention further provides a pharmaceutical composition comprising a pharmaceutically acceptable carrier and a therapeutically effective amount of a compound of Formula (I) or Formula (II) or mixtures thereof to treat or palliate adverse symptoms in a mammal associated with up-regulating Bradykinin B₁ receptor following tissue damage or inflammation.

Described herein is a method for treating or palliating adverse symptoms in a mammal associated with tissue damage or inflammation which method comprises administering a therapeutically effective amount of a compound of Formula (I) or Formula (II) or mixtures thereof or as is more generally the case the pharmaceutical composition.

Described herein is a method for treating or palliating adverse symptoms associated with the presence or secretion of bradykinin B₁ receptor agonists in a mammal which method comprises administering a therapeutically effective amount of a compound of Formula (I) or Formula (II) or mixtures thereof or as is more generally the case the pharmaceutical composition.

Described herein is a method for treating or ameliorating pain, inflammation, septic shock or the scarring process in a mammal mediated at least in part by bradykinin B₁ receptor which method comprises administering a therapeutically effective amount of a compound of Formula (I) or Formula (II) or mixtures thereof or as is more generally the case the pharmaceutical composition.

Described herein is a method for treating or ameliorating adverse symptoms in a mammal associated with burns, perioperative pain, migraine, shock, central nervous system injury, asthma, rhinitis, premature labor, inflammatory arthritis, inflammatory bowel disease or neuropathic pain which method comprises administering a therapeutically effective amount of a compound of Formula (I) or Formula (II) or mixtures thereof or as is more generally the case the pharmaceutical composition.

Described herein is a method for treating or palliating adverse symptoms associated with the presence or secretion of bradykinin B₁ receptor agonists in a mammal which method comprises administering a therapeutically effective amount of a compound of Formula (I) or Formula (II) or mixtures thereof or as is more generally the case the pharmaceutical composition.

Described herein is a method for determining bradykinin B₁ receptor agonist levels in a biological sample which method comprises contacting said biological sample with a compound of Formula (I) or Formula (II), at a predetermined concentration.

### DETAILED DESCRIPTION OF THE PREFERRED EMBODIMENTS

As noted above, this invention is directed to certain 3-amid-5-substituted pyrazole derivatives and related compounds which are useful as bradykinin B₁ receptor antagonists to relieve adverse symptoms in mammals mediated, at least in part, by Bradykinin B₁ receptor including pain, inflammation, septic shock, the scarring process, etc. However, prior to describing this invention in further detail, the following terms will first be defined.

### Definitions

Unless otherwise expressly defined with respect to a specific occurrence of the term, the following terms as used herein shall have the following meanings regardless of whether capitalized or not:

The term "alkyl" or "alk" refers to monovalent alkyl groups having from 1 to 15 carbon atoms and more preferably 1 to 6 carbon atoms and includes both straight chain and branched chain alkyl groups. This term is exemplified by groups such as methyl, t-butyl, n-heptyl, octyl and the like. The term C₁₋₄alkyl refers to alkyl groups with from 1 to 4 carbon atoms.

The term "substituted alkyl" refers to an alkyl group, of from 1 to 15 carbon atoms, preferably, 1 to 6 carbon atoms, having from 1 to 5 substituents, preferably 1 to 3 substituents, independently selected from the group consisting of alkoxy, substituted alkoxy, acyl, acylamino, thiocarbonylamino, acyloxy, amino, substituted amino, amidino, alkylamidino, thioamidino, aminoacyl, aminocarbonylamino, aminothiocarbonylamino, aminocarbonyloxy, aryl, substituted aryl, aryloxy, substituted aryloxy, aryloxylaryl, substituted aryloxyaryl, cyano, halogen, hydroxyl, nitro, oxo, thioxo, carboxyl, carboxyl esters, cycloalkyl, substituted cycloalkyl, guanidino, guanidinosulfone, thiol, thioalkyl, substituted thioalkyl, thioaryl, substituted thioaryl, thiocycloalkyl, substituted thiocycloallcyl, thioheteroaryl, substituted thioheteroaryl, thioheterocyclic, substituted thioheterocyclic, heteroaryl, substituted heteroaryl, heterocyclic, substituted heterocyclic, cycloalkoxy, substituted cycloalkoxy, heteroaryloxy, substituted heteroaryloxy, heterocyclyloxy, substituted heterocyclyloxy, oxycarbonylamino, oxythiocarbonylamino, -OS(O)₂-alkyl, -OS(O)₂-substituted alkyl, -OS(O)₂-aryl, -OS(O)₂-substituted aryl, -OS(O)₂-heteroaryl, -OS(O)₂-substituted heteroaryl, -OS(O)₂-heterocyclic, -OS(O)₂-substituted heterocyclic, -OSO₂NR⁴⁰R⁴⁰ where each R⁴⁰ is hydrogen or alkyl, -NR⁴⁰S(O)₂-alkyl, -NR⁴⁰S(O)₂-substituted alkyl,-NR⁴⁰S(O)₂-aryl, -NR⁴⁰S(O)₂-substituted aryl, -NR⁴⁰S(O)₂-heteroaryl, - NR⁴⁰S(O)₂-substituted heteroaryl, -NR⁴⁰S(O)₂-heterocyclic, -NR⁴⁰S(O)₂-substituted heterocyclic, -NR⁴⁰S(O)₂-NRa⁴⁰-alkyl, -NR40S(O)₂-NR⁴⁰-substituted alkyl, -NR⁴⁰S(O)₂-NR⁴⁰-aryl, -NR⁴⁰S(O)₂-NR⁴⁰ -substituted aryl, -NR⁴⁰S(O)₂-NR⁴⁰ -heteroaryl, -NR⁴⁰S(O)₂-NR⁴⁰-substituted heteroaryl, -NR⁴⁰S(O)₂-NR⁴⁰-heterocyclic, and -NR⁴⁰S(O)₂-NR⁴⁰-substituted heterocyclic where each R⁴⁰ is hydrogen or alkyl.

"Alkoxy" refers to the group "alkyl-O" which includes, by way of example, methoxy, ethoxy, n-propoxy, isopropoxy, n-butoxy, tert-butoxy, sec-butoxy, *n-*pentoxy, n-hexoxy, 1,2-dimethylbutoxy, and the like.

"Substituted alkoxy" refers to the group "substituted akyl-O-".

"Acyl" refers to the groups H-C(O)-, alkyl-C(O)-, substituted alkyl-C(O)-, alkenyl-C(O)-, substituted alkenyl-C(O)-, alkynyl-C(O)-, substituted alkynyl-C(O)-, cycloalkyl-C(O)-, substituted cycloalkyl-C(O)-, aryl-C(O)-, substituted aryl-C(O)-, heteroaryl-C(O)-, substituted heteroaryl-C(O), heterocyclic-C(O)-, and substituted heterocyclic-C(O)- provided that a nitrogen atom of the heterocyclic or substituted heterocyclic is not bound to the -C(O)- group wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Amino" refers to the group -NH₂.

"Substituted amino" refers to the group -NR⁴¹R⁴¹, where each R⁴¹ group is independently selected from the group consisting of hydrogen, alkyl, substituted alkyl, alkenyl, substituted alkenyl, alkynyl, substituted alkynyl, cycloalkyl, substituted cycloallcyl, aryl, substituted aryl, heteroaryl, substituted heteroaryl, heterocyclic, substituted heterocyclic, -SO₂-alkyl, -SO₂-substituted alkyl, -SO₂-alkenyl -SO₂-substitute alkenyl, -SO₂-cycloalkyl, -SO₂-substituted cycloalkyl, -SO₂-aryl -SO₂-substitute aryl, -SO₂-heteroaryl, -SO₂-substituted heteroaryl, -SO₂-heterocyclic -SO₂-substituted heterocyclic, provided that both R⁴¹ groups are not hydrogen; or the R⁴¹ groups can be joined together with the nitrogen atom to form a heterocyclic or substituted heterocyclic ring.

The "acylamino" or as a prefix "carbamoyl" or "carboxamide" or "substituted carbamoyl" or "substituted carboxamide" refers to the group -C(O)NR⁴²R⁴² where each R⁴² is independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, substituted heterocyclic and where each R⁴² is joined to form together with the nitrogen atom a heterocyclic or substituted heterocyclic wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Thiocarbonylamino" or as a prefix "thiocarbamoyl", "thiocarboxamide" or "substituted thiocarbamoyl" or "substituted thiocarboxamide" refers to the group -C(S)NR⁴³R⁴³ where each R⁴³ is independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, substituted heterocyclic and where each R⁴³ is joined to form, together with the nitrogen atom a heterocyclic or substituted heterocyclic wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Acyloxy" refers to the groups acyl-O- where acyl is as defined herein.

"Alkenyl" refers to alkenyl group having from 2 to 10 carbon atoms and more preferably 2 to 6 carbon atoms and having at least 1 and preferably from 1-2 sites of alkenyl unsaturation.

"Substituted alkenyl" refers to alkenyl groups as defined herein, having from 1 to 5 substituents, preferably 1 to 3 substituents, independently selected from the group of substituents defined for substituted alkyl provided that the hydroxyl, thio, oxo or thioxo groups are not attached to a vinyl carbon atom.

"Alkynyl" refers to alkynyl group having from 2 to 10 carbon atoms and more preferably 3 to 6 carbon atoms and having at least 1 and preferably from 1-2 sites of alkynyl unsaturation.

"Substituted alkynyl" refers to alkynyl groups, as defined herein, having from 1 to 5, preferably 1 to 3 substituents, selected from the same group of substituents as defined for substituted alkyl provided that the hydroxyl, thio, oxo or thioxo groups are not attached to a vinyl carbon atom.

"Amidino" refers to the group H₂NC(=NH)- and the term "alkylamidino" refers to compounds having 1 to 3 alkyl groups (e.g., alkylHNC(=NH)-).

"Thioamidino" refers to the group R⁴⁴SC(=NH)- where R⁴⁴ is hydrogen or alkyl where alkyl is as defined herein.

"Aminoacyl" refers to the groups NR⁴⁵C(O)alkyl, -NR⁴⁵C(O)substituted alkyl, -NR⁴⁵C(O)cycloalkyl, -NR⁴⁵C(O)substituted cycloalkyl, -NR⁴⁵C(O)alkenyl, -NR⁴⁵C(O)substituted alkenyl, -NR⁴⁵C(O)alkynyl, -NR⁴⁵C(O)substituted alkynyl, -NR⁴⁵C(O)aryl, -NR⁴⁵C(O)substituted aryl, -NR⁴⁵C(O)heteroaryl, -NR⁴⁵C(O)substituted heteroaryl, -NR⁴⁵C(O)heterocyclic, and NR⁴⁵C(O)substituted heterocyclic where R⁴⁵ is hydrogen or alkyl and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are defined herein.

"Aminocarbonyloxy" refers to the groups -NR⁴⁶C(O)O-alkyl, -NR⁴⁶C(O)O-substituted alkyl, -NR⁴⁶C(O)O-alkenyl, -NR⁴⁶C(O)O-substituted alkenyl, -NR⁴⁶C(O)O-alkynyl, -NR⁴⁶C(O)O-substituted alkynyl, -NR⁴⁶C(O)O-cycloalkyl, -NR⁴⁶C(O)O-substituted cycloalkyl, -NR⁴⁶C(O)O-aryl, -NR⁴⁶C(O)O-substituted aryl, -NR⁴⁶C(O)O-heteroaryl, -NR⁴⁶C(O)O-substituted heteroaryl, -NR⁴⁶C(O)O-heterocyclic, and -NR⁴⁶C(O)O-substituted heterocyclic where R⁴⁶ is hydrogen or alkyl and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Oxycarbonylamino" or as a prefix "carbamoyloxy" or "substituted carbamoyloxy" refers to the groups -OC(O)NR⁴⁷R⁴⁷ where each R⁴⁷ is independently hydrogen, alkyl, substituted alkyl, alkenyl, substituted alkenyl, alkynyl, substituted alkynyl, cycloalkyl, substituted cycloalkyl, aryl, substitute aryl, heteroaryl, substituted heteroaryl, heterocyclic, and substituted heterocyclic or where each R⁴⁷ is joined to form, together with the nitrogen atom a heterocyclic or substituted heterocyclic and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Oxythiocarbonylamino" refers to the groups -OC(S)NR⁴⁸R⁴⁸ where each R⁴⁸ is independently hydrogen, alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic, and substituted heterocyclic or where each R⁴⁸ is joined to form, together with the nitrogen atom a heterocyclic or substituted heterocyclic and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Aminocarbonylamino" refers to the group NR⁴⁹C(O)NR⁴⁹- where R⁴⁹ is selected from the group consisting of hydrogen and alkyl.

"Aminothiocarbonylamino" refers to the group -NR⁵⁰C(S)NR⁵⁰- where R⁵⁰ is selected from the group consisting of hydrogen and alkyl.

"Aryl" or "Ar" refers to an aromatic carbocyclic group of from 6 to 14 carbon atoms having a single ring (i.e., monocyclic) (e.g., phenyl) or multiple condensed rings (e.g., naphthyl or anthryl) which condensed rings may or may not be aromatic (e.g., 2-benzoxazolinone, 2H-1,4-benzoxazin-3(4H)-one, and the like). When at least one of the rings in the fused multicyclic ring system is non-aromatic, the point of attachment of the aryl group to the core structure is on one of the aromatic rings. Preferred aryls include phenyl and naphthyl.

"Substituted aryl" refers to aryl groups, as defined herein, which are substituted with from 1 to 4, preferably 1-3, substituents selected from the group consisting of hydroxy, acyl, acylamino, thiocarbonylamino, acyloxy, alkyl, substituted alkyl, alkoxy, substituted alkoxy, alkenyl, substituted alkenyl, alkynyl, substituted alkynyl, amidino, alkylamidino, thioamidino, amino, substituted amino, aminoacyl, aminocarbonyloxy, aminocarbonylamino, aminothiocarbonylamino, aryl, substituted aryl, aryloxy, substituted aryloxy, cycloalkoxy, substituted cycloalkoxy, heteroaryloxy, substituted heteroaryloxy, heterocyclyloxy, substituted heterocyclyloxy, carboxyl, carboxyl esters cyano, thiol, thioalkyl, substituted thioalkyl, thioaryl, substituted thioaryl, thioheteroaryl, substituted thioheteroaryl, thiocycloalkyl, substituted thiocycloalkyl, thioheterocyclic, substituted thioheterocyclic, cycloalkyl, substituted cycloalkyl, guanidino, guanidinosulfone, halo, nitro, heteroaryl, substituted heteroaryl, heterocyclic, substituted heterocyclic, oxycarbonylamino, oxythiocarbonylamino, -S(O)₂-alkyl, -S(O)₂-substituted alkyl, -S(O)₂-cycloalkyl, -S(O)₂-substituted cycloalkyl, -S(O)₂-alkenyl, -S(O)₂-substituted alkenyl, -S(O)₂-aryl, -S(O)₂-substituted aryl, -S(O)₂-heteroaryl, -S(O)₂-substituted heteroaryl, -S(O)₂-heterocyclic, -S(O)₂-substituted heterocyclic, -OS(O)₂-alkyl, -OS(O)₂-substituted alkyl, -OS(O)₂-aryl,-OS(O)₂-substituted aryl, -OS(O)₂-heteroaryl, -OS(O)₂-substituted heteroaryl, -OS(O)₂-heterocyclic, -OS(O)₂-substituted heterocyclic, -OSO₂-NR⁵¹R⁵¹ where each R⁵¹ is hydrogen or alkyl, -NR⁵¹S(O)₂-alkyl, -NR⁵¹S(O)₂-substituted alkyl, -NR⁵¹S(O)₂-aryl, -NR⁵¹S(O)₂-substituted aryl, -NR⁵¹S(O)₂-heteroaryl, -NR⁵¹S(O)₂-substituted heteroaryl, -NR⁵¹S(O)₂-heterocyclic, -NR⁵¹S(O)₂-substituted heterocyclic, -NR⁵¹S(O)₂-NR⁵¹-alkyl, -NR⁵¹S(O)₂-NR⁵¹-substituted alkyl, -NR⁵¹S(O)₂-NR⁵¹-aryl, -NR⁵¹S(O)₂-NR⁵¹-substituted aryl, -NR⁵¹S(O)₂-NR⁵¹-heteroaryl, -NR⁵¹S(O)₂-NR⁵¹-substituted heteroaryl, -NR⁵¹S(O)₂-NR⁵¹-heterocyclic, -NR⁵¹S(O)₂-NR⁵¹-substituted heterocyclic where each R⁵¹ is hydrogen or alkyl, wherein each of the terms is as defined herein.

"Aryloxy" refers to the group aryl-O- which includes, by way of example, phenoxy, naphthoxy, and the like.

"Substituted aryloxy" refers to substituted aryl-O- groups.

"Aryloxyaryl" refers to the group -aryl-O-aryl.

"Substituted aryloxyaryl" refers to aryloxyaryl groups substituted with from 1 to 4, preferably 1-3 substituents on either or both aryl rings independently selected from the same group consisting of substituents as defined for substituted aryl.

"Carboxyl" refers to the group -COOH and pharmaceutically acceptable salts thereof.

"Carboxyl esters" refer to any one of the following esters: -COO-alkyl, -COO-substituted alkyl, -COO-cycloalkyl, -COO-substituted cycloalkyl, -COO-aryl, -COO-substituted aryl, -COO-hetereoaryl, -COO-substituted heteroaryl, -COO-hetereocyclic, and -COO-substituted heterocyclic.

"Cycloalkyl" refers to cyclic alkyl groups of from 3 to 20 carbon atoms having a single or multiple cyclic rings including, by way of example, cyclopropyl, cyclobutyl, cyclopentyl, cyclooctyl, adamantanyl, and the like. Cycloalkyl groups of the present invention also include fused multicyclic rings wherein one or more of the rings within the multicyclic ring system are aryl, cycloalkenyl, heteroaryl, and/or heterocyclic, as long as the point of attachment to the core or backbone of the structure is on the non-aromatic cycloalkyl ring.

"Cycloalkenyl" refers to cyclic alkenyl groups of from 4 to 20 carbon atoms having single or multiple unsaturation and having a single or multiple cyclic unsaturated but not aromatic rings. Suitable cycloalkenyl groups include, by way of example, cyclopentenyl, cyclooctenyl, and the like. Cycloalkenyl groups of the present invention also include fused multicyclic rings wherein one or more of the rings within the multicyclic ring system are aryl, heteroaryl, cycloalkyl and/or heterocyclic, as long as the point of attachment to the core or backbone of the structure is on the non-aromatic cycloalkenyl ring.

"Substituted cycloalkyl" and "substituted cycloalkenyl" refer to a cycloalkyl and cycloalkenyl groups, as defined herein, having from 1 to 5, preferably 1-3 substituents independently selected from the same group of substituents as defined for substituted alkyl.

"Cycloallcoxy" refers to -O-cycloalkyl groups where cycloalkyl is as defined herein.

"Substituted cycloalkoxy" refers to -O-substituted cycloalkyl groups where substituted cycloalkyl is as defined herein.

"Guanidino" or "substituted guanidino" refers to the groups -NR⁵²C(=NR⁵²)NR⁵²R⁵² where each R⁵² is independently hydrogen or alkyl.

"Guanidinosulfone" refers to the groups -NR⁵³C(=NR⁵³)NRSO₂-alkyl, -NR⁻⁵³C(=NR¹³)NR⁵³SO₂-substituted alkyl, -NR⁵³C(=NR⁵³)NR⁵³SO₂-alkenyl, -NR⁵³C(=NR⁵³)NR⁵³SO₂-substituted alkenyl, -NR⁵³C(=NR⁵³)NR⁵³SO₂-alkenyl, -NR⁵³C(=NR⁵³)NR⁵³SO₂-substituted alkenyl, -NR⁵³C(=NR⁵³)NR⁵³SO₂-aryl, -NR⁵³C(-NR⁵³)NR⁵³SO₂-substituted aryl, -NR⁵³C(=NR⁵³)NR⁵³SO₂-cycloalkyl, -NR⁵³C(=NR⁵³)NR⁵³SO₂-substituted cycloalkyl, -NR⁵¹C(-NR⁵³)NR⁵³SO₂-heteroaryl, -NR⁵³C(=NR⁵³)NR⁵³SO₂-substituted heteroaryl, -NR⁵³C(=NR⁵³)NR⁵³SO₂-heterocyclic, and -NR⁵³C(=NR⁵³)NR⁵³ SO₂-substituted heterocyclic where each R⁵³ is independently hydrogen and alkyl.

"Halo" or "halogen" refers to fluoro, chloro, bromo and iodo.

"Heteroaryl" refers to an aromatic group of from 2 to 10 ring carbon atoms and 1 to 4 ring heteroatoms selected from oxygen, nitrogen and sulfur within the ring. Such heteroaryl groups can have a single ring (i.e., monocyclic) (e.g., pyridyl or furyl) or multiple condensed rings (e.g., indolizinyl or benzothienyl) which may be non-heteroaryl. When at least one of the rings in the fused multicyclic ring system is non-heteroaryl such as aryl, cycloalkyl, cycloalkenyl, or heterocyclic, the point of attachment of the heteroaryl group to the core structure is on one of the heteroaryl atoms. Preferred heteroaryls include pyridyl, pyrrolyl, indolyl and furyl.

"Substituted heteroaryl" refers to heteroaryl groups, as defined above, which are substituted with from 1 to 3 substituents independently selected from the same group of substituents as defined for "substituted aryl".

"Heteroaryloxy" refers to the group -O-heteroaryl and "substituted heteroaryloxy" refers to the group -O-substituted heteroaryl where heteroaryl and substituted heteroaryl are as defined above.

"Heterocycle" or "heterocyclic" refers to a saturated or unsaturated, but not heteroaromatic, group having a single ring or multiple condensed rings, from 2 to 20 ring carbon atoms and from 1 to 4 ring hetero atoms selected from nitrogen, sulfur or oxygen within the ring. "Heterocycle" or "heterocyclic" groups of the present invention also include fused multicyclic rings wherein one or more of the rings within the multicyclic ring system is not heterocyclic (e.g., cycloalkyl, cycloalkenyl, aryl or heteroaryl), as long as the point of attachment to the core or backbone of the structure is on the heterocyclic ring.

"Substituted heterocyclic" refers to heterocycle groups, as defined above, which are substituted with from 1 to 3 substituents independently selected from the group consisting of oxo (=O), thioxo (=S), plus the same group of substituents as defined for substituted aryl.

Examples of heterocycles and heteroaryls include, but are not limited to, azetidine, pyrrole, imidazole, pyrazole, pyridine, pyrazine, pyrimidine, pyridazine, indolizine, isoindole, indole, dihydroindole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, phthalimide, 1,2,3,4-tetrahydro-isoquinoline, 4,5,6,7-tetrahydrobenzo[b]thiophene, thiazole, thiazolidine, thiophene, benzo[b]thiophene, morpholino, thiomorpholino, piperidinyl, pyrrolidine, tetrahydrofuranyl, and the like.

"Heterocyclyloxy" refers to the group -O-heterocyclic and "substituted heterocyclyloxy" refers to the group -O-substituted heterocyclic where heterocyclic and substituted heterocyclyoxy are as defined above.

"Thiol" refers to the group -SH.

"Thioalkyl" or "thioalkoxy" refer to the groups -S-alkyl.

"Substituted thioalkyl," and "substituted thioalkoxy"refer to the group -S-substituted alkyl.

"Thiocycloalkyl" refers to the groups -S-cycloalkyl.

"Substituted thiocycloalkyl" refers to the group -S-substituted cycloalkyl.

"Thioaryl" refers to the group -S-aryl and "substituted thioaryl" refers to the group -S-substituted aryl.

"Thioheteroaryl" refers to the group -S-heteroaryl and "substituted thioheteroaryl" refers to the group -S-substituted heteroaryl.

"Thioheterocyclic" refers to the group -S-heterocyclic and "substituted thioheterocyclic" refers to the group -S-substituted heterocyclic.

Amino acid refers to any of the naturally occurring amino acids, as well as synthetic analogs (e.g., D-stereoisomers of the naturally occurring amino acids, such as D-threonine) and derivatives thereof. α-Amino acids comprise a carbon atom to which is bonded an amino group, a carboxyl group, a hydrogen atom, and a distinctive group referred to as a "side chain". The side chains of naturally occurring amino acids are well known in the art and include, for example, hydrogen (e.g., as in glycine), alkyl (e.g., as in alanine, valine, leucine, isoleucine, proline), substituted alkyl (e.g., as in threonine, serine, methionine, cysteine, aspartic acid, asparagine, glutamic acid, glutamine, arginine, and lysine), arylalkyl (e.g., as in phenylalanine and tryptophan), substituted arylalkyl (e.g., as in tyrosine), and heteroarylalkyl (e.g., as in histidine). The term "naturally occurring amino acids" refers to these amino acids.

Unnatural amino acids are also known in the art, as set forth in, for example, Williams (ed.), Synthesis of Optically Active alpha.-Amino Acids, Pergamon Press (1989); Evans et al., J. Amer. Chem. Soc., 112:4011-4030 (1990); Pu et al., J. Org Chem., 56:1280-1283 (1991); Williams et al., J. Amer. Chem. Soc., 113:9276-9286 (1991); and all references cited therein.

"Pharmaceutically acceptable salt" refers to pharmaceutically acceptable salts of a compound of Formula (I) or Formula (II) which salts are derived from a variety of organic and inorganic counter ions well known in the art and include, by way of example only, sodium, potassium, calcium, magnesium, ammonium, tetraalkylammonium, and the like; and when the molecule contains a basic functionality, salts of organic or inorganic acids, such as hydrochloride, hydrobromide, tartrate, mesylate, acetate, maleate, oxalate and the like.

It is understood that the substitution patterns defined herein do not include any chemically impossible substitution patterns. Moreover, when a group is defined by the term "substituted" such as substituted aryl and a possible substituent is the substituted group itself, e.g., substituted aryl substituted with substituted aryl, it is not intended that such substitution patterns be repeated indefinitely such as to produce a polymer,e.g., (substituted aryl)ₙ. Accordingly, in all cases, the maximum number of repetitions is 4. That is too say that n is an integer from 1 to 4.

### Compound Preparation

The compounds of this invention can be prepared from readily available starting materials using the following general methods and procedures. It will be appreciated that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. Suitable protecting groups for various functional groups as well as suitable conditions for protecting and deprotecting particular functional groups are well known in the art. For example, numerous protecting groups are described in T. W. Greene and G. M. Wuts, Protecting Groups in Organic Synthesis, Third Edition, Wiley, New York, 1999, and references cited therein.

The compounds of this invention will typically contain one or more chiral centers. Accordingly, if desired, such compounds can be prepared or isolated as pure stereoisomers, i.e., as individual enantiomers or diastereomers, or as stereoisomer-enriched mixtures. All such stereoisomers (and enriched mixtures) are included within the scope of this invention, unless otherwise indicated. Pure stereoisomers (or enriched mixtures) may be prepared using, for example; optically active starting materials or stereoselective reagents well-known in the art. Alternatively, racemic mixtures of such compounds can be separated using, for example, chiral column chromatography, chiral resolving agents and the like.

The starting materials for the following reactions are generally know compounds or can be prepared by known procedures or obvious modifications thereof. For example, many of the starting materials are available from commercial suppliers such as Aldrich Chemical Co. (Milwaukee, Wisconsin, USA), Bachem (Torrance, California, USA), Emka-Chemce or Sigma (St. Louis, Missouri, USA). Others may be prepared by procedures, or obvious modifications thereof, described in standard reference texts such as Fieser and Fieser's Reagents for Organic Synthesis, Volumes 1-15 (John Wiley and Sons, 1991), Rodd's Chemistry of Carbon Compounds, Volumes 1-5 and Supplementals (Elsevier Science Publishers, 1989), Organic Reactions, Volumes 1-40 (John Wiley and Sons, 1991), March's Advanced Organic Chemistry, (John Wiley and Sons, 4th Edition), and Larock's Comprehensive Organic Transformations (VCH Publishers Inc., 1989).

Specifically, the substituted pyrazoles and various intermediates useful in the preparation of substituted pyrazoles are preferably prepared as shown in the following Schemes.

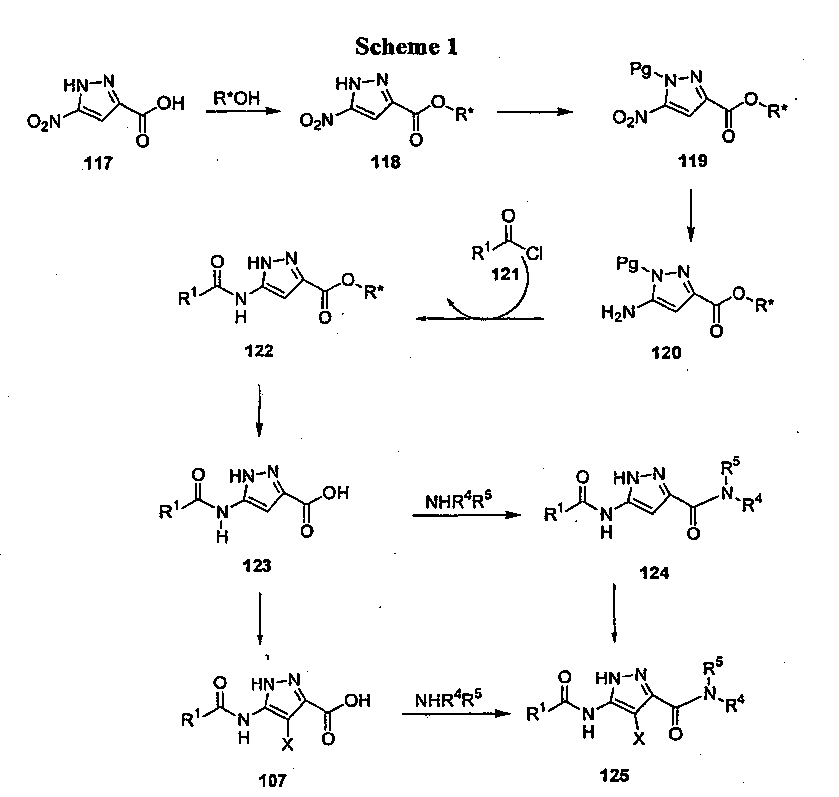

Specifically, in Scheme 1 (where R¹, R³, R⁴, R⁵, X, are as defined above and R* is alkyl), commercially available 3-nitro-5-carboxyl pyrazole, compound **117,** is esterified under conventional conditions using a suitable alcohol, such as methanol, in acidic medium to provide for the corresponding ester, compound **118.** The particular alcohol employed is not critical and is typically selected based on ease of synthesis and costs. The reaction is preferably conducted at an elevated temperature of from about 25 to about 100 °C until the reaction is substantially complete, which is typically 2 to 12 hours. The resulting product, compound **118,** can be recovered by conventional methods, such as chromatography, filtration, crystallization, and the like or, alternatively, used in the next step without purification and/or isolation.

The 3-nitro-5-carboxyl ester pyrazole, compound **118,** is protected with a conventional protecting group, Pg, under conventional conditions. The selected protecting group is one that is removed under conditions other than hydrogenation. A preferred protecting group is the Boc group.

The nitro group of the protected 3-nitro-5-carboxyl ester pyrazole, compound **119,** is reduced to an amine using standard reduction reactions. For example, compound **119** is reacted with hydrogen gas at about 10 to 60 psi, in the presence of a suitable catalyst such as Pd on carbon to afford the corresponding amine, compound 120. The reaction is preferably conducted at a temperature of from about 20 to about 80 °C until the reaction is substantially complete, which is typically 1 to 5 hours. The resulting amine, compound 120, can be recovered by conventional methods, such as chromatography, filtration, crystallization, and the like or, alternatively, used in the next step without purification and/or isolation.

The 3-amino-5-carboxyl ester pyrazole, compound **120,** is acylated under conventional conditions by reaction with at least a stoichiometric amount and preferably an excess of a desired acyl chloride, compound **121.** The reaction is preferably conducted in the presence of a conventional activating agent such as DMAP in the presence of a base such as pyridine that scavenges the acid generated. The reaction is preferably conducted in an inert solvent such as dichloromethane, chloroform and the like although a liquid base such as pyridine can be employed as the solvent and to scavenge the acid generated. The reaction is preferably conducted at a temperature of from about -5 to about 35°C until the reaction is substantially complete, which is typically 2 to 12 hours. The resulting product, compound **122,** is obtained after a standard deprotection reaction, and can be recovered by conventional methods, such as chromatography, filtration, crystallization, and the like or, alternatively, used in the next step without purification and/or isolation.

Hydrolysis of compound **122,** using conventional conditions such as lithium hydroxide and water in methanol and/or THF, affords the 3-(R¹CO)-5-carboxylic acid pyrazole, compound **123.**

Compound **123** is functionalized at the 4-position of the pyrazole ring by conventional methods to provide for compound **107.** For example, when X is halo, compound 123 is contacted with at least a stoichiometric amount of a suitable halogenation agent such as N-halo succinimide, bromine, and the like. The reaction is conducted in an inert diluent such as dimethylformamide, dichloromethane, and the like at a temperature sufficient to effect halogenation. Typically, the reaction is conducted at from about 0° to about 40°C until the reaction is substantially complete which typically occurs in about 0.1 to 10 hours. The resulting product, compound **107,** can be recovered by conventional methods, such as chromatography, filtration, crystallization, and the like or, alternatively, used in the next step without purification and/or isolation.

Subsequently, the carboxylic acid group of compound **107** is amidated using at least a stoichiometric amount and preferably an excess of a suitable amine, HNR⁴R⁵, under conventional conditions well known in the art preferably using an activating agent to effect coupling such as HOBT, EDC·HCL, NMM and the like. The resulting compound **125** can be recovered by conventional methods, such as chromatography, filtration, crystallization, and the like.

Alternatively, compound **123** may be amidated as described above to form compound **124,** which can be recovered by conventional methods, such as chromatography, filtration, crystallization, and the like. Compound **124** can be functionalized at the 4-position of the pyrazole ring by conventional methods to provide for compound **125** using the same methods described for the conversion of compound **123** to compound **107.**

In an alternative synthetic embodiment, compounds of Formula (I) or Formula (II) where X is alkyl or hydrogen can be prepared as shown in Scheme 2 below:

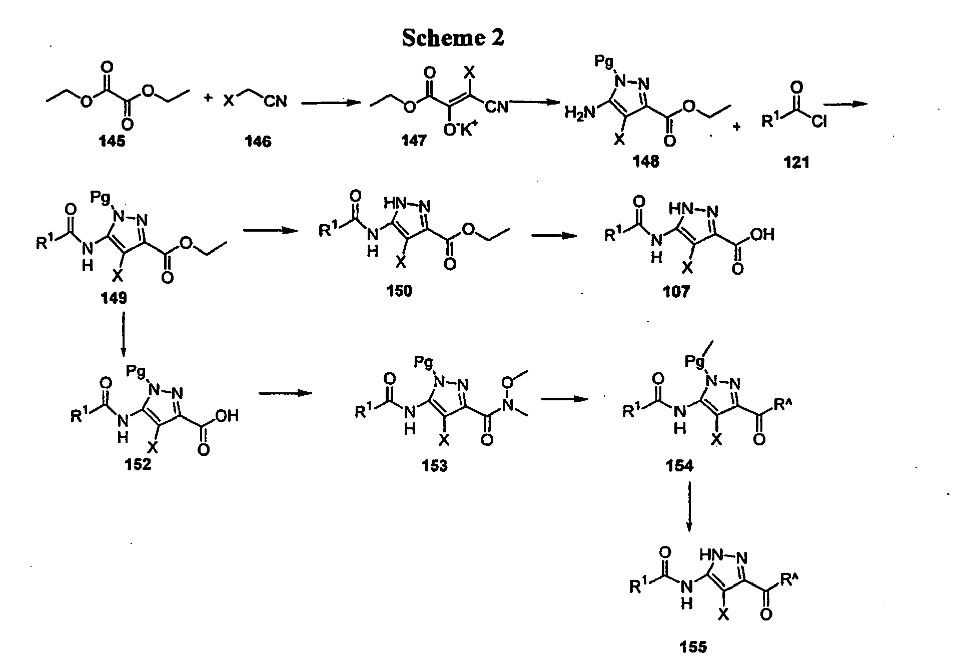

Specifically, in Scheme 2, wherein R¹ is defined above, commercially available oxalic acid diethyl ester, compound **145,** is combined with at least a stoichiometric amount of an alkylnitrile, compound **146,** in the presence of a suitable base such as potassium ethoxide in ethanol. The reaction is preferably maintained at a temperature of from about 60°C to about 100°C until the reaction is substantially complete, which is typically 12 to 24 hours. The resulting product, compound **147,** can be recovered by conventional methods, such as chromatography, filtration, crystallization, and the like or, alternatively, used in the next step without purification and/or isolation.

Compound **147** is then cyclized using a slight excess of t-butyl hydrazine hydrochloride (not shown) in ethanol. The reaction is preferably conducted at elevated temperatures and pressures such as a temperature of from about 75 to about 150°C and a pressure of from about 1 to 10 atm until the reaction is substantially complete, which is typically 12 to 24 hours. The resulting product, compound **148,** can be recovered by conventional methods, such as chromatography, filtration, crystallization, and the like or, alternatively, used in the next step without purification and/or isolation.

Reaction of compound **148** proceeds in the manner described above for compound 120 to provide for compounds of Formula (I) or Formula (II) where X is alkyl.

Scheme 2 further illustrates derivatization of the carboxyl group of the 2-(Pg)-3-(-NHC(O)R¹)-4-(X)-5-carboxyl pyrazole, compound **149.** Specifically, conventional hydrolysis of the ester provides for compound **152** which is then converted to the methoxymethylamide by reaction with commercially available *N*-*O*-dimethylhydroxylamine hydrochloride under conventional coupling conditions in a suitable inert diluent such as tetrahydrofuran, dioxane, and the like optionally in the presence of an activating agent. The reaction is maintained under conditions sufficient to afford compound **153** including, for example, a temperature of from about 0 to about 40°C for a period of from 12 to 24 hours. The resulting product, compound 153, can be recovered by conventional methods, such as chromatography, filtration, crystallization, and the like or, alternatively, used in the next step without purification and/or isolation.

Compound **153** is then derivatized by contact with at least a stoichiometric amount, and preferably an excess, of R^-Li where R^ is selected from the group consisting of alkyl, substituted alkyl, cycloalkyl, substituted cycloakyl, heterocycloalkyl, substituted heterocycloalkyl, aryl, substituted aryl, heteroaryl and substituted heteroaryl. The reaction is typically conducted in an inert solvent such as tetrahydrofuran, dioxane, and the like at a reduced temperature of from about 0 °C to about -78 °C for a period of time sufficient for substantial reaction completion which typically occurs in about 12 to 24 hours. The resulting product, compound **155,** can be recovered by conventional methods, such as chromatography, filtration, crystallization, and the like

The starting materials employed in the reactions described above are either commercially available and/or can be prepared by methods well known in the art. For example, acid halides of the formula R¹C(O)X are readily prepared from the corresponding carboxylic acid by reaction with, e.g., oxalyl halide, thionyl halide and the like. Acids of the formula R¹C(O)OH are extremely well known and include aromatic acids (e.g., R¹ is aryl)

Alternatively, *o-*(Ar-S-CH₂-)benzoyl chloride, compound **143,** can be prepared as illustrated in Scheme 3 below where Ar is an aryl, substituted aryl, heteroaryl, substituted heteroaryl, heterocyclic, and substituted heterocyclic:

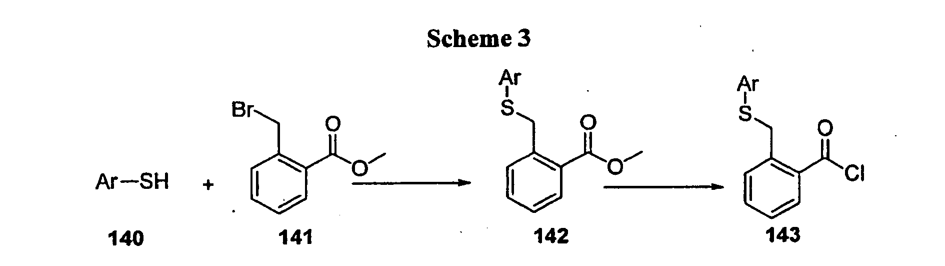

Specifically, compound **140** is coupled to o-bromomethyl-benzoic acid methyl ester, compound **141** (prepared as per Dvornikovs J. Org. Chem, 2002, 67, 2160) , in the presence of about 30 equivalents potassium carbonate in DMF. This reaction is typically conducted at a temperature of from about 0 to about 30 °C until the reaction is substantially complete, which is typically 1 to 15 days. The resulting product, compound **142,** can be recovered by conventional methods, such as chromatography, filtration, crystallization, and the like or, alternatively, used in the next step without purification and/or isolation.

*o*-(Ar-S-CH₂-)benzoyl chloride, compound **143,** is then prepared by conventional hydrolysis of the methyl ester in compound **142** followed by conventional conversion of the carboxyl group to the carboxyl halide using, e.g., oxalyl halide in the presence of a base to scavenge the acid generated. The reaction is typically conducted in an inert solvent such as dichloromethane. This reaction is typically run at a temperature of about -20 to about 10 °C until the reaction is substantially complete, which is typically 1 to 12 hours. The resulting product, compound **143,** can be recovered by conventional methods, such as filtration, crystallization, and the like or, alternatively, used in the next step without purification and/or isolation.

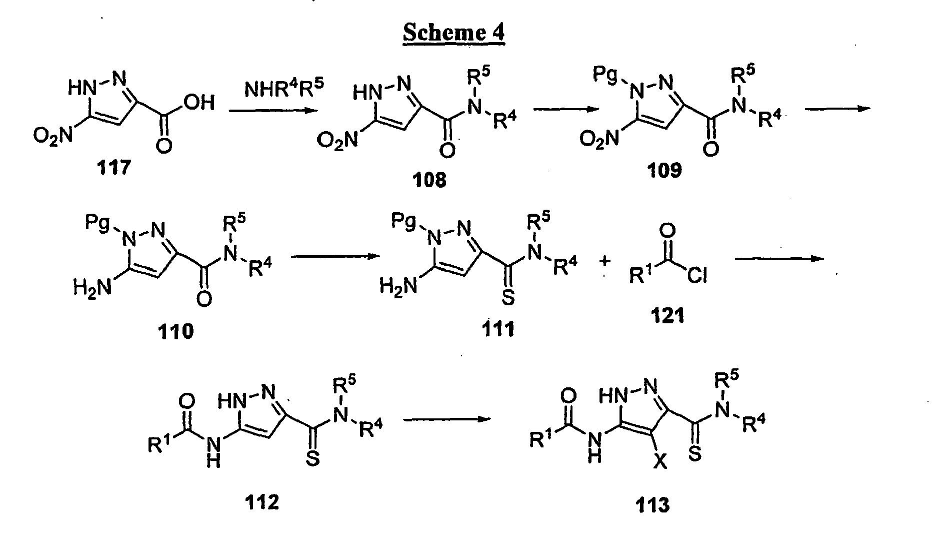

In one embodiment, Z' of the substituted pyrazoles of Formula 1 is sulfur. These substituted pyrazoles are prepared as shown in Scheme 4, where Pg, X, R⁴, R⁵ and R¹ are as defined herein above.

Specifically, in Scheme 4, commercially available 3-nitro-5-carboxyl pyrazole, compound **117,** is coupled to an amine using conventional conditions, for example, by using an activating agent such as HOBT, EDC·HCl, NMM and the like to effect coupling as described herein above. The resulting compound **108** can be recovered by conventional methods such as chromatography, filtration, crystallization and the like.

The 3-nitro-5-carboxyl amide pyrazole, compound **108,** is protected with a protecting group, Pg, under conventional conditions to afford compound **109.** The selected protecting group is one that is removed under conditions other than hydrogenation. A preferred protecting group is the Boc group.

The nitro group of the protected 3-nitro-5-carboxyl amide pyrazole, compound **109,** is reduced to an amine using standard reduction reactions. For example, compound **109** is reacted with hydrogen gas at about 10 to 60 psi, in the presence of a suiable catalyst such as Pd on carbon to afford the corresponding amine, compound **110.**

The 3-amino-5-carboxyl amide pyrazole, compound **110,** is converted to the thioamide, compound **111,** under conventional conditions known in the art. Formation of thioamides from amides can be accomplished using a number of known methods including the use of P₄S₁₀ or Lawesson's reagent as well as other methods know in the art such as those illustrated by Ernst Schaumann in Comprehensive Organic Synthesis Barry M. Trost, Ed.; Pergamon Press: Oxford, 1991; Vol. 6, Chapter 2.4.

The 3-amino-5-thiocarboxyl amide pyrazole, compound **111,** is acylated under conventional conditions by reaction with a desired acyl chloride, compound **121.** The reaction is preferably conducted in the presence of a conventional activating agent such as DMAP in the presence of a base such as pyridine that scavenges the acid generated. The reaction is preferably conducted in an inert solvent such as dichloromethane, chloroform and the like. Alternatively a liquid base such as pyridine can be employed as the solvent and to scavenge the acid generated. The reaction is typically conducted at a temperature of about -5 to about 35 °C until completion, usually about 2 to about 12 hours. The resulting product, compound **112,** is obtained after a standard deprotection reaction, and can be recovered by conventional methods, such as chromatography, filtration, crystallization, and the like or, alternatively, used in the next step without purification and/or isolation.

Compound **112,** is functionalized at the 4-position of the pyrazole ring by conventional methods to provide for compound **113.** For example, when X is halo, compound **112** is contacted with at least a stoichiometric amount of a suitable halogenation agent such as N-halo succinimide, Br₂, and the like. The reaction is conducted in an inert diluent such as dimethylformamide, dichloromethane and the like at a temperature sufficient to effect halogenation. Typically, the reaction is conducted at from about 0 to about 40 °C until reaction is substantially complete which typically occurs in about 0.1 to 10 hours. The resulting product, compound **113,** can be recovered by conventional methods, such as chromatography, filtration, crystallization, and the like or, alternatively, used in the next step without purification and/or isolation.

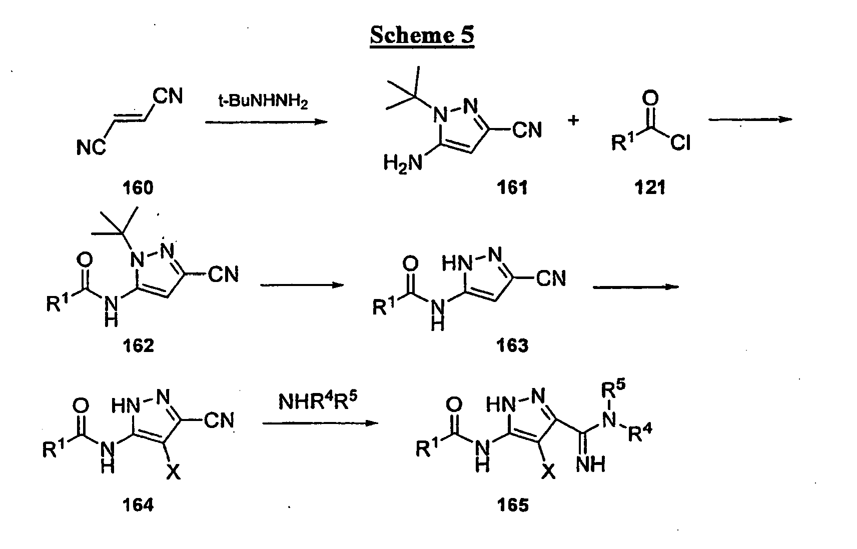

In one embodiment the substituted pyrazoles of Formula 1 in which Z' is NH, the substituted pyrazoles are prepared as shown in Scheme 5.

Specifically, in Scheme 5, 3-amino-5-cyano pyrazole, compound **161,** is prepared by the addition of *tert-*butylhydrazine to fumaronitrile; compound **160.** This reaction is typically run at a temperature of from about 0 to about 110 °C until substantially complete, usually about 1 to about 48 hours. The resulting product, compound **161** can be recovered by conventional methods, such as chromatography, filtration, crystallization, and the like or, alternatively, used in the next step without purification and/or isolation.

The 3-amino-5-cyano pyrazole, compound **161,** is acylated under conventional conditions by reaction with a desired acyl chloride, compound **121** to provide compound **162.** The reaction is preferably conducted in the presence of a conventional activating agent such as DMAP in the presence of a base such as pyridine that scavenges the acid generated. The reaction is preferably conducted in an inert solvent such as dichloromethane, chloroform and the like although a liquid base such as pyridine can be employed as the solvent and to scavenge the acid generated. The resulting product, compound **163,** is obtained after a standard deprotection of compound **162,** and can be recovered by conventional methods, such as chromatography, filtration, crystallization, and the like or, alternatively, used in the next step without purification and/or isolation.

**Compound 163,** is functionalized at the 4-position of the pyrazole ring by conventional methods to provide for compound **164.** For example, when X is halo, compound **163** is contacted with at least a stoichiometric amount of a suitable halogenation agent such as N-halo succinimide, Bromine, and the like. The reaction is conducted in an inert diluent such as dimethylformamide, dichloromethane and the like at a temperature sufficient to effect halogenation. Typically, the reaction is conducted at from about 0 to about 40 °C until reaction is substantially complete which typically occurs in about 0.1 to 10 hours. The resulting product, compound **164,** can be recovered by conventional.methods, such as chromatography, filtration, crystallization, and the like or, alternatively, used in the next step without purification and/or isolation.

Compound **164** is converted to the amidine, compound 165, under conventional conditions known in the art. Formation of amidines from nitriles can be accomplished using a number of known methods including condensation with amines. Other methods of preparing amidines are illustrated by Willi Kantlehner in Comprehensive Organic Synthesis Barry M. Trost, Ed.; Pergamon Press: Oxford, 1991; Vol. 6, Chapter 2.7.

Amines of the formula HNR⁴R⁵ are well known in the art. One skilled in the art will understand how to obtain the aryl or heteroaryl amines used in the syntheses of the present invention. Briefly, aryl or heteroaryl amines can be obtained by reduction of the corresponding nitro compounds obtained by nitration of substituted aryl or heteroaryl rings or Curtius rearrangement of optionally substituted aryl or heteroaryl carboxylic acids. Many other aryl or heteroaryl amines are commercially available from chemical suppliers such as Aldrich, TCI and Lancaster Synthesis, for example.

Compounds of Formula (I) or Formula (II) wherein R¹ is such a sulfonated amino group may be prepared as shown in Scheme 6 below where X, Z', Q, R², R³, R², R²¹ and R²² are as defined herein.

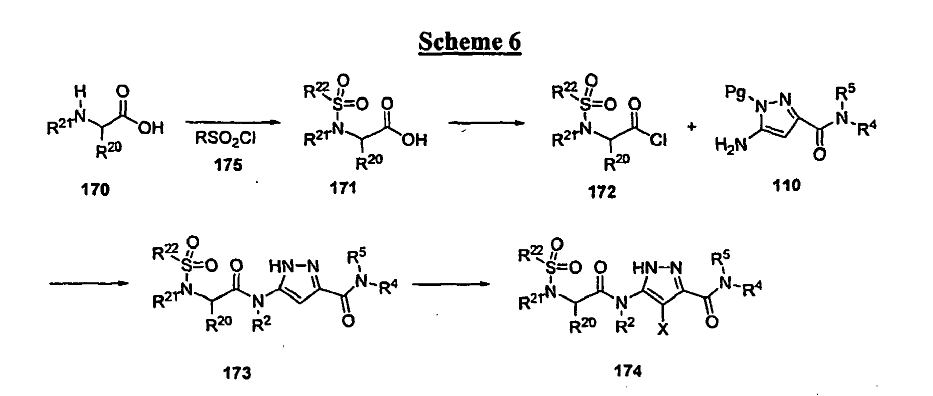

The amine group of compound **170** is converted to the sulfonamide using a suitable sulfonyl chloride, compound **175,** and standard reactions conditions. For example, compound **170** may be reacted with an aryl sulfonyl chloride, compound **175,** in the presence of a suitable base such as sodium carbonate an inert solvent such as water at a temperature of about 0 °C to about 100 °C until the reaction is substantially complete, typically 1 to about 24 hours. The product, compound **171,** can be recovered by conventional methods, such as chromatography, filtration, crystallization, and the like or, alternatively, used in the next step without purification and/or isolation.

Compound **171** is then converted to the acyl chloride using standard conditions. For example, compound **171** may be reacted with SOC1₂ in an inert solvent such as dichloromethane at a temperature of about -10 °C to about 39 °C until the reaction is substantially complete, typically 1 to about 24 hours. The product, compound **172,** can be recovered by conventional methods, such as filtration, crystallization, and the like or, alternatively, used in the next step without purification and/or crystallization.

### Pharmaceutical Formulations

When employed as pharmaceuticals, the compounds of Formula (I) or Formula (Il) are usually administered in the form of pharmaceutical compositions. These compounds can be administered by a variety of routes including oral, rectal, transdermal, subcutaneous, intravenous, intramuscular, and intranasal. These compounds are effective as both injectable and oral compositions. Such compositions are prepared in a manner well known in the pharmaceutical art and comprise at least one active compound.

This invention also includes pharmaceutical compositions that contain, as the active ingredient, one or more of the compounds of Formula (I) or Formula (II) above associated with pharmaceutically acceptable carriers. In making the compositions of this invention, the active ingredient is usually mixed with an excipient, diluted by an excipient or enclosed within such a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions of the invention can be formulated so as to provide quick, sustained or delayed release of the active ingredient after administration to the patient by employing procedures known in the art.

The compositions are preferably formulated in a unit dosage form, each dosage containing 5 to about 100 mg, more usually about 10 to about 30 mg, of the active ingredient. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

The active compound is effective over a wide dosage range and is generally administered in a pharmaceutically effective amount. It, will be understood, however, that the amount of the compound actually administered will be determined by a physician, in the light of the relevant circumstances, including the condition to be treated, the chosen route of administration, the actual compound administered, the age, weight, and response of the individual patient, the severity of the patient's symptoms, and the like.

For preparing solid compositions such as tablets, the principal active ingredient is mixed with a pharmaceutical excipient to form a solid preformulation composition containing a homogeneous mixture of a compound of the present invention. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation is then subdivided into unit dosage forms of the type described above containing from, for example, 0.1 to about 500 mg of the active ingredient of the present invention.

The tablets or pills of the present invention may be coated or otherwise compounded to provide a dosage form affording the advantage of prolonged action. For example, the,tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the novel compositions of the present invention may be incorporated for administration orally or by injection include aqueous solutions suitably flavored syrups, aqueous or oil suspensions, and flavored emulsions with edible oils such as cottonseed oil, sesame oil, coconut oil, or peanut oil, as well as elixirs and similar pharmaceutical vehicles.

Compositions for inhalation or insu$lation include solutions and suspensions in pharmaceutically acceptable, aqueous or organic solvents, or mixtures thereof, and powders. The liquid or solid compositions may contain suitable pharmaceutically acceptable excipients as described *supra.* Preferably the compositions are administered by the oral or nasal respiratory route for local or systemic effect. Compositions in preferably pharmaceutically acceptable solvents may be nebulized by use of inert gases. Nebulized solutions may be breathed directly from the nebulizing device or the nebulizing device may be attached to a face masks tent, or intermittent positive pressure breathing machine. Solution, suspension, or powder compositions may be administered, preferably orally or nasally, from devices which deliver the formulation in an appropriate manner.

The following formulation examples illustrate the pharmaceutical compositions of the present invention.

### Formulation Example 1

Hard gelatin capsules containing the following ingredients are prepared:

| Ingredient | Quantity (mg/capsule) |
|---|---|
| Active Ingredient | 30.0 |
| Starch | 305.0 |
| Magnesium stearate | 5.0 |

The above ingredients are mixed and filled into hard gelatin capsules in 340 mg quantities.

### Formulation Example 2

A tablet formula is prepared using the ingredients below:

| Ingredient | Quantity (mg/tablet) |
|---|---|
| Active Ingredient | 25.0 |
| Cellulose, microcrystalline | 200.0 |
| Colloidal silicon dioxide | 10.0 |
| | Stearic acid 5.0 |

The components are blended and compressed to form tablets, each weighing 240 mg.

### Formulation Example 3

A dry powder inhaler formulation is prepared containing the following components:

| Ingredient | Weight % |
|---|---|
| Active Ingredient | 5 |
| Lactose | 95 |

The active mixture is mixed with the lactose and the mixture is added to a dry powder inhaling appliance.

### Formulation Example 4

Tablets, each containing 30 mg of active ingredient, are prepared as follows:

| Ingredient | Quantity (mg/tablet) |
|---|---|
| Active Ingredient | 30.0 mg |
| Starch | 45.0 mg |
| Microcrystalline cellulose | 35.0 mg |
| Polyvinylpyrrolidone (as 10% solution in water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U.S. sieve. The granules so produced are dried at 50E to 60EC and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 150 mg.

### Formulation Example 5

Capsules, each containing 40 mg of medicament are made as follows:

| Ingredient | Quantity (mg/capsule) |
|---|---|
| Active Ingredient | 40.0 mg |
| Starch | 109.0 mg |
| Magnesium stearate | 1.0 mg |
| Total | 150.0 mg |

The active ingredient, cellulose, starch, an magnesium stearate are blended, passed through a No. 20 mesh U.S. sieve, and filled into hard gelatin capsules in 150 mg quantities.

### Formulation Example 6

Suppositories, each containing 25 mg of active ingredient are made as follows:

| Ingredient | Amount |
|---|---|
| Active Ingredient | 25 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

### Formulation Example 7

Suspensions, each containing 50 mg of medicament per 5.0 mL dose are made as follows:

| Ingredient | Amount |
|---|---|
| Active Ingredient | 50.0 mg |
| Xanthan gum | 4.0 mg |
| Sodium carboxymethyl cellulose (11%) | |
| Microcrystalline cellulose (89%) | 50.0 mg |
| Sucrose | 1.75 g |
| Sodium benzoate | 10.0 mg |
| Flavor and Color | q.v. |
| | Purified water to 5.0 mL |

The medicament, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

### Formulation Example 8

| Ingredient | Quantity (mg/capsule) |
|---|---|
| Active Ingredient | 15.0 mg |
| Starch | 407.0 mg |
| Magnesium stearate | 3.0 mg |
| Total | 425.0 mg |

The active ingredient, cellulose, starch, and magnesium stearate are blended, passed through a No. 20 mesh U.S. sieve, and filled into hard gelatin capsules in 560 mg quantities.

### Formulation Example 9

An intravenous formulation may be prepared as follows:

| Ingredient | Quantity |
|---|---|
| Active Ingredient | 250.0 mg |
| Isotonic saline | 1000 mL |

### Formulation Example 10

A topical formulation may be prepared as follows:

| Ingredient | Quantity |
|---|---|
| Active Ingredient | 1-10 g |
| Emulsifying Wax | 30 g |
| Liquid Paraffin | 20 g |
| White Soft Paraffin | to 100 g |

The white soft paraffin is heated until molten. The liquid paraffin and emulsifying wax are incorporated and stirred until dissolved. The active ingredient is added and stirring is continued until dispersed. The mixture is then cooled until solid.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. See, e.g., U.S. Patent 5,023,252, issued June 11,1991. Such patches may be constructed for continuous, pulsatile, or on demand delivery of pharmaceutical agents.

When it is desirable or necessary to introduce the pharmaceutical composition to the brain, either direct or indirect techniques may be employed. Direct techniques usually involve placement of a drug delivery catheter into the host's ventricular system to bypass the blood-brain barrier. One such implantable delivery system used for the transport of biological factors to specific anatomical regions of the body is described in U.S. Patent 5,011,472.

Indirect techniques, which are generally preferred, usually involve formulating the compositions to provide for drug latentiation by the conversion of hydrophilic drugs into lipid-soluble drugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions which can transiently open the blood-brain barrier.

### Utility

Bradykinin ("BK") is a kinin that plays an important role in the patho-physiological processes accompanying acute and chronic pain and inflammation. Bradykinins, like other related kinins, are autocoid peptides produced by the catalytic action of kallikrein enzymes on plasma and tissue precursors termed kininogens. Inhibition of bradykinin B1 receptors by compounds that are bradykinin B1 antagonists or inverse agonists would provide relief from maladies that mediate undesirable symptoms through a BK B1 receptor pathway.

The compounds of this invention are the bradykinin B₁ receptor antagonists and therefore are suitable for use in blocking or ameliorating pain as well as hyperalgesia in mammals. Such compounds would be effective in the treatment or prevention of pain including, for example, bone and joint pain (osteoarthritis), repetitive motion pain, dental pain, cancer pain, myofascial pain (muscular injury, fibromyalgia), perioperative pain (general surgery, gynecological) and chronic pain. In particular, inflammatory pain such as, for example, inflammatory airways disease (chronic obstructive pulmonary disease) would be effectively treated by bradykinin B1 antagonist compounds.

The compounds of this invention are also useful in the treatment of disease conditions in a mammal that are mediated, at least in part, by bradykinin B₁ receptor. Examples of such disease conditions include asthma, inflammatory bowel disease, rhinitis, pancreatitis, cystitis (interstitial cystitis), uveitis, inflammatory skin disorders, rheumatoid arthritis and edema resulting from trauma associated with burns, sprains or fracture. They may be used subsequent to surgical intervention (e.g. as post-operative analgesics) and to treat inflammatory pain of varied origins (e.g. osteoarthritis, rheumatoid arthritis, rheumatic disease, tenosynovitis and gout) as well as for the treatment of pain associated with angina, menstruation of cancer. They may be used to treat diabetic vasculopathy, post capillary resistance or diabetic symptoms associated with insulitis (e.g. hyperglycemia, diuresis, proteinuria and increased nitrite and kallikrein urinary excretion). They may be used as smooth muscle relaxants for the treatment of spasm of the gastrointestinal tract or uterus or in the therapy of Crohn's disease, ulcerative colitis or pancreatitis. Such compounds may be used therapeutically to treat hyperreactive airways and to treat inflammatory events associated with airways disease e.g. asthma, and to control, restrict or reverse airways hyperreactivity in asthma. They may be used to treat intrinsic and extrinsic asthma including allergic asthma (atopic or non-atopic) as well as exercise-induced asthma, occupational asthma, asthma post-bacterial infection, other non-allergic asthmas and "wheezy-infant syndrome". They may also be effective against pneumoconiosis, including aluminosis, anthracosis, asbestosis, chalicosis, ptilosis, siderosis, silicosis, tabacosis and byssinosis was well as adult respiratory distress syndrome, chronic obstructive pulmonary or airways disease, bronchitis, allergic rhinitis, and vasomotor rhinitis. Additionally, they may be effective against liver disease, multiple sclerosis, atherosclerosis, Alzheimer's disease, septic shock e.g. as anti-hypovolemic and/or anti-hypotensive agents, cerebral edema, headache, migraine, closed head trauma, irritable bowel syndrome and nephritis. Finally, such compounds are also useful as research tools *(in vivo* and *in vitro).*

As noted above, the compounds of this invention are typically administered to the mammal in the form of a pharmaceutical composition. Pharmaceutical compositions of the invention are suitable for use in a variety of drug delivery systems. Suitable formulations for use in the present invention are found in Remington's Pharmaceutical Sciences, Mace Publishing Company, Philadelphia, PA, 17th ed. (1985).

In order to enhance serum half-life, the compounds may be encapsulated, introduced into the lumen of liposomes, prepared as a colloid, or other conventional techniques may be employed which provide an extended serum half-life of the compounds. A variety of methods are available for preparing liposomes, as described in, e.g., Szoka, et al., U.S. Patent No. 4,235,871, Geho, et al., U.S. Patent No. 4,501,728 and Allen, et al., U.S. Patent No. 4,837,028.

The amount administered to the patient will vary depending upon what is being administered, the purpose of the administration, such as prophylaxis or therapy, the state of the patient, the manner of administration, and the like all of which are within the skill of the attending clinician. In therapeutic applications, compositions are administered to a patient already suffering from a disease in an amount sufficient to cure or at least partially arrest the symptoms of the disease and its complications. An amount adequate to accomplish this is defined as "therapeutically effective dose." Amounts effective for this use will depend on the disease condition being treated as well as by the judgment of the attending clinician depending upon factors such as the severity of the inflammation, the age, weight and general condition of the patient, and the like.

The compositions administered to a patient are in the form of pharmaceutical compositions described above. These compositions may be sterilized by conventional sterilization techniques, or may be sterile filtered. The resulting aqueous solutions may be packaged for use as is, or lyophilized, the lyophilized preparation being combined with a sterile aqueous carrier prior to administration. The pH of the compound preparations typically will be between 3 and 11, more preferably from 5 to 9 and most preferably from 7 to 8. It will be understood that use of certain of the foregoing excipients, carriers, or stabilizers will result in the formation of pharmaceutical salts.

The therapeutic dosage of the compounds of the present invention will vary according to, for example, the particular use for which the treatment is made, the manner of administration of the compound, the health and condition of the patient, and the judgment of the prescribing physician. For example, for intravenous administration, the dose will typically be in the range of about 20 µg to about 500 µg per kilogram body weight, preferably about 100 µg to about 300 µg per kilogram body weight. Effective doses can be extrapolated from dose-response curves derived from *in vitro* or animal model test systems.

Alternatively, about 0.1 mg/day to about 1,000 mg/day of a compound, or mixture of compounds, of the present invention may be admistered orally, preferably from about 1 mg/day to about 100 mg /day, and more preferably from 5 mg/day to about 50 mg/day. From about 0.5 to about 100 mg/day may be given to a patient for parenteral, sublingual, intranasal or intrathecal administration; for depo administration and implants, from about 0.5 mg/day to about 50 mg/day; for topical administration from about 0.5 mg/day to about 200 mg/day; for rectal administration from about 0.5 mg to about 500 mg; and more preferably for parenteral administration, from about 5 to about 50 mg daily.

The following synthetic and biological examples are offered to illustrate this invention and are not to be construed in any way as limiting the scope of this invention. Unless otherwise stated, all temperatures are in degrees Celsius.

### EXAMPLES

In the examples below, the following abbreviations have the following meanings. If an abbreviation is not defined, it has its generally accepted meaning.
- Ac =: Acetyl
- aq. =: aqueous
- Boc =: t-butoxycarbonyl
- bs =: broad singlet
- d =: doublet
- dd =: doublet of doublets
- DMAP =: 4*-N,N-*dimethylaminopyridine
- DMF =: *N,N-*dimethylformamide
- EDC or EDC·HCL =: 1-(3-dimethylaminopropyl)-3-ethylcarbodiimidehydrochloride
- Et =: ethyl
- EtOH =: ethanol
- eq. =: equivalents
- g =: gram
- HATU =: O-(7-azabenzotriazol-1-yl)-1,1,3-,3-tetramethyluronium hexafluorophosphate
- HOBT =: 1-hydroxybenzothiazole hydrate
- Hz =: hertz
- HPLC =: high performance liquid chromatography
- MS =: mass spectroscopy
- Me =: methyl
- MeOH =: methanol
- m =: multiplet
- M =: molar
- mg =: milligram
- min. =: minutes
- mL =: milliliter
- MHz =: Megahertz
- MP =: meso porous
- N =: Normal
- NBC =: *N*-bromosuccinamide
- NMR =: nuclear magnetic resonance
- NMM =: *N*-methylmorpholine
- OAc =: acetate
- PS =: polystyrene
- psi =: pounds per square inch
- q =: quartet
- rt =: room temperature
- s =: singlet
- sat. =: saturated
- t =: triplet
- THF =: tetrahydrofuran
- wt/wt =: weight to weight ratio
- µL =: microliters

In the following examples and procedures, the term "Aldrich" indicates that the compound or reagent used in the procedure is commercially available from Aldrich Chemical Company, Inc., Milwaukee, WI 53233 USA; the term "Sigma" indicates that the compound or reagent is commercially available from Sigma, St. Louis MO 63178 USA; the term "TCI" indicates that the compound or reagent is commercially available from TCI America, Portland OR 97203; the term "Frontier" or "Frontier Scientific" indicates that the compound or reagent is commercially available from Frontier Scientific, Utah, USA; the term "Bachem" indicates that the compound or reagent is commercially available from Bachem, Torrance, California, USA. The term "Matrix" indicates that the compound or reagent is commercially available from Matrix Scientific, Columbia, SC, USA. The term "Ambinter" indicates that the compound or reagent is commercially available from Ambinter Paris, France.

The following general procedures illustrate general synthetic pathways for preparing 3-amido-5-substituted pyrazole derivatives of Formula (I) or Formula (II) and amine intermediates useful in preparing the same.

### GENERAL PROCEDURE 1

### Preparation of 4-Bromo-5-(2-chlorobenzoylamino)-1H-pyrazole-3-carboxylic acid (7).

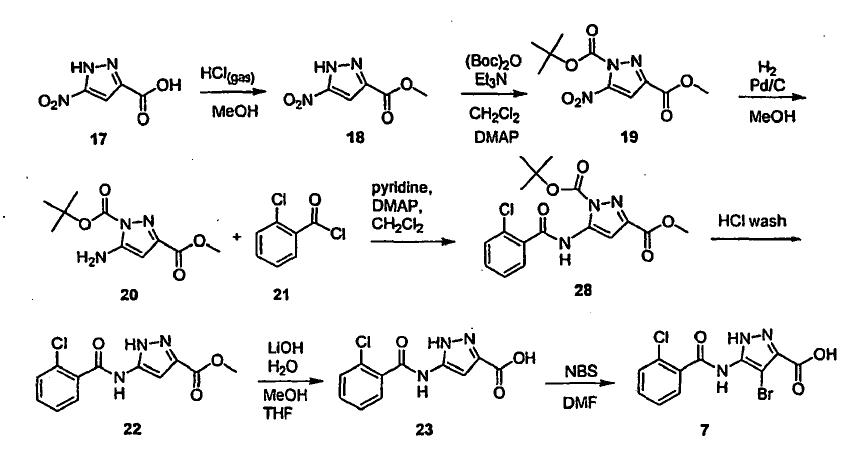

**Preparation of 3-Methoxycarbonyl-5-nitropyrazole (18).** A solution of 5-nitro-1*H*-pyrazole-3-carboxylic acid (**17,** Aldrich, cat. no. 41,483-2) in MeOH was prepared. While stirring, HCl was bubbled through the solution for 2 min. The reaction mixture was refluxed for a time sufficient for complete esterification and allowed to cool to rt. The solvent was removed by rotary evaporation. The crude material was basified by addition of saturated aqueous NaHCO₃ and extracting with EtOAc. The combined organic extracts were dried over MgSO₄ and filtered. The filtrate was rotary evaporated and dried under vacuum to yield **18.**

**Preparation of 1-*tert*-Butoxycarbonyl-3-methoxycarbonyl-5-nitropyrazole (19).** A solution of 1.0 eq. of **18,** 1.1 eq. of (Boc)₂O, 1.5 eq. of Et₃N, and 0.05 eq. of DMAP in CH₂Cl₂ was prepared. The reaction mixture was stirred at rt for a time sufficient for reaction completion and the solvent was removed by rotary evaporation. The crude material was dried under vacuum to afford product **19.**

**Preparation of 5-Amino-1-*tert*-butoxycarbonyl-3-methoxycarbonylpyrazole (20).** A mixture of 1.0 eq. of **19** and 0.1 wt/wt eq. of 10% Pd on carbon was hydrogenated at 10-60 psi of hydrogen for a time sufficient for reaction completion. The reaction mixture was filtered through Celite. The filtrate was concentrated by rotary evaporation. The crude material was dried under vacuum to give product **20.**

**Preparation of 5-(2-Chloro-benzoylamino)-pyrazole-1,3-dicarboxylic acid 1-tert-butyl ester 3-methyl ester (28).** A solution of 1.0 eq. of 20, 1.5 eq. of pyridine, and 0.04 eq. of DMAP in CH2Cl2 was prepared. After cooling to 0 °C, 1.1 eq. of 2-chlorobenzoyl chloride (Aldrich, cat. no. 10,391-8) was added. The reaction solution was allowed to warm to rt and after a time sufficient for reaction completion, the solvent was removed by rotary evaporation to afford crude **28.**

**Preparation of 5-(2-Chlorobenzoylamino)-3-methoxycarbonylpyrazole (22).** A solution of **28** in 1 M HCl was stirred for 5 min and extracted with EtOAc. The combined organic extracts were washed with saturated aqueous NaHCO3, followed by drying over MgSO4 and filtering. The filtrate was rotary evaporated and dried under vacuum to yield **22.**

**Preparation of 5-(2-Chlorobenzoylamino)-1H-pyrazole-3-carboxylic acid (23).** A solution of 1.0 eq. of **22** and 5.0 eq. of LiOH·H₂O in THF, MeOH, and H₂O (2:1:1) was stirred at rt. After a time sufficient for reaction completion, the reaction mixture was rotary evaporated. The mixture was acidified with concentrated HCl. As the pH of the solution reached about 2, a precipitate formed. The solid was collected by filtration and after drying under vacuum, product **23** was obtained.

**Preparation of the title compound (7).** A solution of 1.0 eq. of **23** in DMF was prepared. While stirring, a solution of 1.2 eq. of *N*-bromosuccinamide in DMF was added. After stirring at rt for a time sufficient for reaction completion, H₂O was added. The mixture was extracted with EtOAc. The combined organic extracts were washed with 1 M HCl, followed by drying over MgSO₄ and filtering. The filtrate was rotary evaporated.
The crude material was triturated with CH₂Cl₂ and dried under vacuum to yield 7.

### GENERAL PROCEDURE 2

### Preparation of 4-Chloro-5-(2-chlorobenzoylamino)-1H-pyrazole-3-carboxylic acid (24).

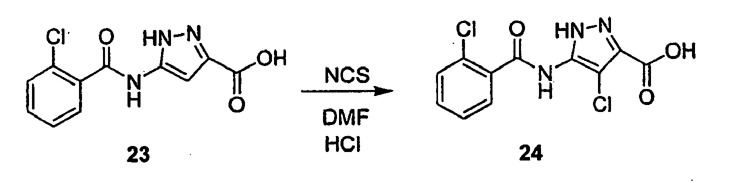

A solution of 1.0 eq. of **23** in DMF was prepared. While stirring, 1.3 eq. of *N*-chlorosuccinamide and a small amount of concentrated HCl were added. After stirring at rt for a time sufficient for reaction completion, H₂O was added. The quenched reaction solution was extracted with EtOAc. The combined organic extracts were dried over MgSO₄ and filtered. The filtrate was rotary evaporated. The crude material was triturated with CH₂Cl₂ and dried under vacuum to yield **24.**

### GENERAL PROCEDURE 3

### Preparation of (R)-4-Bromo-5-(2-chlorobenzoylamino)-1H-pyrazole-3-carboxylic acid amides (25).

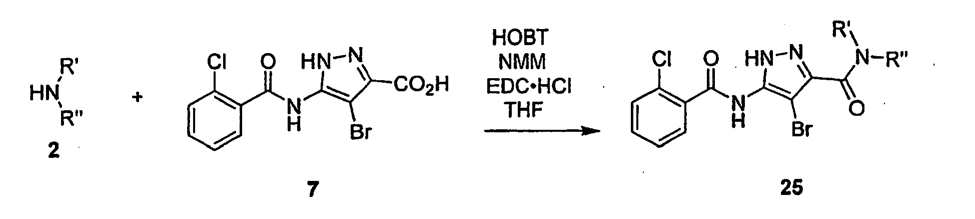

Compound **7** was prepared as shown in General Procedure 1. Compound **25** was prepared as shown in General Procedure 3. A mixture of 1.0 eq. of **7,** 1.1 eq. of **2,** 1.2 eq. of HOBT, 2.2 eq. of NMM, and 1.2 eq. of EDC•HCl in THF was stirred at rt. After a time sufficient for reaction completion, the reaction mixture was adsorbed onto silica gel and flash chromatographed using a mixture of EtOAc/hexanes as eluant to give product **25.**

### General Procedure 4

### Preparation of 4-Bromo-5-(2-chlorobenzoylamino)-1H-pyrazole-3-carboxylic acid anilinoamides (27).

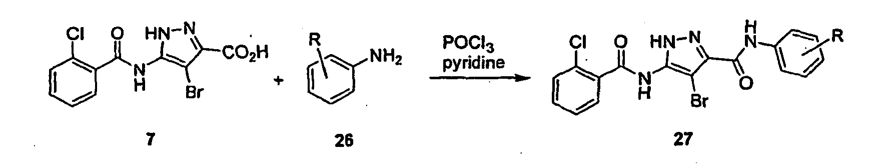

A solution of 1.0 eq. of **7** and 1.3 eq. of **26** in dry pyridine was cooled to -10°C. While stirring, 1.1 eq. of POCl₃ was added dropwise. The cooling bath was removed after 10 min and the mixture was stirred at rt. After 10 min, 1.0 M HCl was added. The mixture was extracted with EtOAc. The combined organic extracts were washed with saturated aqueous NaHCO₃, followed by drying over MgSO₄ and filtering. The filtrate was rotary evaporated. The crude material was flash chromatographed using a mixture of EtOAc-hexanes as eluant to yield **27.**

### GENERAL PROCEDURE 5

### Preparation of 5-[(2-Chloro-benzoyl)-methyl-amino]-1H-pyrazole-3-carboxylic acid methyl ester (29).

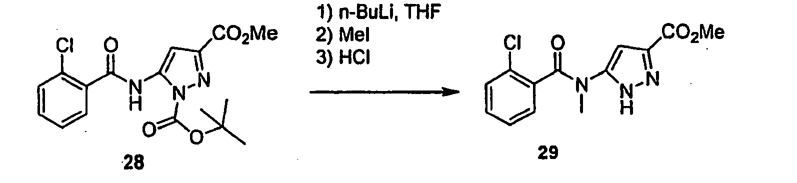

A suspension of 1.0 eq. of ester **28,** prepared in General Procedure 1, in THF was stirred at -78 °C as 2.0 eq. of a 2.5 M solution of n-BuLi in THF was added. The cooling bath was removed and the reaction mixture was allowed to stir while warming for 10 min. The mixture was cooled back to -78°C and 2.0 eq. of MeI was added. The bath was again removed and the reaction mixture was allowed to warm to rt. After a time sufficient for reaction completion, the reaction was quenched with 1 M HCl and extracted with EtOAc. The organic layer was washed with sat. aq. NaHCO₃, dried over MgSO₄, filtered and the solvent removed by rotary evaporation. The material was purified by flash chromatography on silica gel using a mixture of EtOAc-hexanes as eluant to afford **29.**

### GENERAL PROCEDURE 6

### Preparation of 4-Bromo-5-[2-(quinolin-8-ylthiomethyl)benzoylamino]-1H-pyrazole-3-carboxylic acid (43).

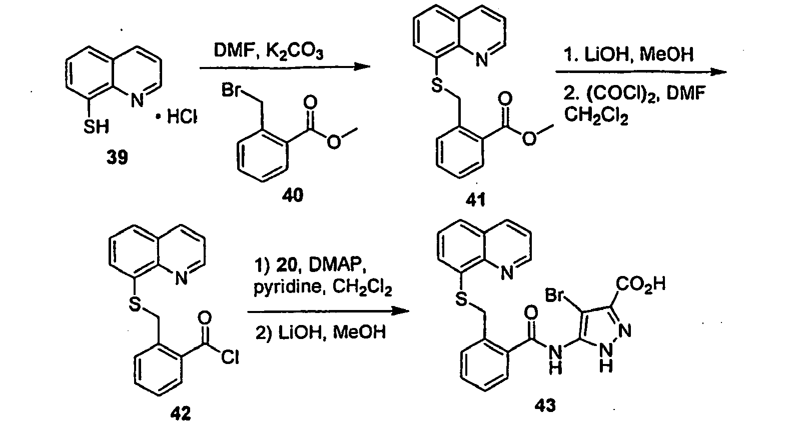

**Preparation of 2-(Quinolin-8-ylthiomethyl)benzoic acid methyl ester (41).** A solution of 4.0 eq. of quinoline-8-thiol hydrochloride (39, Aldrich, cat. no. 35,978-5) was dissolved in DMF. To this was added 32.0 eq. of potassium carbonate. The mixture was stirred at room temperature for 20 minutes and 1.0 eq of 2-bromomethyl-benzoic acid methyl ester (40, J. Org. Chem, 2002, 67, 2160) was added. The mixture was stirred at room temperature for a time sufficient enough for reaction completion. The mixture was diluted with 0.1 M citric acid and extracted with EtOAc. The organic layer was washed with brine and dried with Na₂SO₄, filtered, and concentrated. The product was purified by flash chromatography on silica gel using a mixture of EtOAc-hexanes as eluant to give 41.

**Preparation of 2-(Quinolin-8-ylthiomethyl)benzoyl chloride (42).** A solution of 1.0 eq of ester 41 and 3.0 eq. of LiOH in methanol and water was heated to 65°C for a time sufficient for completion of the hydrolysis. The mixture was cooled to room temperature and concentrated, then diluted with H₂O. The pH of the aqueous mixture was adjusted to 4.5 and extracted with EtOAc. The organic layer was washed with brine and dried over Na₂SO₄, filtered, and concentrated to give the intermediate benzoic acid.

A solution of 1.0 eq. of 2-(quinolin-8-ylthiomethyl)benzoic acid in CH₂Cl₂ was cooled to 0°C. To this was added 1.1 eq. of oxalyl chloride followed by one drop of DMF. The mixture was warmed to room temperature and stirred for a time sufficient for reaction completion. The mixture was concentrated to give **42** which was used directly.

**Preparation of the title compound (43).** The procedure described for compound 22 was employed using 2-(quinolin-8-ylthiomethyl)benzoyl chloride **(42).** Hydrolysis of the methyl ester as described for compound **23** afforded acid **43.**

### General Procedure 7

### Preparation of 4-Bromo-5-(2-chlorobenzoylamino)-1H-pyrazole-3-carboxylic acid amides (44).

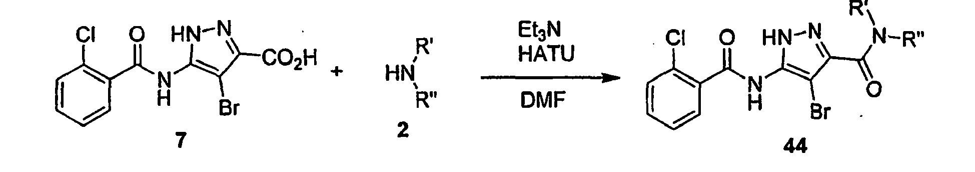

A solution of 1.0 eq. of acid 7, 1.0 eq. of amine 2, and 8.1 eq. of Et₃N in DMF was prepared. While stirring, a solution of 1.0 eq. of HATU dissolved in DMF was added. After stirring at rt for a time sufficient for reaction completion, 6.0 eq. of MP-carbonate resin and 6.0 eq. of PS-trisamine resin (both from Argonaut Technologies, Inc.) were added. The mixture was stirred at rt for 16 hrs, filtered, and washed with DMF and MeOH. The crude material was purified by reverse-phase HPLC using a mixture of acetonitrile-water as eluant. The purified material was concentrated and dried to afford amide **44.**

### GENERAL PROCEDURE 8

### Preparation of 5-(2-Chloro-benzoylamino)-4-methyl-1H-pyrazole-3-carboxylic acid (51).

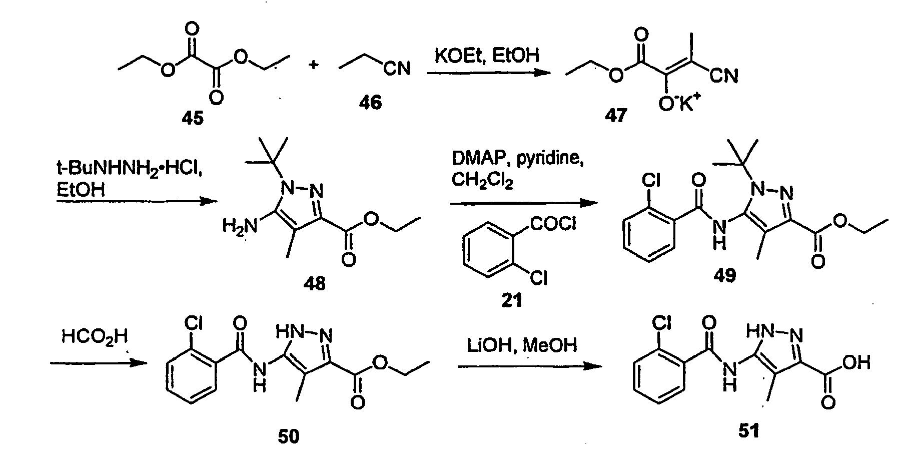

**Preparation of Potassium 2-cyano-1-ethoxycarbonyl-2-methylethenolate (47).** Placed potassium ethoxide (1.0 eq.) in a sealed tube with EtOH and shook until dissolved. A mixture of diethyl oxalate (1.0 eq.) and propionitrile (1.0 eq.) was added to the sealed tube and the mixture was capped and stirred at reflux. After a time sufficient for reaction completion, the reaction was cooled and the precipitate collected and washed with diethyl ether to afford **47.**

**Preparation of 5-Amino-1-*tert*-butyl-4-methyl-1*H*-pyrazole-3-carboxylic acid ethyl ester (48).** Placed 47 (1.0 eq.) into a sealed pressure reaction flask. Added EtOH and t-butylhydrazine hydrochloride (1.1 eq.). The pressure flask was capped and heated to reflux. After a time sufficient for reaction completion, the mixture was evaporated to dryness and the solid obtained was dissolved in equal amounts of EtOAc and water. The organic layer was washed with saturated aqueous NaHCO₃, brine, dried with MgSO₄, and evaporated. This slightly yellow solid was triturated with hexanes and filtered to afford ester **48.**

**Preparation of 1-*tert*-Butyl-5-(2-chloro-benzoylamino)-4-methyl-1H-pyrazole-3-carboxylic acid ethyl ester (49).** The procedure described for compound **22** was employed using 5-amino-1-*tert*-butyl-4-methyl-1*H*-pyrazole-3-carboxylic acid ethyl ester **(49).**

**Preparation of 5-(2-Chloro-benzoylamino)-4-methyl-1*H*-pyrazole-3-carboxylic acid ethyl ester (50).** Ester **48** was dissolved in a minimal amount of formic acid and heated to 80°C for a time sufficient for reaction completion. Formic acid was removed via rotary evaporation to yield **50.**

**Preparation of the title compound (51).** The procedure described for compound **23** was employed using 5-(2-chloro-benzoylamino)-4-methyl-1*H*-pyrazole-3-carboxylic acid ethyl ester **(50).**

### GENERAL PROCEDURE 9

### Preparation of N-(5-Carboxyalkyl-4-methyl-2H-pyrazol-3-yl)-2-chloro-benzamide (55).

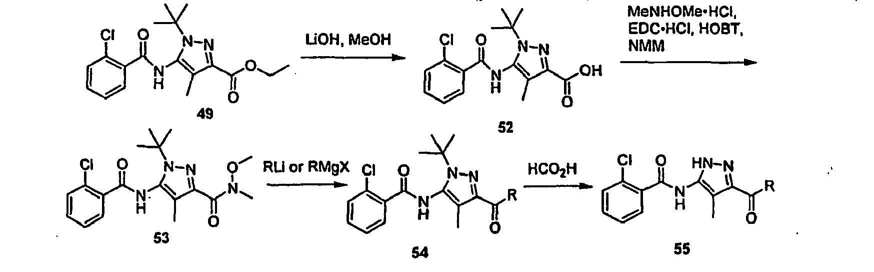

**Preparation of 1-*tert*-Butyl-5-(2-chloro-benzoylamino)-4-methyl-1*H*-pyrazole-3-carboxylic acid (52).** The procedure described for compound **23** was employed using 1-*tert*-butyl-5-(2-chlorobenzoylamino)-4-methyl-1*H*-pyrazole-3-carboxylic acid ethyl ester **(49)** to afford acid **52.**

**Preparation of 1-*tert*-Butyl-5-(2-chlorobenzoylamino)-4-methyl-1*H-*pyrazole-3-carboxylic acid methoxymethylamide (53).** The procedure described for compound 3 was employed using 1-*tert*-butyl-5-(2-chloro-benzoylamino)-4-methyl-1*H-*pyrazole-3-carboxylic acid **(52)** and *N*,*O*-dimethylhydroxylamine hydrochloride (Aldrich, cat. no. D16,370-8).

**Preparation of *N*-(5-Carboxyalkyl-2-*tert*-butyl-4-methyl-2H-pyrazol-3-yl)-2-chlorobenzamides (54).** To a flask equipped with a stirbar was added 53 (1.0 eq.) dissolved in THF under a nitrogen atmosphere. The mixture was cooled to -10°C and a 1.4 M solution of MeLi (6.0 eq.) in diethyl ether was added dropwise. The mixture was allowed to slowly warm to room temperature and stirred for a time sufficient for reaction completion. The reaction was poured into 0.1 N HCl and extracted with dichloromethane, dried over MgSO₄, filtered and concentrated to a crude oil. The crude material was purified by column chromatography eluting with a mixture of EtOAc-hexanes to afford 54.

**Preparation of the title compound (55).** The procedure described for compound 50 was employed using benzamide 54.

### GENERAL PROCEDURE 10

### Preparation of 4-Bromo-5-(2-m-tolylthiomethylbenzoylamino)-1H-pyrazole-3-carboxylic acid (62).

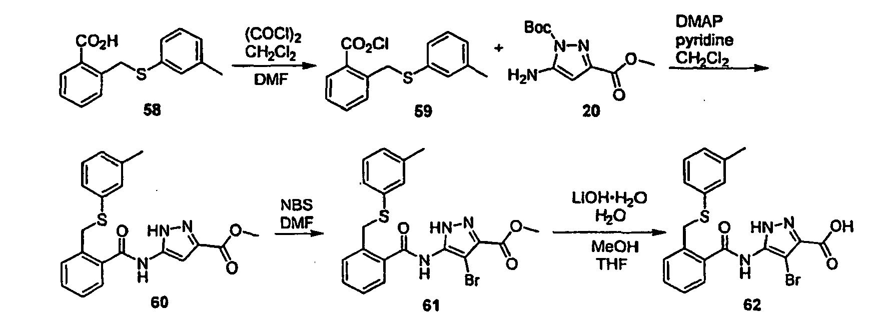

**Preparation of *2-m-*Tolylthiomethyl-benzoyl chloride (59).** A solution of 1.0 eq. of **58** (Coll. Czech. Chem. Comm. 1982, 47, 3094) in CH₂Cl₂ was prepared. While stirring, 1.1 eq. of oxalyl chloride and one drop of DMF was added. After stirring at rt for a time sufficient for reaction completion, the reaction mixture was rotary evaporated and dried under vacuum to produce **59.**

**Preparation of 5-(2-*m*-Tolylthiomethybenzoylamino)-1*H*-pyrazole-3-carlboxylic acid methyl ester (60).** A solution of 1.1 eq. of **20,** 1.1 eq. of pyridine, and 0.07 eq. of DMAP in CH₂Cl₂ was cooled to 0°C. While stirring, a solution of 1.0 eq. of **59** in CH₂Cl₂ was added. The reaction solution was allowed to warm to rt. After a time sufficient for reaction completion, the reaction solution was concentrated by rotary evaporation and 1.0 M HCl was added. The acidified solution was extracted with EtOAc. The combined organic extracts were washed with saturated aqueous NaHCO₃, followed by drying over MgS0₄ and filtering. The filtrate was rotary evaporated and the crude material was purified by flash chromatography on silica gel using a mixture of EtOAc-hexanes as eluant to give **60.**

**Preparation of 4-Bromo-5-(2-*m*-tolylthiomethylbenzoylamino)-1*H* pyrazole-3-carboxylic acid methyl ester (61).** A solution of 1.0 eq. of **60** in DMF was prepared. While stirring, a solution of 1.1 eq. of NBS in DMF was added. After stirring at rt for a time sufficient for reaction completion, water was added. The solution was extracted with EtOAc. The combined organic extracts dried over MgSO₄ and vacuum filtered. The filtrate was rotary evaporated and the crude material was purified by flash chromatography on silica gel using a mixture of EtOAc-hexanes as eluant to give **61.**

**Preparation of the title compound (62).** The procedure described for compound **23** was employed with methyl ester **61** to afford acid **62.**

### GENERAL PROCEDURE 11

### Preparation of 3-(4-Amino-phenyl)-propionitrile (66)

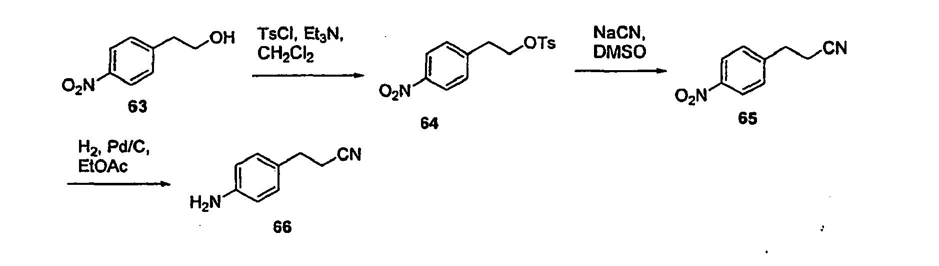

**Preparation of *p*-Toluene-4-sulfonic acid 2-(4-nitrophenyl)ethyl ester (64).** To a solution of 1.0 eq. of 2-(4-nitrophenyl)ethanol **63** (Aldrich, 18,346-6) and 1.1 eq. triethylamine in CH₂Cl₂ was added *p*-toluenesulfonyl chloride (1.0 eq.). The mixture was stirred overnight under nitrogen and concentrated to dryness. The crude solid was taken up in equal amounts of EtOAc and sat. aq. copper sulfate solution. The organic layer was washed with brine and dried over Na₂SO₄. Removing the solvent followed by trituration with Et₂O afforded compound **64** as a slightly tan solid.

**Preparation of 3-(4-nitrophenyl)propionitrile (65).** To a solution of sodium cyanide (1.3 eq.) in DMSO (5 ml) pre-heated to 70°C was added 1.0 eq. of *p-*toluene-4-sulfonic acid 2-(4-nitrophenyl)ethyl ester **(64).** Upon completion of addition, the reaction mixture was heated to over 90°C and stirred for 2 hours. The brown solution was cooled to rt and water was added to give a white precipitate which was extracted into CH₂Cl₂. The extracts were then washed with water and dried over Na₂SO₄. Column chromatography with gradients of EtOAc/hexanes quickly removed baseline impurities and afforded a light orange oil which was sufficiently pure for further elaboration.

**Preparation of 3-(4-Aminophenyl)propionitrile (66).** 3-(4-Nitrophenyl) propionitrile, compound (65), was dissolved into EtOAc and placed in a Parr hydrogenation bottle. A spatula tip of 5 % Pd/C sat. with H₂O) was added to the bottle and the mixture placed on Parr hydrogenation shaker at 30 psi for approximately 3 hours. The reaction was filtered through Celite, dried over Na₂SO₄ to afford compound 66.

### GENERAL PROCEDURE 13

### Preparation of 4-Bromo-5-(2-chloro-benzoylamino)-1H-pyrazole-3-carboxylic acid {4-[2-(4,5-dihydro-1H-imidazol-2-yl)ethyl]phenyl}amide (68)

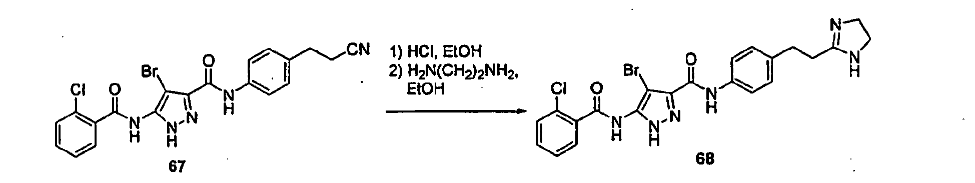

HCl gas was bubbled for 10 minutes into a solution of **67** (prepared as shown in General Procedure 4 using compound **66** and pyrazole acid 7) in EtOH and cooled to 0 °C (1.0 eq.). After 30 minutes of stirring a white precipitate formed. The mixture was stirred for an additional 5 hours and then evaporated to dryness. The crude mixture was then dissolved in EtOH and ethylene diamine (1.1 eq.) was added. The reaction mixture was then stirred under nitrogen overnight. The mixture was evaporated to dryness and purified by preparative HPLC to afford compound **68** as the trifluoroacetate salt.

### GENERAL PROCEDURE 14

### Preparation of [3-(4-Aminophenyl)propyl]carbamic acid tert-butyl ester (70).

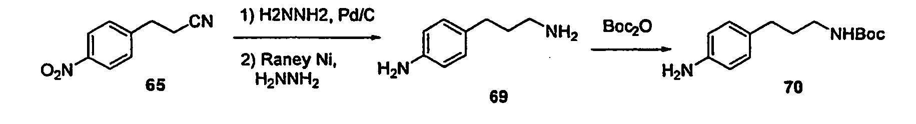

**Preparation of 4-(3-Aminopropyl)phenylamine (69).** Nitrile **65** (8g, 45mmol) was dissolved in absolute ethanol (500mL) under argon. Palladium on carbon (10%, 0.8g) was added followed by hydrazine monohydrate (6.6mL, 136mmol)). After 2 hr, the catalyst was filtered off and Raney nickel (8g) was added followed by more hydrazine monohydrate (6.6mL, 136mmol) and the reaction mixture was heated under argon to 53 °C. After 1hr, a further 6.6mL hydrazine monohydrate was added and the mixture was heated for a further 2 hr. Removal of the catalyst gave diamine **69** as an oil which was used without further purification.
**Preparation of [3-(4-Aminophenyl)propyl]carbamic acid *tert*-butyl ester (70).** Diamine **69** (6.6g, 44mmol) was dissolved in anhydrous CH₂Cl₂ (150mL) and cooled in an ice bath. Di-*tert*-butyl-dicarbonate (9.59g, 44mmol) was added as a solution (100mL anhydrous CH₂Cl₂) slowly while the bath was maintained at 0°C. After 2 hr at 0°C, the solution was washed with sat. aq. NaHCO₃ (100mL), brine (100mL) and dried over Na₂SO₄. The crude product was purified by column chromatography (silica, 30-50% EtOAc:hexanes) to give aniline **70** as a yellow solid.

### GENERAL PROCEDURE 15

### preparation of 5-Amino-1-methyl-1H-pyrazole-3-carboxylic acid methyl ester (74) and 5-Amino-2-methyl-2H-pyrazole-3-carboxylic acid methyl ester (73).

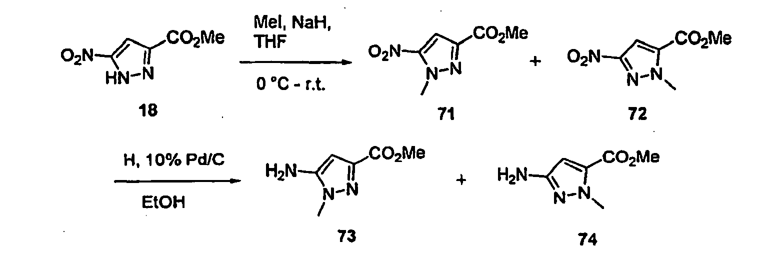

**Preparation of 1-Methyl-5-nitro-1H-pyrazole-3-carboxylic acid methyl ester (71) and 2-Methyl-5-nitro-2H-pyrazole-3-carboxylic acid methyl ester (72).** A suspension of 60% (weight) NaH dispension in mineral oil (10.9 g, 273 mmol) was added in portions into a stirred solution of 5-nitro-1H-pyrazole-3-carboxylic acid methyl ester **(18)** (18.6 g, 109 mmol) in anhydrous THF (200 rnL) cooled under an ice-water bath. After stirring for 35 min, methyl iodide (20.4 mL, 327 mmol) was added, and the reaction mixture was stirred for 20 hr. The solvent was evaporated and the residue was taken up in EtOAc (200 mL), washed with water (60 mL), and stirred over anhydrous MgSO₄ for 20 min. After filtration and concentration, a colorless oil (22.2 g) was obtained, which was confirmed by HPLC/MS and NMR analyses as a mixture of **71** and **72** (see: Baraldi, Pier Giovanni, et al; Molecules [Electronic Publication], 1998, 3(2), M46) in a 1:2.27 ratio.

**Preparation of 5-Amino-1-methyl-1H-pyrazole-3-carboxylic acid methyl ester (73) and 5-Amino-2-methyl-2H-pyrazole-3-carboxylic acid methyl ester (74).** A solution of a mixture of methyl esters **71** and **72** was dissolved in ethanol (60 mL), 10% Pd/C (1.0 g) was added, and the mixture was hydrogenated at 30 psi of H₂ for 16 hr. The mixture was filtered through a layer of Celite and evaporated to afford a yellow solid (16.0 g), which was indicated by HPLC-MS analysis to be a mixture of **73** and **74.** The two isomers were separated by flash chromatography (1:1 EtOAc/hexanes) to yield **74** (9.90 g, 63.9 mmol, 59 %) (Ho H. Lee, et al; J. Org. Chem. 1989, 54, 428-431) and **73** (4.14 g, 26.7 mmol, 24.5%) (Ho H. Lee, et al; J. Org. Chem. 1989, 54, 428-431).
¹H-NMR **(74)** (CDCl₃) δ 6.13 (s, 1H), 4.00 (s, 3H), 3.85 (s, 3H),3.62 (br, 2H).
¹H-NMR **(73)** (CDCl₃) δ 6.06 (s, 1H), 3.86 (s, 3H), 3.72 (s, 3H),3.66 (br, 2H).

### GENERAL PROCEDURE 16

### Preparation of 5-Amino-1-phenyl-1H-pyrazole-3-carboxylic acid ethyl ester (76).

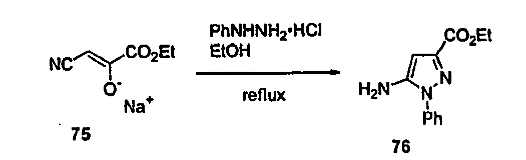

A suspension of 1.0 eq. of 3-cyano-2-oxopropanoic acid ethyl ester **(75)** (Degussa, NACOPE) and 1.2 eq. of phenylhydrazine hydrochloride in absolute EtOH was stirred at reflux for 3 days. The reaction mixture was cooled to rt and filtered through Celite. The solvent was removed by rotary evaporation. Purification of the material on silica gel using 50% EtOAc-hexanes as eluant afforded compound **76.**

### Example 1

### Preparation of 4-Bromo-5-(2-chloro-benzoylamino)-1H-pyrazole-3-carboxylic acid (4-diethylamino-phenyl)-amide

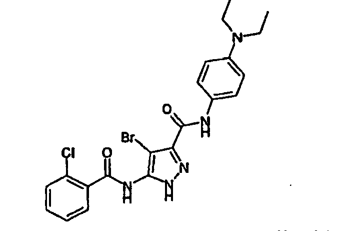

The pyrazole acid, prepared as described in General Procedure 1, was coupled with N,N-diethyl-1,4-phenylenediamine (Aldrich, 26,151-3) using the method described in General Procedure 3.
MS⁺(m/z) = 490.0
¹H-NMR (CDCl₃) δ 12.51 (br, 1H), 9.19 (s, 1H), 8.76 (s, 1H), 8.02 (d, J = 7.5 Hz, 1H), 7.55-7.41 (m, 5H), 6.64 (d, J = 9.0 Hz, 2 H), 3.33 (q, J = 7.0 Hz, 4 H), 1.15 (t, J = 7.0 Hz, 6H).

### Example 2

### Preparation of 4-Bromo-5-(2-chloro-benzoylamino)-1H-pyrazole-3-carboxylic acid (4-dimethylamino-phenyl)-amide

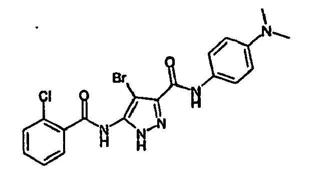

The pyrazole acid, prepared as described in General Procedure 1, was coupled with N,N-dimethyl-1,4-phenylenediamine (Aldrich, 19,399-2) using the method described in General Procedure 3.
MS+ = 462.1
¹H-NMR (DMSO-d6) δ 7.61-7.46 (m, 6H), 6.72 (d, J = 8.9 Hz, 2H), 2.87 (s, 6H).

### Example 3

### Preparation of 4-Bromo-5-(2-chloro-benzoylamino)-1H-pyrazole-3-carboxylic acid o- tolylamide

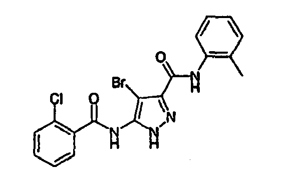

The pyrazole acid, prepared as described in General Procedure 1, was coupled with o-toluidine (Aldrich, 18,542-6) using the method described in General Procedure 3.
MS+ = 433.0
¹H-NMR (CDCl3) δ 12.68 (br, 1H), 9.32 (s, 1H), 8.92 (s, 1H), 8.04 (d, J = 8.0 Hz, 1H), 7.91 (d, J = 7.6 Hz, 1H), 7.53 (m, 2H), 7.38 (m, 1H), 7.23 (m, 2H), 7.13 (m, 1H), 2.35 (s,3H).

### Example 4

### Preparation of 5-(2-Chloro-benzoylamino)-4-methyl-1H-pyrazole-3-carboxylic acid phenylamide

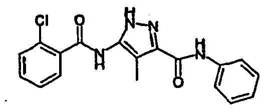

The pyrazole acid prepared as described in General Procedure 8 was coupled to aniline (Aldrich, 24,228-4 using the method described in General Procedure 3.
MS+ = 355.0
¹H-NMR (DMSO-d6) δ 7.82 (d, J = 7.7 Hz, 2H), 7.63-7.30 (m, 6H), 7.07 (t, J=7.7 Hz, 1H), 2.20 (s, 3H).

### Example 5

### Preparation of 5-(2-Chloro-benzoylamino)-4-ethyl-1H-pyrazole-3-carboxylic acid phenylamide

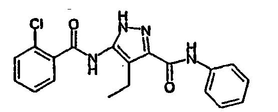

The pyrazole acid prepared from diethyl oxylate (**45**) and butyronitrile following the method of General Procedure 8 was coupled with aniline (Aldrich, 24,228-4) using the method of General Procedure 3.
MS+= 369.1
¹H-NMR (CD3OD) δ 7.72-7.34 (m, 8H), 7.14 (d, J = 7.3 Hz, 1H), 2.83 (q, J = 7.5 Hz, 2H), 1.21 (t, J = 6.6 Hz, 3H).

### Example 6

### Preparation of 5-(2-Chloro-benzoylamino)-4-propyl-1H-gyrazole-3-carboxylic acid phenylamide

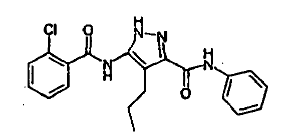

The pyrazole acid prepared from diethyl oxylate (45) and valeronitrile (Aldrich, 15,509-8) following the method of General Procdedure 8 was coupled with aniline (Aldrich, 24,228-7) using the method of General Procedure 3.
MS+= 383.0
¹H-NMR (DMSO-d6) δ 13.40 (br s, 1H), 10.54 (br s, 1H), 9.95 (br s, 1H), 7.79 (d, J = 7.9 Hz, 2H), 7.61-7.47 (m, 4H), 7.32 (t, J = 7.7 Hz, 2H), 7.07 (t, J = 7.3 Hz, 1H), 2.66 (t, J = 7.2 Hz, 2H),1.52 (m, 2H), .86 (t, J = 7.2 Hz, 3H).

### Example 7

### Preparation of 4-Bromo-5-(2-chloro-benzoylamino)-1H-pyrazole-3-carboxylic acid phenylamide

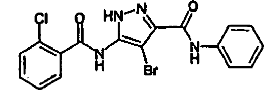

The pyrazole acid, prepared as described in General Procedure 1, was coupled with aniline (Aldrich, 24,228-4) using the method described in General Procedure 3.
MS+ = 419.0
¹H-NMR (DMSO-d6) δ 10.83 (br, 1H), 10.20 (br, 1H), 7.77 (m, 2H), 7.54 (m, 4H), 7.34 (m, 2H), 7.10 (m, 1H).

### Example 8

### Preparation of 4-Bromo-5-(2-chloro-benzoylamino)-1H-pyrazole-3-carboxylic acid methylphenyl-amide

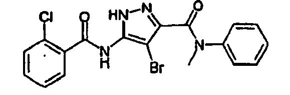

The pyrazole acid, prepared as described in General Procedure 1, was coupled with N-methylaniline (Aldrich, 23,623-3) using the method described in General Procedure 3.
MS+ = 434.9
¹H-NMR (CDCl3) δ 11.88 (br, 1H), 8.99 (br, 1H), 7.92 (d, J = 7.7 Hz, 1H), 7.47 (m, 2H), 7.41 (m, 1H), 7.23 (m, 5H), 3.49 (s, 3H).

### Example 9

### Preparation of 4-Bromo-5-(2-chloro-benzoylamino)-1H-pyrazole-3-carboxylic acid naphthalen-1-ylamide

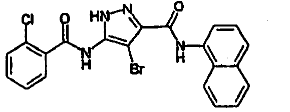

The pyrazole acid, prepared as described in General Procedure 1, was coupled with 1-naphthylamine (Fluka, 70731) using the method described in (General Procedure 3.
MS+ = 469.0
¹H-NMR (DMSO-d6) δ 10.86 (br, 1H), 10.26 (br, 1H), 7.74 (m, 11H).

### Example 10

### Preparation of 4-Bromo-5-(2-chloro-benzoylamino)-1H-pyrazole-3-carboxylic acid p-tolylamide

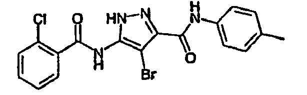

The pyrazole acid, prepared as described in General Procedure 1, was coupled withp-toluidine (Aldrich, 23,631-4) using the method described in General Procedure 3.
MS+ = 433.0

### Example 11

### Preparation of 4-Bromo-5-(2-chloro-benzoylamino)-1H-pyrazole-3-carboxylic acid (4-chloro-phenyl)-amide

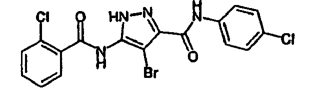

The pyrazole acid, prepared as described in General Procedure 1, was coupled with 4-chloroaniline (Aldrich, 47,722-2) using the method described in General Procedure 3.
MS+ = 452.9

### Example 12

### Preparation of 4-Bromo-5-(2-chloro-benzoylamino)-1H-pyrazole-3-carboxylic acid (3,4-dichloro-phenyl)-amide

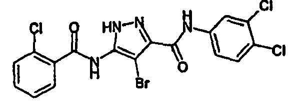

The pyrazole acid, prepared as described in General Procedure 1, was coupled with 3,4-dichloroaniline (Aldrich, 43,777-8) using the method described in General Procedure 3.
MS+ = 486.9

### Example 13

### Preparation of 4-Bromo-5-(2-chloro-benzoylamino)-1H-pyrazole-3-carboxylic acid (4-methoxy-phenyl)-amide

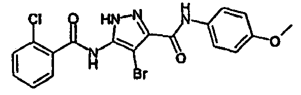

The pyrazole acid, prepared as described in General Procedure 1, was coupled with *p*-Anisidine (Fluka, 10490) using the method described in General Procedure 3.
MS+ = 448.9

### Example 14

### Preparation of 4-Bromo-5-(2-chloro-benzoylamino)-1H-pyrazole-3-carboxylic acid pyridin-3-ylamide

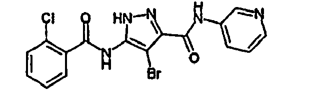

The pyrazole acid, prepared as described in General Procedure 1, was coupled with 2-Aminopyridine (Acros, 40106) using the method described in General Procedure 3.
MS+ = 419.9

### Example 15

### Preparation of 4-Bromo-5-(2-chloro-benzoylamino)-1H-pyrazole-3-carboxylic acid pyridin-4-ylamide

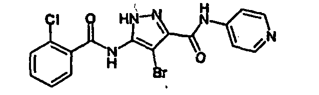

The pyrazole acid, prepared as described in General Procedure 1, was coupled with 4-aminopyridine (Fluka, 09370) using the method described in General Procedure 3.
MS+ = 419.9

### Example 16

### Preparation of 4-Bromo-5-(2-chloro-benzoylamino)-1H-pyrazole-3-carboxylic acid (2-chloro-phenyl)-amide

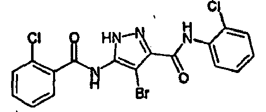

The pyrazole acid, prepared as described in General Procedure 1, was coupled with 2-chloroaniline (Fluka, 23300) using the method described in General Procedure 3.
MS+ = 452.9

### Example 17

### Preparation of 4-Chloro-5-(2-chloro-benzoylaminol-1H-pyrazole-3-carboxylic acid phenylamide

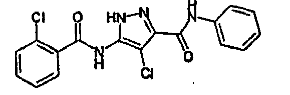

The title compound was prepared as shown in General Procedure 2 followed by coupling to aniline (Aldrich, 24,228-4) using the method described in General Procedure 3.
MS+ = 375.0
¹H- NMR (CD3OD) δ 7.86 (m, 1H), 7.48 (m, 7H), 7.15 (m, 1H).

### Example 18

### Preparation of 4-Bromo-5-(2-chloro-benzoylamino)-1H-pyrazole-3-carboxylic acid (3-hydroxy-4-methoxy-phenyl)-amide

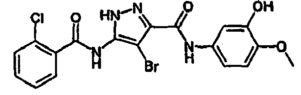

The pyrazole acid, prepared as described in General Procedure 1, was coupled with 5-Amino-2-methoxyphenol (Acros Organics, 33491) using the methods described in General Procedure 4.
MS+ = 465.0
¹H-NMR (CD₃OD) δ 7.64 (m, 1H), 7.48 (m, 3H), 7.26 (m, 1H, 7.09 (m, 1H), 6.91 (m, 1H), 3.86 (m, 3H).

### Example 19

### Preparation of 4-Bromo-5-(2-chloro-benzoylamino)-1H-pyrazole-3-carboxylic acid (6-methoxy-pyridin-3-yl)-amide

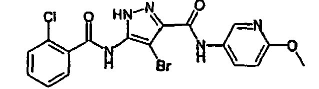

The pyrazole acid, prepared as described in General Procedure 1, was coupled with 5-amino-2-methoxypyridine (Pfaltz & Bauer Chemicals, A24130) using the method of General Procedure 4.
MS+ = 450.0
¹H- NMR (CD₃OD) δ 8.46 (m, 1H), 8.01 (m, 1H), 7.64 (m, 1H), 7.50 (m, 3H), 6.82 (d, J = 9.3 Hz, 1H), 3.91 (s, 3H).

### Example 20

### Preparation of 4-Bromo-5-(2-chloro-benzoylamino)-1H-pyrazole-3-carboxylic acid (3,5-dimethoxy-phenyl)-amide

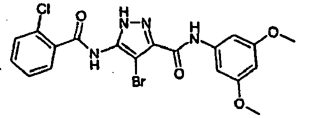

The pyrazole acid, prepared as described in General Procedure 1, was coupled with 3,5-dimethoxyaniline (Fluka, 38600) using the method of General Procedure 7.
MS+ = 479.0

### Example 21

### Preparation of 4-Bromo-5-(2-chloro-benzoylamino)-1H-pyrazole-3-carboxylic acid pyrazin-2-ylamide

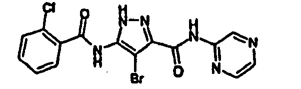

The pyrazole acid, prepared as described in General Procedure 1, was coupled with aminopyrazine (Fluka, 09332) using the method of General Procedure 7.
MS+ = 442.2 (M+Na)

### No Examples 22

### Reference Example 23

### Preparation of 4-Bromo-5-(2-chloro-benzoylamino)-2H-pyrazole-3-carboxylic acid (4-ethylphenyl)-amide

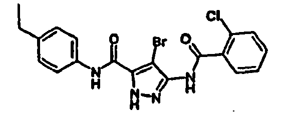

The pyrazole acid, prepared as described in General Procedure 1, was coupled with 4-ethylaniline (Fluka, 03070) using the method of General Procedure 7.
MS+ = 447.0

### Reference Example 24

### Preparation of 4-Bromo-5-(2-chloro-benzoylamino)-2H-pyrazole-3-carboxylic acid (4-sec-butyl-phenyl)-amide

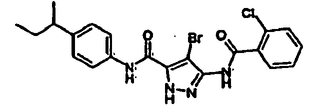

The pyrazole acid, prepared as described in General Procedure 1, was coupled with 4-sec-butylaniline (Fluka, 19559) using the method of General Procedure 7.
MS+ = 475.1

### Example 25

### Preparation of 4-Bromo-5-(2-chloro-benzoylamino)-1H-pyrazole-3-carboxylic acid (1-methyl-1H-pyrazol-3-yl)amide

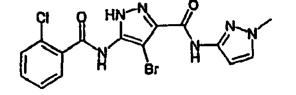

The pyrazole acid was prepared using the methods described in General Procedure 1 followed by coupling to 1-methyl-1H-pyrazol-3-amine (TimTec, Inc., TBB019586) using the method of General Procedure 3.
MS+423.0
¹H-NMR (CD₃OD δ 7.61 (m, 1H), 7.51 (m, 4H), 6.66 (m, 1H), 3.85 (s, 3H).

### Example 26

### Preparation of 4-Bromo-5-(2-chloro-benzoylamino)-1H-pyrazole-3-carboxylic acid (9H-fluoren-9-yl)-amide

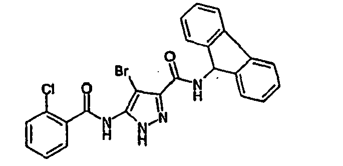

The pyrazole acid, prepared as described in General Procedure 1, was coupled with 9-aminofluorene hydrochloride (Acros, 26928) using the method described in General Procedure 3.
MS+ = 507.0
¹H-NMR (DMSO-d6) δ 8.75 (d, J = 8.3 Hz, 1H), 7.87 (d, J = 7.4 Hz, 2H), 7.58-7.31 (m, 13H), 6.14 (m, 1H).

### Example 27

### Preparation of 4-Bromo-5-(2-chlorobenzoylamino)-1H-pyrazole-3-carboxylic acid isoquinolin-1-ylamide

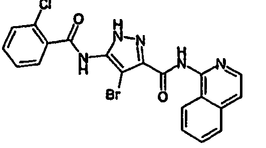

The pyrazole acid was prepared using the methods described in General Procedure 1 followed by coupling to 1-aminoisoquinoline (Aldrich, 17,859-4) using the method of General Procedure 3.
MS+ = 469.8
¹H-NMR (CDC13) δ 8.08 (m, 2H), 7.81 (m, 4H), 7.66 (m, 2H), 7.53 (m, 2H).

### Example 28

### Preparation of 4-Bromo-5-(2-chlorobenzoylamino)-1H-pyrazole-3-carboxylic acid isoquinolin-3-ylamide

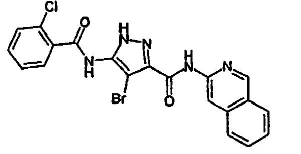

The pyrazole acid was prepared using the methods described in General Procedure 1 followed by coupling to 3-isoquinolinamine (Ryan Scientific, BTB 10019) using the method of General Procedure 3.
MS+ = 469.8
¹H-NMR (DMSO-d6) δ 8.47 (d, J = 6.7 Hz, 1H), 8.39 (d, J = 6.8 Hz,1H), 7.98 (d, J = 6.1 Hz, 1H), 7.88 (d, J = 6.3 Hz, 1H), 7.76 (m, 1H), 7.58 (m, 5H).

### Example 29

### Preparation of 4-Bromo-5-(2-chlorobenzoylamino)-1H-pyrazole-3-carboxylic acid (4-phenylthiazol-2-yl)amide

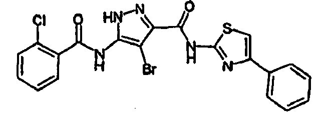

The pyrazole acid was prepared using the methods described in General Procedure 1 followed by coupling to 2-amino-4-phenylthiazole (Alfa, A18488) using the method of General Procedure 3.
MS+ = 502.0
¹H-NMR (DMSO-d6) δ 7.59 (m, 9H).

### Example 30

### Preparation of 4-Bromo-5-(2-chlorobenzoylamino)-1H-pyrazole-3-carboxylic acid [4-(4-pyridin-4-yl-piperazin-1-yl)phenyl]amide

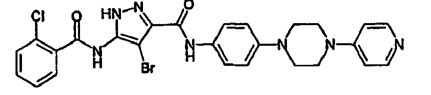

The pyrazole acid was prepared using the methods described in General Procedure 1 followed by coupling to 4-(4-pyridin-4-yl-piperazin-1-yl)phenylamine (WO 02/099388) using the method of General Procedure 3.
MS+ = 580.0
¹H-NMR (DMSO-d6) δ 14.05 (br, 1H), 13.55 (br, 1H), 10.08 (br, 1H), 8.28 (d, J=4.8 Hz, 2H), 7.61 (m, 6H), 7.26 (d, J = 4.7 Hz, 2H), 7.01 (d, J = 6.1 Hz, 2H), 3.87 (m, 4H), 3.33 (m, 4H).

### Example 31

### Preparation of 4-Bromo-5-(2-chlorobenzoylamino)-1H-pyrazole-3-carboxylic acid (4-[1,4'-bipiperidin]-1'-yl-phenyl)amide

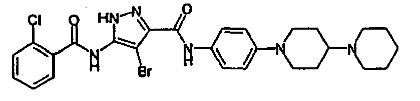

The pyrazole acid was prepared using the methods described in General Procedure 1 followed by coupling to 4-[1,4'-bipiperidin]-1'-yl-phenylamine (WO 02/099388) using the method of General Procedure 3.
MS+ = 585.1
¹H-NMR (DMSO-d6) δ 9.94 (br, 1H), 7.56 (m, 6H), 6.93 (d, J = 6.4 Hz, 2H), 4.10 (m, 2H), 3.70 (m, 2H), 2.60 (m, 4H), 2.36 (m, 1H), 1.81 (m, 2H), 1.55 (m, 6H), 1.40 (m, 2H).

### Example 32

### Preparation of 4-Bromo-5-(2-chlorobenzoylamino)-1H-pyrazole-3-carboxylic acid (1H-benzimidazol-2-yl)amide

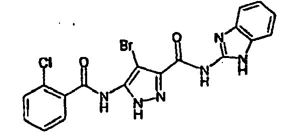

The pyrazole acid was prepared using the methods described in General Procedure 1 followed by coupling to 2-aminobenzimidazole (Aldrich, 17,177-8) using the method of General Procedure 3.
MS+= 458.9

### Example 33

### Preparation of 4-Bromo-5-(2-chlorobenzoylamino)-1H-pyrazole-3-carboxylic acid benzothiazol-2-ylamide

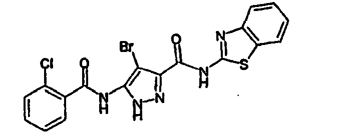

The pyrazole acid was prepared using the methods described in General Procedure 1 followed by coupling to 2-aminobenzothiazole (Aldrich, 10,881-2) using the method of General Procedure 3.
MS+ = 475.9

### Example 34

### Preparation of 4-Bromo-5-(2-chlorobenzoylamino)-1H-pyrazole-3-carboxylic acids (4,5-dihydro-1H-imidazol-2-yl)ethyl)phenyl)amide

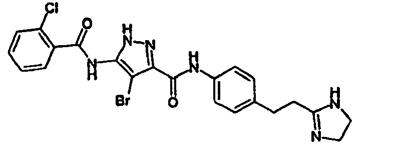

The title compound was prepared as described in General Procedure 13.
MS+ = 517.0
¹H-NMR (CD₃OD δ 7.67 (m, 2H), 7.55 (m, 4H), 7.26 (m, 2H), 3.99 (br m, 4H), 3.00 (br m, 2H), 2.85 (br m, 2H).

### Example 35

### Preparation of 4-Bromo-5-(2-chlorobenzoylamino)-1H-pyrazole-3-carboxylic acid (4-(3-aminopropyl)phenyl)amide

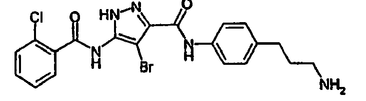

The pyrazole acid 7, prepared as described in Procedure 1 was coupled with aniline 70, prepared as described in Procedure 14, using the method of Procedure 4 followed by treatment with TFA.
MS+ = 476.0
¹H-NMR (DMSO-d6) δ 7.62 (m, 6H), 7.18 (m, 2H), 2.76 (m, 2H), 2.60 (m, 2H), 1.81 (m,2H).

### Examples 36

### Preparation of 4-Bromo-5-(2-chlorobenzoylamino)- 1H-Dymzole-3-carboxylic acid (4-(2-aminoethyl)phenyl-)amide

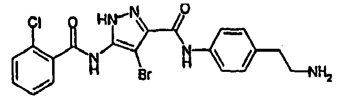

The pyrazole acid 7, prepared as described in Procedure 1 was coupled with (2-(4-aminophenyl)ethyl)carbamic acid tert-butyl ester (J & W PharmLab LLC, 20-0111) using the method of Procedure 4 followed by treatment with TFA.
MS+ = 462.1
¹H-NMR (DMSO-d6) δ 14.10 (br, 1H), 10.84 (br, 1H), 10.20 (br, 1H), 7.78 (m, 3H), 7.59 (m, 4H), 7.23 (m, 2H), 3.04 (m, 2H), 2.83 (m, 2H).

### Example 37

### Preparation of 4-bromo-5-(2-fluorobenzoylamino)-1H-pyrazole-3-carboxylic acid (4-(1,4'-bipiperidin]-1'-yl-phenyl)amide

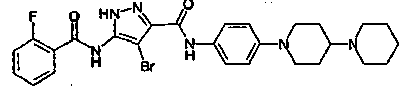

The pyrazole acid was prepared as described for compound 22 using amine 20 and 2-fluorobenzoyl chloride (Aldrich, 12,084-7) followed by bromination as shown in General Procedure 8. The amine is prepared as described in Internation patent application WO 02/099388, and coupled the pyrazole acid as shown in General Procedure 4.
MS+ = 569.2
¹H-NMR (DMSO-d6) δ 10.06 (br, 1H), 7.83 (m, 111), 7.70 (m, 3H), 7.48 (m, 2H), 7.01 (m, 2H), 4.12 (m, 2H), 2.69 (m, 2H), 2.59 (m, 4H), 2.43 (m, 1H), 1.88 (m, 2H), 1.47 (m, 6H), 1.27 (m, 2H).

### Example 38

### Preparation of 4-Bromo-5-(2-chlorobenzoylamino)-1H-pyrazole-3-carboxylic acid (4-(2-(1,4.5,6-tetrahydropyrimidin-2-yl)phenyl)amide

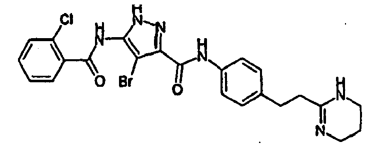

The title compound was prepared as shown in General Procedure 13 using propylene diamine.
MS+= 531.1
MS-NMR (CD3OD) δ 7.68 (d, 2H, J=8.7 Hz), 7.53 (m, 2H), 7.46 (m, 2H), 7.24 (d, 2H, J=8.7 Hz), 3.37 (m, 4H), 2.97 (t, 2H, J=7.2 Hz and 6.6 Hz), 2.69 (t, 2H, J=6.6 and 7.2 Hz), 1.92 (m, 2H).

### Example 39

### Preparation of 4-Bromo-5-(2-chlorobenzoylamino-1H-pyrazole-3-carbozylic acid (4-(4,5-dilydro-1 H-imidazol-2-yl)phenyl)amide

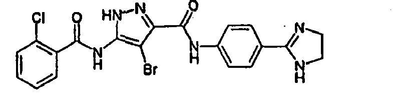

The pyrazole acid, prepared as described in General Procedure 1, was coupled to 4-aminobenzonitrile (Aldrich, 14,775-3), using the method of General Procedure 4. Treatment with ethylene diamine as described in Procedure 13 afforded the title compound.
MS+ = 487.0
¹H-NMR (DMSO-d6) δ 14.26 (br, 1H), 10.90 (br, 1H), 10.75 (br, 1H), 10.40 (br, 2H), 8.10 (m, 2H), 7.93 (m, 2H), 7.56 (m, 4H), 4.00 (s, 4H).

### Example 40

### Preparation of 4-Bromo-5-(2-chlorobenzoylamino)-1H-pyrazole-3-carbozylic acid (4-fluoro-3-cyano-phenyl)amide

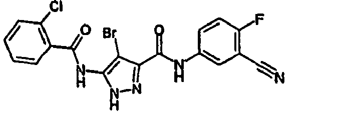

The pyrazole acid was prepared as shown in General Procedure 1. The amine was purchased from Oakwood (Cat number 013105). The amine and the pyrazole acid were coupled as shown in General Procedure 4.
MS+ = 461.9
¹H-NMR (DMSO-d6) δ 14.20 (b, 1H), 10.86 (b, 1H), 10.66 (s, 1H), 8.32 (b, 1H, 8.14 (b, 1H), 7.58 (m, 5H).

### Example 41

### Preparation of 4-Bromo-5-(2-chlorobenzoylamino)-1-methyl-pyrazole-3-carboxylic acid (4-[1,4'-bipiperidin]-1'-yl-phenyl)amide

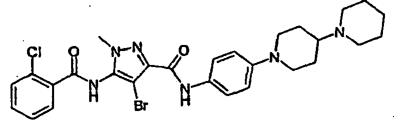

The pyrazole acid prepared by coupling methyl ester 73 (General Procedure 15) with 2-chlorobenzoyl chloride (21) followed by hydrolysis and bromination using the method of General Procedure e 1 was coupled with 4-[1,4'-bipiperidin]-1'-yl-phenylamine (WO 2/099388) using the method of General Procedure 4.
MS+ = 600.0
¹H-NMR (CD3OD) δ 6.69 (d, J=6.0 Hz, 1H), 7.59-7.49 (m, 5H), 7.02 (d, J=9.0 Hz, 2H), 3.98 (s, 3H), 3.77 (d, J=12.0 Hz, 2H), 2.75-2.67 (m, 6H), 2.53 (m, 1H, 2.06-2.03 (m, 2H), 1.78-1.67 (m, 6H), 1.53 (m, 2H)

### Example 42

### Preparation of 4-Bromo-5-(2-chlorobenzoylamino)-1-methyl-pyrazole-3-carboxylic acid[14-(4-pyridin-4-yl-piperazin-1-yl)phenyl]amide

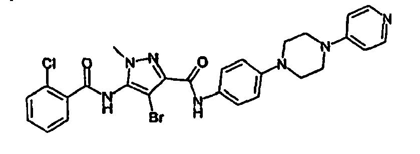

The pyrazole acid prepared by coupling methyl ester 73 (General Procedure 15) with 2-chlorobenzoyl chloride (21) followed by hydrolysis and bromination using the method of General Procedure 1 was coupled with 4-(4-pyridin-4-yl-piperazin-1-yl)phenylamine (WO 2/099388) using the method of General Procedure 4.
MS+ = 594.0
¹H-NMR (CD3OD) δ 8.15 (d, J=5.7 Hz, 2H), 7.65-7.49 (m, 3H), 7.42-7.38 (m, 3H), 7.03 (d, J=9.0 Hz, 2H), 6.90 (d, J=6.3 Hz, 2H), 3.89 (s, 3H), 3.61-3.55 (m, 4H), 3.33-3.25 (m, 4H)

### Example 43

### Preparation of 4-methyl-5-(2-fluorobenzoylamino)-1-t-butyl-pyrazole-3-carboxylic acid (4-(2-cyanoethyl)phenyl)amide

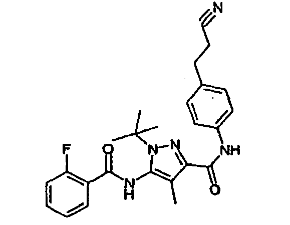

The pyrazole acid prepared by coupling methyl ester 48 with 2-fluorobenzoyl chloride (Aldrich, 12,084-7) using the method of General Procedure 8 followed by hydrolysis using the method of General Procedure 9 was coupled with amine 66 using the method of General Procedure 4.
MS+ = 448.1
¹H-NMR (CDC13) δ 8.76 (s, 1H), 8.14 (t, 1H, J=7.3 Hz), 8.01 (d, 1H, J=14.1 Hz), 7.64 (d, 2H, J=8.2 Hz), 7.59 (m, 1H), 7.34 (t, 1H, J=7.6 Hz), 7.20 (d, 2H, J=8.2 Hz), 2.93 (t, 2H, J=7.8 Hz), 2.61 (t, 2H, J=7.8 Hz), 2.21 (s, 3H), 1.67 (s, 9H).

### Example 44

### Preparation of 4-methyl-5-(2-fluorobenzoylamino)-1-(t-butyl)-pyrazole-3-carboxylic acid (4-(2-(4 5-dihydro-1H-imidazol-2-yl)ethyl)phenyl)amide

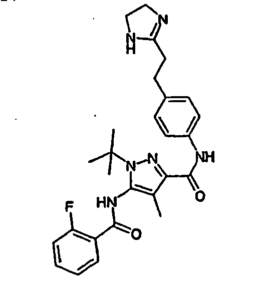

The compound from Example 43 was treated with ethylene diamine using the method of Procedure 13.
MS+ = 491.2
¹H-NMR (CDC13) δ 8.85 (s, 1H), 8.24 (d, 1H, J=13.1 Hz), 8.07 (t, 1H, J=7.7 Hz), 7.58 (m, 1H), 7.47 (d, 2H, J=8.2 Hz), 7.32-7.18 (m, 2H), 7.08 (d, 2H, J=8.2 Hz), 2.89 (m, 2H), 2.69 (m, 2H), 2.23 (br s, 4H), 2.12 (s, 3H), 1.64 (s, 9H).

### Example 45

### Preparation of 4-methyl-5-(2-fluorobenzoylamino)-1H-pyrazole-3-carboxylic acid (4-(2-(4,5-dihydro-1H-imidazol-2-yl)ethyl)phenyl)amide

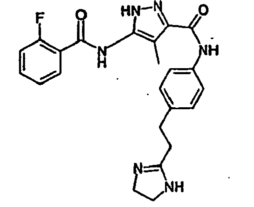

The pyrazole acid, prepared as described in General Procedure 8 using 2-fluorobenzoyl chloride instead of compound 21, was coupled to 4-aminobenzyl cyanide (Aldrich, A4,205-0) using the method of General Procedure 4. Treatment with ethylene diamine using the method of General Procedure 13 afforded the title compound.
MS+ = 435.2

### Example 46

### Preparation of 4-methyl-5-(2-fluorobenzoylamino)-1-phenyl-pyrazole-3-carboxylic acid (4-(2-(4,5-dihydro-1H-imidazol-2-yl)ethyl)phenyl)amide

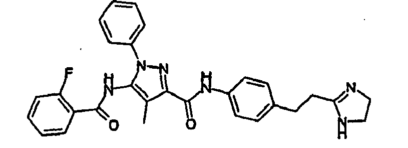

The compound from Example 47 was treated with ethylene diamine using the method of General Procedure 13.
MS+ = 511.2
¹H-NMR (CD-3OD) δ 7.43-7.71 (m, 5H), 7.23-7.32 (m, 3H), 3.85 (s, 3H, 3.31 (s, 2H), 2.78-3.01 (m, 4H), 2.32 (s, 2H).

### Example 47

### Preparation of 4-methyl-5-(2-fluorobenzoylamino)-1-phenyl-pyrazole-3-carboxylic acid (4-(2-cyanoethyl)phenyl)amide

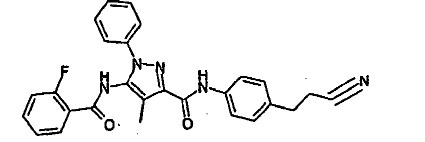

The pyrazole acid, prepared as described in General Procedure 1 using 5-amino-4-methyl-1-phenyl-1H-pyrazole-3-carboxylic acid ethyl ester (prepared as described in General Procedure 16 using 3-cyano-3-methyl-2-oxopropanoic acid ethyl ester (US 4652669)) in place of compound 20 and 2-fluorobenzoyl chloride in place of compound 21, was coupled to 4-aminobenzyl cyanide (Aldrich, A4,205-0)) using the method of General Procedure 10.
MS+ = 490.1
¹H-NMR (CDC13) δ 8.89 (s, 1H), 8.07-8.18 (m, 2H), 7.72 (d, 2H, J=9.3 Hz), 7.60-7.64 (m, 3H), 7.47-7.56 (m, 3H), 7.37 (t, 1H, J=7.5 Hz), 7.20-7.28 (m, 3H), 3.00 (t, 2H, J=7.5 Hz), 2.67 (t, 2H, J=7.5 Hz), 2.43 (s, 3H).

### Example 48

### Preparation of 4-Bromo-5-(2-chlorobenzoylamino)-1-methyl-pyrazole-3-carboxylic acid (4-(3-aminopropyl)phenyl)amide

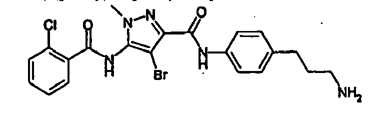

The pyrazole acid, prepared by coupling methyl ester 73 (General Procedure 15) with 2-benzoyl chloride (21) followed by hydrolysis and bromination as shown in General Procedure 1, was coupled to aniline 70 (General Procedure 14) using the method of General Procedure 4. Treatment with TFA afforded the title compound.
MS+ = 490.0
¹H-NMR (DMSO-d6) δ 7.60 (m, 6H), 7.16 (m, 2H), 3.87 (s, 3H), 2.76 (m, 2H), 2.59 (m, 2H), 1.79 (m, 2H).

### Example 49

### Preparation of 4-Bromo-5-(2-chlorobenzoylamino)-1-methyl-pylazole-3-carboxylic acid (4-(2-(4,5-dihydro-1H-imidazol-2-yl)ethyl)phenyl)amide

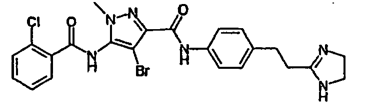

The pyrazole acid, prepared by coupling methyl ester 73 (General Procedure 15) with 2-benzoyl chloride (21) followed by hydrolysis and bromination as shown in General Procedure 1, was coupled to 4-aminobenzyl cyanide (Aldrich, A4,205-0) using the method of General Procedure 4. Treatment with ethylene diamine using the method of General Procedure 13 afforded the title compound.
MS+ = 529.1
¹H-NMR (DMSO-d6) δ 7.73 (m, 3H), 7.52 (m, 3H), 7.24 (m, 2H), 3.91 (s, 3H), 3.80 (s, 4H), 2.96 (m, 2H), 2.83 (m, 2H).

### BIOLOGICAL EXAMPLE

The potency and efficacy to inhibit the bradykinin B₁ receptor was determined for the compounds of this invention in a cell-based fluorescent calcium-mobilization assay. The assay measures the ability of test compounds to inhibit bradykinin B₁ receptor agonist-induced increase of intracellular free Ca⁺² in a native human bradykinin B₁ receptor-expressing cell line.

In this example, the following additional abbreviations have the meanings set forth below. Abbreviations heretofore defined are as defined previously. Undefined abbreviations have the art recognized meanings.
- BSA =: bovine serum albumin
- DMSO =: dimethylsulfoxide
- FIBS =: fetal bovine serum
- MEM =: minimum essential medium
- mM =: millimolar
- ng =: nanogram
- µg =: micrograms
- µM =: micromolar

Specifically, calcium indicator-loaded cells are pre-incubated in the absence or presence of different concentrations of test compounds followed by stimulation with selective bradykinin B1 receptor agonist peptide while Ca-dependent fluorescence is monitored.

IMR-90 human lung fibroblast cells (CCL 186, American Type Tissue Collection) are grown in MEM supplemented with 10% FBS as recommended by ATCC. Confluent cells are harvested by trypsinization and seeded into black wall/clear bottom 96-well plates (Costar #3904) at approximately 13,000 cells/well. The following day, cells are treated with 0.35 ng/mL interleukin-1ß in 10% FBS/MEM for 2 hours to up-regulate bradykinin B₁ receptors. Induced cells are loaded with fluorescent calcium indicator by incubation with 2.3 µM Fluo-4/AM (Molecular Probes) at 371C for 1.5 hrs in the presence of an anion transport inhibitor (2.5 mM probenecid in 1% FBS/MEM). Extracellular dye is removed by washing with assay butter (2.5 mM probenecid, 0.1% BSA, 20 mM HEPES in Hank's Balanced Salt Solution without bicarbonate or phenol red, pH 7.5) and cell plates are kept in dark until used. Test compounds are assayed at 7 concentrations in triplicate wells. Serial dilutions are made in half log-steps at 100-times final concentration in DMSO and then diluted in assay buffer. Compound addition plates contain 2.5-times final concentrations of test compounds or controls in 2.5% DMSO/assay buffer. Agonist plates contain 5-times the final concentration of 2.5 nM (3 x EC50) bradykinin B₁ receptor agonist peptide des-Arg¹⁰-kallidin (DAKD, Bachem) in assay buffer. Addition of test compounds to cell plate, incubation for 5 min at 351C, followed by the addition of bradykinin B₁ receptor agonist DAKD is carried out in the Fluorometric Imaging Plate Reader (FLIPR, Molecular Devices) while continuously monitoring Ca-dependent fluorescence. Peak height of DAKD-induced fluorescence is plotted as function of concentration of test compounds. IC₅₀ values are calculated by fitting a 4-parameter logistic function to the concentration-response data using non-linear regression (Xlfit, IDBS (ID Business Solutions Ltd.)).

Typical potencies observed for bradykinin B₁, receptor agonist peptides are Ec₅₀ approximately 0.8 nM and approximately 100 nM for des-Arg¹⁰-kallidin and des-Arg⁹-bradyldnin, respectively, while for bradykinin B₁ receptor antagonist peptide des-Arg¹⁰, Leu⁹-kallidin IC₅₀ is approximately 1 nM.

The compounds of this invention have potency in the above assay as demonstrated by results of less than 50 micromolar. It is advantageous that the assay results be less than 1 micromolar, even more advantageous for the results to be less than 0.5 micromolar.

In view of the above, all of these compounds exhibit bradykinin B₁ receptor antagonistic properties and, accordingly, are useful in treating disease conditions mediated at least in part by bradykinin B₁ receptor.

## Claims

1. A compound selected from compounds of Formula (I) and Formula (II) and pharmaceutically acceptable salts thereof: 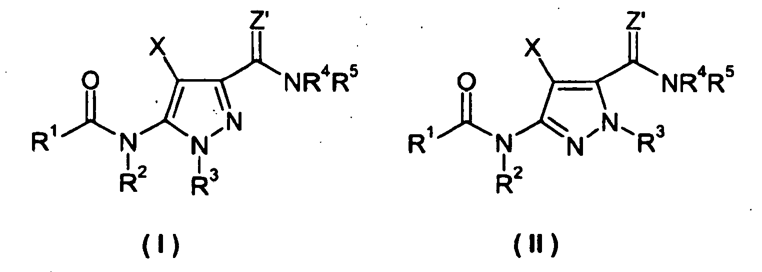 wherein:
Z' is selected from: O, S, and NH;
R¹ is selected from: 2-chlorophenyl and 2-fluorophenyl;
R² is selected from:
hydrogen,
alkyl, and substituted alkyl;
R³ is selected from:
hydrogen,
alkyl, substituted alkyl,
alkenyl, substituted alkenyl,
alkynyl, substituted alkynyl,
aryl, substituted aryl,
cycloalkyl, substituted cycloalkyl,
heteroaryl, substituted heteroaryl,
heterocyclic, and substituted heterocyclic;
R⁴ is selected from:
aryl, substituted aryl,
heteroaryl, and substituted heteroaryl;
R⁵ is selected from:
hydrogen,
alkyl, and substituted alkyl;
X is selected from:
hydrogen,
alkyl, substituted alkyl,
alkoxy, substituted alkoxy,
aryl, substituted aryl,
carboxyl,
carboxyl esters,
cyano,
halo,
heteroaryl, substituted heteroaryl,
hydroxy,
nitro,
amino, substituted amino,
acylamino, and
aminoacyl;
with the proviso that the compound of Formula (I) is not
(A') 4-Bromo-5-(2-chloro-benzoylamino)-1H-pyrazole-3-carboxylic acid phenylamide.

2. A compound according to claim 1, wherein R¹ is:
2-chlorophenyl.

3. A compound according to claim 1 or claim 2, wherein R² is selected from:
hydrogen,
methyl,
ethyl,
iso-propyl,
2-methoxyeth-1-yl,
pyrid-3-ylmethyl,
phenyl,
benzyl,
t-butyl, and
t-butoxycarbonyl-methyl.

4. A compound according to any one of claims 1 to 3, wherein R³ is selected from:
hydrogen,
C₁₋₄ alkyl,
monocyclic aryl, substituted monocyclic aryl,
monocyclic heteroaryl, and substituted monocyclic heteroaryl.

5. A compound according to any one of claims 1 to 4, wherein R⁵ is selected from:
hydrogen,
methyl,
ethyl,
iso-propyl,
2-methoxyethyl, and
pyrid-3-yl-methyl.

6. A compound according to any one of claims 1 to 5, wherein R⁴ is selected from:
4-(N,N-diethylamino)phenyl;
4-(N,N-dimethylamino)phenyl;
2-methylphenyl;
phenyl;
1-naphthyl;
4-methylphenyl;
4-chlorophenyl;
3,4-dichlorophenyl;
4-methoxyphenyl;
pyridin-3-yl;
pyridin-4-yl;
2-chlorophenyl;
4-methoxy-3-hydroxyphenyl;
2-methoxypyridin-5-yl;
3,5-Dimethoxyphenyl;
pyrazin-2-yl;
4-ethylphenyl;
4-(1-(RorS)-1-methylprop-1-yl);
1-methyl-1 H-pyra2ol-3-yl;
9H-fluoren-9-yl;
isoquinolin-1-yl;
isoquinolin-3-yl;
4-phenylthiazol-2-yl;
4-(4-pyridin-4-yl-piperazin-1-yl)phenyl;
4-[1,4'-bipiperidin]-1'-yl-phenyl;
1H-benzimidazol-2-yl;
benzothiazol-2-yl;
4-(2-(4,5-dihydro-1H-imidazol-2-yl)ethyl)phenyl;
4-(3-aminopropyl)phenyl;
4-(2-aminoethyl)phenyl;
4-[1,4'-bipiperidin]-1'-yl-phenyl;
4-(2-(1,4,5,6-tetrahydropyrimidin-2-yl)ethyl)phenyl;
4-(4,5-dihydro-1H-imidazol-2-yl)phenyl;
4-fluoro-3-cyano-phenyl; and
4-(2-cyanoethyl)phenyl.

7. A compound according to any one of claims 1 to 5, wherein R⁴ is selected from:
4-(4-pyridin-4-y)-piperazin-1-yl)pheny),
4-(N,N-diethylamino)phenyl,
4-(N,N-dimethylamino)phenyl, and
4-[1,4'-bipiperidin]-1'-yl-phenyl.

8. A compound according to any one of claims 1 to 5, wherein R⁴ is selected from:
2-ethoxyphenyl;
3-(2-methylthiazol-5-yl)-pyrazol-5-yl; pyrazol-2-yl;
4-aminophenylamino;
4-(1H-imidazol-2-ylmethyl)-phenylamino;
4-[2-(1H-imidazol-2-yl)-ethyl]-phenylamino;
4-aminomethyl-phenylamino;
4-(1H-imidazol-2-yl)-phenylamino;
4-[N, N'-diethylamidino]-phenylamino;
4-[N,N'-dimethylamidino]-phenylamino;
4-[N,N'-diphenylamidino]-phenylamino;
4-(4,5-dihydro-1H-imidazo)-2-ylmethyl)-phenylamino;
4-(1H-benzimidazol-2-yl)-phenylamino;
4-[N-(4,5-dihydro-1H-imidazol-2-yl)aminomethyl]-phenylamino;
4-(1H-benzimidazol-2-ylmethyl)-phenylamino;
4-(1,4,5,6-tetrahydro-pyrimidin-2-yl)-phenylamino; and
4-(1,4,5,6-tetrahydro-pyrimidin-2-ylmethyl)-phenylamino.

9. A compound according to any one of claims 1 to 8, wherein X is selected from:
hydrogen,
bromine,
chlorine,
fluorine, and
methyl.

10. A compound according to claim 1, selected from the following compounds and pharmaceutically acceptable salts thereof:
(1) 4-bromo-5-(2-chlorobenzoylamino)-1H-pyrazole-3-carboxylic acid (4-diethylaminophenyl)amide;
(2) 4-bromo-5-(2-chlorobenzoylamino)-1H-pyrazole-3-carboxylic acid (4-dimethylaminophenyl)amide;
(3) 4-bromo-5-(2-chlorobenzoylamino)-1H-pyrazole-3-carboxylic acid o-tolylamide;
(4) 5-(2-chlorobenzoylamino)-4-methyl-1H-pyrazole-3-carboxylic acid phenylamide;
(5) 5-(2-chlorobenzoylamino)-4-ethyl-1H-pyrazole-3-carboxylic acid phenylamide;
(6) 5-(2-chlorobenzoylamino)-4-propyl-1H-pyrazole-3-carboxylic acid phenylamide;
(7) 4-bromo-5-(2-chlorobenzoylamino)-1H-pyrazole-3-carboxylic acid phenylamide;
(8) 4-bromo-5-(2-chlorobenzoylamino)-1H-pyrazole-3-carboxylic acid methylphenyl-amide;
(9) 4-bromo-5-(2-chlorobenzoylamino)-1H-pyrazole-3-carboxylic acid naphthalene-1-ylamide;
(10) 4-bromo-5-(2-chlorobenzoylamino)-1H-pyrazole-3-carboxylic acid p-tolylamide;
(11) 4-bromo-5-(2-chlorobenzoylamino)-1H-pyrazole-3-carboxylic acid (4-chlorophenyl)amide;
(12) 4-bromo-5-(2-chlorobenzoylamino)-1H-pyrazole-3-carboxylic acid (3,4-dichlorophenyl)amide;
(13) 4-bromo-5-(2-chlorobenzoylamino)-1H-pyrazole-3-carboxylic acid (4-methoxyphenyl)amide;
(14) 4-bromo-5-(2-chlorobenzoylamino)-1H-pyrazole-3-carboxylic acid pyridine-3-ylamide;
(15) 4-bromo-5-(2-chlorobenzoylamino)-1H-pyrazole-3-carboxylic acid pyridine-4-ylamide;
(16) 4-bromo-5-(2-chlorobenzoylamino)-1H-pyrazole-3-carboxylic acid (2-chlorophenyl)amide;
(17) 4-chloro-5-(2-chlorobenzoylamino)-1H-pyrazole-3-carboxylic acid phenylamide;
(18) 4-bromo-5-(2-chlorobenzoylamino)-1H-pyrazole-3-carboxylic acid (3-hydroxy-4-methoxyphenyl)amide;
(19) 4-bromo-5-(2-chlorobenzoylamino)-1H-pyrazole-3-carboxylic acid (6-methoxypyridin-3-yl)amide;
(20) 4-bromo-5-(2-chlorobenzoylamino)-1H-pyrazole-3-carboxylic acid (3,5-dimethoxyphenyl)amide;
(21) 4-bromo-5-(2-chlorobenzoylamino)- 1H-pyrazole-3-carboxylic acid pyrazin-2-ylamide;
(22) 4-bromo-5-(2-chlorobenzoylamino)-2H-pyrazole-3-carboxylic acid (4-ethylphenyl)amide;
(23) 4-bromo-5-(2-chlorobenzoylaminor2H-pyrazole-3-carboxylic acid (4-sec-butylphenyl)amide;
(24) 4-bromo-5-(2-chlorobenzoylamino)-1H-pyrazole-3-carboxylic acid (1-methyl-1H-pyrazol-3-yl)amide;
(25) 4-bromo-5-(2-chlorobenzoylamino)-1H-pyrazole-3-carboxylic acid (9H-fluoren-9-yl)amide;
(26) 4-bromo-5-(2-chlorobenzoylamino)-1H-pyrazole-3-carboxylic acid isoquinolin-1-ylamide;
(27) 4-bromo-5-(2-chlorobenzoylamino)-1 H-pyrazole-3-carboxylic acid isoquinolin-3-ylamide;
(28) 4-bromo-5-(2-ch)orobenzoytamino)-1H-pyrazole-3-carboxylic acid (4-phenylthiazol-2-yl)amide;
(29) 4-bromo-5-(2-chlorobenzoylamino)-1H-pyrazole-3-carboxylic acid [4-(4-pyridin-4-yl-piperazin-1-yl)phenyl]amide;
(30) 4-bromo-5-(2-chlorobenzoylamino)-1H-pyrazole-3-carboxylic acid (4-[1,4'-bipiperidin]-1'-yl-phenyl)amide;
(31) 4-bromo-5-(2-chlorobenzoylamino)-1H-pyrazole-3-carboxylic acid (1H-benzimidazol-2-yl)amide;
(32) 4-bromo-5-(2-chlorobenzoy)amino)-1H-pyrazole-3-carboxylic acid benzothiazol-2-ylamide;
(33) 4-bromo-5-(2-chlorobenzoylamino)-1 H-pyrazole-3-carboxylic acid (4-(2-(4,5-dihydro-1 H-imidazol-2-yl)ethyl)phenyl)amide;
(34) 4-bromo-5-(2-chlorobenzoylamino)-1H-pyrazole-3-carboxylic acid (4-(3-aminopropyl)phenyl)amide;
(35) 4-bromo-5-(2-chlorobenzoylamino)-1H-pyrazole-3-carboxylic acid (4-2-aminoethyl)phenyl)amide;
(36) 4-bromo-5-(2-fluorobenzoylamino)-1 H-pyrazole-3-carboxylic acid (4-[1,4'-bipiperidin]-1-yl-phenyl)amide:
(37) 4-bromo-5-(2-chlorobenzoylamino)-1H-pyrazole-3-carboxylic acid (4-(2-(1,4,5,6-tetrahydropyrimidin-2-yl)ethyl)phenyl)amide;
(38) 4-bromo-5-(2-chlorobenzoylamino)-1H-pyrazole-3-carboxylic acid (4-(4,5-dihydro-1 H-imidazol-2-yl)phenyl)amide;
(39) 4-bromo-5-(2-chlorobenzoylamino)-1H-pyrazole-3-carboxylic acid (4-fluoro-3-cyano-phenyl)amide;
(40) 4-bromo-5-(2-chlorobenzoylamino)-1-methyl-pyrazole-3-carboxylic acid (4-[1,4'-bipiperidin]-1'-yl-phenyl)amide;
(41) 4-bromo-5-(2-chlorobenzoylamino)-1-methyl-pyrazole-3-carboxylic acid [4-(4-pyridin-4-yl-piperazin-1-yl)phenyl]amide;
(42) 4-methyl-5-(2-fluorobenzoylamino)-1-*t*-butyl-pyrazole-3-carboxylic acid (4-(2-cyanoethyl)phenyl)amide;
(43) 4-methyl-5-(2-fluorobenzoylamino)-1-t butyl-pyrazole-3-carboxylic acid (4-(2-(4,5-dihydro-1 H-imidazol-2-yl)ethyl)phenyl)amide;
(44) 4-methyl-5-(2-fluorobenzoylamino)-1H-pyrazole-3-carboxylic acid (4-(2-(4,5-dihydro-1 H-imidazol-2-yl)ethyl)phenyl)amide;
(45) 4-methyl-5-(2-fluorobenzoylamino)-1-phenyl-pyrazole-3-carboxylic acid (4-(2-(4,5-dihydro-1H-imidazol-2-yl)ethyl)phenyl)amide;
(46) 4-methyl-5-(2-fluorobenzoylamino)-1-phenyl-pyrazole-3-carboxylic acid (4-(2-cyanoethyl)phenyl)amide;
(47) 4-bromo-5-(2-chlorobenzoylamino)-1-methyl-pyrazole-3-carboxylic acid (4-(3-aminopropyl)phenyl)amide; and
(48) 4-bromo-5-(2-chlorobenzoylamino)-1-methyl-pyrazole-3-carboxylic acid (4-(2-(4,5-dihydro-1H-imidazol-2-yl)ethyl)phenyl)amide.

11. A pharmaceutical composition comprising a pharmaceutically acceptable carrier and a therapeutically amount of a compound according to any one of claims 1 to 10.

12. A pharmaceutical composition comprising a pharmaceutically acceptable carrier and a therapeutically effective amount of a compound according to any one of claims 1 to 10, or a mixture thereof, effective to treat or palliate adverse symptoms in mammals mediated by bradykinin B₁ receptor.

13. A pharmaceutical composition comprising a pharmaceutically acceptable carrier and a therapeutically effective amount of a compound according to any one of claims 1 to 10, or a mixture thereof, effective to treat or palliate adverse symptoms in mammals associated with up-regulating bradykinin B₁ receptor following tissue damage or inflammation.

14. A method for selectively inhibiting bradykinin B₁ receptor over bradykinin B₂ receptor, in vitro, which method comprises using a compound according to any one of claims 1 to 10.

15. A method for determining bradykinin B₁ receptor agonist levels in a biological sample, in vitro, which method comprises contacting said biological sample with a compound according to any one of claims 1 to 10, at a predetermined concentration.

16. A compound according to any one of claims 1 to 10, for use in a method of treatment of human or animal body by therapy.

17. A compound according to any one of claims 1 to 10, for use in a method of treating or palliating adverse symptoms in mammals mediated by bradykinin B₁ receptor.

18. Use of a compound according to any one of claims 1 to 10, in the manufacture of a medicament for treating or palliating adverse symptoms in mammals mediated by bradykinin B₁ receptor.

19. A compound according to any one of claims 1 to 10, for use in a method of treating or palliating adverse symptoms in mammals associated with tissue damage or inflammation.

20. Use of a compound according to any one of claims 1 to 10, in the manufacture of a medicament for treating or palliating adverse symptoms in mammals associated with tissue damage or inflammation.

21. A compound according to any one of claims 1 to 10, for use in a method of treating or palliating adverse symptoms associated with the presence or secretion of bradykinin B₁ receptor agonists in mammals.

22. Use of a compound according to any one of claims 1 to 10, in the manufacture of a medicament for treating or palliating adverse symptoms associated with the presence or secretion of bradykinin B₁ receptor agonists in mammals.

23. A compound according to any one of claims 1 to 10, for use in a method of treating or ameliorating pain, inflammation, septic shock or the scarring process in mammals mediated by bradykinin B₁ receptor in such mammals.

24. Use of a compound according to any one of claims 1 to 10, in the manufacture of a medicament for treating or ameliorating pain, inflammation, septic shock or the scarring process in mammals mediated by bradykinin B₁ receptor in such mammals.

25. A compound according to any one of claims 1 to 10, for use in a method of treating or ameliorating adverse symptoms associated with burns, perioperative pain, migraine, shock, central nervous system injury, asthma, rhinitis, premature labor, inflammatory arthritis, inflammatory bowel disease or neuropathic pain.

26. Use of a compound according to any one of claims 1 to 10, in the manufacture of a medicament for treating or ameliorating adverse symptoms associated with burns, perioperative pain, migraine, shock, central nervous system injury, asthma, rhinitis, premature labor, inflammatory arthritis, inflammatory bowel disease or neuropathic pain.

## Patentansprüche

1. Eine Verbindung, die aus den Verbindungen der Formel (1) und Formel (II) und pharmazeutisch zulässigen Salzen dieser ausgewählt wird: 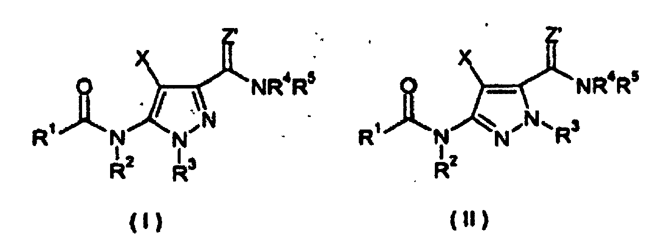 worin:
Z' ausgewählt wird aus: O, S und NH;
R¹ ausgewählt wird aus: 2-Chlorphenyl und 2-Fluorphenyl;
R² ausgewählt wird aus:
Wasserstoff,
Alkyl und substituiertes Alkyl;
R³ ausgewählt wird aus:
Wasserstoff,
Alkyl, substituiertes Alkyl,
Alkenyl, substituiertes Alkenyl,
Alkynyl, substituiertes Alkynyl,
Aryl, substituiertes Aryl,
Cycloalkyl, substituiertes Cycloalkyl,
Heteroaryl, substituiertes Heteroaryl,
heterozyklische und substituierte heterozyklische Gruppe;
R⁴ ausgewählt wird aus:
Aryl, substituiertes Aryl,
Heteroaryl, substituiertes Heteroaryl;
R⁵ ausgewählt wird aus:
Wasserstoff,
Alkyl und substituiertes Alkyl;
X ausgewählt wird aus:
Wasserstoff,
Alkyl, substituiertes Alkyl,
Alkoxy, substituiertes Alkoxy,
Aryl, substituiertes Aryl,
Carboxyl,
Carboxylester,
Cyano,
Halogen,
Heteroaryl, substituiertes Heteroaryl,
Hydroxy,
Nitro,
Amino, substituiertes Amino,
Acylamino und
Aminoacyl;
unter der Bedingung, dass die Verbindung der Formel (1) nicht
(A') 4-Brom-5-(2-chlorbenzoylamino)-1H-pyrazol-3-carboxylsäure-phenylamid ist.

2. Eine Verbindung gemäß Anspruch 1, worin R¹ ist:
2-Chlorphenyl

3. Eine Verbindung gemäß Anspruch 1 oder Anspruch 2, worin R² ausgewählt wird aus:
Wasserstoff,
Methyl,
Ethyl,
Isopropyl,
2-Methoxyethyl-1-yl,
Pyrid-3-yl-methyl
Phenyl,
Benzyl,
t-Butyl und
t-Butoxycarbonylmethyl

4. Eine Verbindung gemäß einer der Ansprüche 1 bis 3, worin R³ ausgewählt wird aus:
Wasserstoff,
C₁₋₄-alkyl,
monozyklisches Aryl, substituiertes monozyklisches Aryl,
monozyklisches Heteroaryl und substituiertes monozyklisches Heteroaryl.

5. Eine Verbindung gemäß einer der Ansprüche 1 bis 4, worin R⁵ ausgewählt wird aus:
Wasserstoff,
Methyl,
Ethyl,
Isopropyl,
2-Methoxyethyl und
Pyrid-3-yl-methyl.

6. Eine Verbindung gemäß einer der Ansprüche 1 bis 5, worin R⁴ ausgewählt wird aus:
4-(N,N-diethylamino)phenyl;
4-(N,N-dimethylamino)phenyl;
2-Methylphenyl;
Phenyl;
1-Naphthyl;
4-Methylphenyl;
4-Chlrophenyl;
3,4-Dichlorphenyl;
4-Methoxyphenyl;
Pyridin-3-yl;
Pyridin-4-yl;
2-Chlorphenyl;
4-Methoxy-3-hydroxyphenyl;
2-Methoxypyridin-5-yl;
3,5-Dimethoxyphenyl;
Pyrazin-2-yl;
4-Ethylphenyl;
4-(1-(RorS)-1-methylprop-1-yl);
1-Methyl-1 H-pyrazol-3-yl;
9H-Fluoren-9-yl;
Isochinolin-1-yl;
Isochinolin-3-yl;
4-Phenylthiazol-2-yl;
4-(4-Pyridin-4-yl-piperazin-1-yl)phenyl;
4-[1,4'-Bipiperidin]-1'-yl-phenyl;
1H-Benzimidazol-2-yl;
Benzothiazol-2-yl;
4-(2-(4,5-Dihydro-1 H-imidazol-2-yl)ethyl)phenyl;
4-(3-Aminopropyl)phenyl;
4-(2-Aminoethyl)phenyl;
4-[1,4'-Bipiperidin]-1'yl-phenyl;
4-(2-(1,4,5,6-Tetrahydropyrimidin-2-yl)ethyl)phenyl;
4-(4,5-Dihydro-1H-imidazo-2-yl)phenyl;
4-Fluor-3-cyanophenyl; und
4-(2-Cyanoethyl)phenyl.

7. Eine Verbindung gemäß einer der Ansprüche 1 bis 5, wobei R⁴ ausgewählt wird aus:
4-(4-Pyridin-4-yl-piperazin-1-yl)phenyl,
4-(N,N-Diethylamino)phenyl,
4-(N,N-Dimethylamino)phenyl und
4-[1,4'-Bipiperidin]-1'-yl-phenyl.

8. Eine Verbindung gemäß einer der Ansprüche 1 bis 5, wobei R⁴ ausgewählt wird aus:
2-Ethoxyphenyl;
3-(2-Methylthiazol-5-yl)-pyrazol-5-yl;
Pyrazol-2-yl;
4-Aminophenylamino;
4-(1H-Imidazol-2yl-methyl)-phenylamino;
4-[2-(1H-Imidazol-2-yl)-ethyl]-phenylamino;
4-Aminomethyl-phenytamino;
4-(1H-Imidazol-2-yl)-phenylamino;
4-[N,N'-Diethylamidino]-phenylamino;
4-[N,N'-Dimethylamidino]-phenylamino;
4-[N,N'-Diphenylamidino]-phenylamino;
4-(4,5-Dihydro-1H-imidazol-2-yl-methyl)-phenylamino;
4-(1H-Benzimidazol-2-yl)-phenylamino;
4-[N-(4,5-Dihydro-1H-imidazol-2-yl)aminomethyl]-phenylamino;
4-(1H-Benzimidazol-2-yl-methyl)-phenylamino;
4-(1,4,5,6-Tetrahydro-pyrimidin-2-yl)-phenylamino; und
4-(1,4,5,6-Tetrahydro-pyrimidin-2-yl-methyl)-phenylamino.

9. Eine Verbindung gemäß einer der Ansprüche 1 bis 8, worin X ausgewählt wird aus:
Wasserstoff,
Brom,
Chlor,
Fluor und
Methyl.

10. Eine Verbindung gemäß Anspruch 1, ausgewählt aus den folgenden Verbindungen und pharmazeutisch zulässigen Salzen dieser:
(1) 4-Brom-5-(2-chlorbenzoylamino)-1H-pyrazol-3-carboxylsäure (4-diethylaminophenyl)amid;
(2) 4-Brom-5-(2-chlorbenzoylamino)-1H-pyrazol-3-carboxylsäure (4-dimethylaminophenyl)amid;
(3) 4-Brom-5-(2-chlorbenzoylamino)-1H-pyrazol-3-carboxylsäure o-tolylamid;
(4) 5-(2-chlorbenzoylamino)-4-methyl-1H-pyrazol-3-carboxylsäure phenylamid;
(5) 5-(2-chlorbenzoylamino)-4-ethyl-1H-pyrazol-3-carboxylsäure phenylamid;
(6) 5-(2-chlorbenzoylamino)-4-propyl-1H-pyrazol-3-carboxylsäure phenylamid;
(7) 4-Brom-5-(2-chlorbenzoylamino)-1H-pyrazol-3-carboxylsäure phenylamid;
(8) 4-Brom-5-(2-chlorbenzoylamino)-1H-pyrazol-3-carboxylsäure methylphenylamid;
(9) 4-Brom-5-(2-chlorbenzoylamino)-1H-pyrazol-3-carboxylsäure naphtalen-1-yl-amid;
(10) 4-Brom-5-(2-chlorbenzoylamino)-1H-pyrazol-3-carboxylsäure p-tolylamid;
(11) 4-Brom-5-(2-chlorbenzoylamino)-1H-pyrazol-3-carboxylsäure (4-chlorphenyl)amid;
(12) 4-Brom-5-(2-chlorbenzoylamino)-1H-pyrazol-3-carboxylsäure (3,4-dichlorphenyl)amid;
(13) 4-Brom-5-(2-chlorbenzoylamino)-1H-pyrazol-3-carboxylsäure (4-methoxyphenyl)amid;
(14) 4-Brom-5-(2-chlorbenzoylamino)-1H-pyrazol-3-carboxylsäure pyridin-3-yl-amid;
(15) 4-Brom-5-(2-chlorbenzoylamino)-1H-pyrazol-3-carboxylsäure pyridin-4-yl-amid;
(16) 4-Brom-5-(2-chlorbenzoylamino)-1H-pyrazol-3-carboxylsäure (2-chlorphenyl)amid;
(17) 4-Chlor-5-(2-chlorbenzoylamino)-1H-pyrazol-3-carboxylsäure phenylamid;
(18) 4-Brom-5-(2-chlorbenzoylamino)-1H-pyrazol-3-carboxylsäure (3-hydroxy-4-methoxyphenyl)amid;
(19) 4-Brom-5-(2-chlorbenzoylamino)-1H-pyrazol-3-carboxylsäure (6-methoxypyridin-3-yl)amid;
(20) 4-Brom-5-(2-chlorbenzoylamino)-1H-pyrazol-3-carboxylsäure (3,5-dimethoxyphenyl)amid;
(21) 4-Brom-5-(2-chlorbenzoylamino)-1H-pyrazol-3-carboxylsäure pyrazin-2-yl-amid;
(22) 4-Brom-5-(2-chlorbenzoylamino)-2H-pyrazol-3-carboxylsäure (4-ethylphenyl)amid;
(23) 4-Brom-5-(2-chlorbenzoylamino)-2H-pyrazol-3-carboxylsäure (4-sec-butylphenyl)amid;
(24) 4-Brom-5-(2-chlorbenzoylamino)-1H-pyrazol-3-carboxylsäure (1-methyl-1 H-pyrazol-3-yl)amid;
(25) 4-Brom-5-(2-chlorbenzoylamino)-1H-pyrazol-3-carboxylsäure (9H-fluoren-9-yl)amid;
(26) 4-Brom-5-(2-chlorbenzoylamino)-1H-pyrazol-3-carboxylsäure isochinolin-1-yl-amid;
(27) 4-Brom-5-(2-chlorbenzoylamino)-1H-pyrazol-3-carboxylsäure isochinolin-3-yl-amid;
(28) 4-Brom-5-(2-chlorbenzoylamino)-1H-pyrazol-3-carboxylsäure (4-phenylthiazol-2-yl)amid;
(29) 4-Brom-5-(2-chlorbenzoylamino)-1H-pyrazol-3-carboxylsäure [4-(4-pyridin-4-yl-piperazin-1-yl)phenyl]amid;
(30) 4-Brom-5-(2-chlorbenzoylamino)-1H-pyrazol-3-carboxylsäure (4-[1,4'-bipiperidin]-1'-yl-phenyl)amid;
(31) 4-Brom-5-(2-chlorbenzoylamino)-1H-pyrazol-3-carboxylsäure (1H-benzimidazol-2-yl)amid;
(32) 4-Brom-5-(2-chlorbenzoylamino)-1H-pyrazol-3-carboxylsäure benzothiazol-2-yl-amid;
(33) 4-Brom-5-(2-chlorbenzoylamino)-1H-pyrazol-3-carboxylsäure (4-(2-(4,5-dihydro-1 H-imidazol-2-yl)ethyl)phenyl)amid;
(34) 4-Brom-5-(2-chlorbenzoylamino)-1H-pyrazol-3-carboxylsäure (4-(3-aminopropyl)phenyl)amid;
(35) 4-Brom-5-(2-chlorbenzoylamino)-1H-pyrazol-3-carboxylsäure (4-2-aminoethyl)phenyl)amid;
(36) 4-Brom-5-(2-chlorbenzoylamino)-1H-pyrazol-3-carboxylsäure (4-[1,4'-bipiperidin]-1'-yl-phenyl)amid;
(37) 4-Brom-5-(2-chlorbenzoylamino)-1H-pyrazol-3-carboxylsäure (4-(2-(1,4,5,6-tetrahydropyrimidin-2-yl)ethyl)phenyl)amid;
(38) 4-Brom-5-(2-chlorbenzoylamino)-1H-pyrazol-3-carboxylsäure (4-(4,5-dihydro-1 H-imidazol-2-yl)phenyl)amid;
(39) 4-Brom-5-(2-chlorbenzoylamino)-1H-pyrazol-3-carboxylsäure (4-fluor-3-cyanophenyl)amid;
(40) 4-Brom-5-(2-chlorbenzoylamino)-1-methyl-pyrazol-3-carboxylsäure (4-[1,4'-bipiperidin]-1'-yl-phenyl)amid;
(41) 4-Brom-5-(2-chlorbenzoylamino)-1-methyl-pyrazol-3-carboxylsäure [4-(4-pyridin-4-yl-piperazin-1-yl)phenyl]amid;
(42) 4-Brom-5-(2-fluorbenzoylamino)-1-*t*-butyl-pyrazol-3-carboxylsäure (4-(2-cyanoethyl)phenyl)amid;
(43) 4-Brom-5-(2-fluorbenzoylamino)-1-*t*-butyl-pyrazol-3-carboxylsäure (4-(2-(4,5-dihydro-1 H-imidazol-2-yl)ethyl)phenyl)amid;
(44) 4-Brom-5-(2-fluorbenzoylamino)-1H-pyrazol-3-carboxylsäure (4-(2-(4,5-dihydro-1 H-imidazol-2-yl)ethyl)phenyl)amid;
(45) 4-Methyl-5-(2-fluorbenzoylamino)-1-phenyl-pyrazol-3-carboxylsäure (4-(2-(4,5-dihydro-1H-imidazol-2-yl)ethyl)phenyl)amid;
(46) 4-Methyl-5-(2-fluorbenzoylamino)-1-phenyl-pyrazol-3-carboxylsäure (4-(2-cyanoethyl)phenyl)amid;
(47) 4-Brom-5-(2-chlorbenzoylamino)-1-methyl-pyrazol-3-carboxylsäure (4-(3-aminopropyl)phenyl)amid; und
(48) 4-Brom-5-(2-chlorbenzoylamino)-1-methyl-pyrazol-3-carboxylsäure (4-(2-(4,5-dihydro-1H-imidazol-2-yl)ethyl)phenyl)amid.

11. Eine pharmazeutische Zusammensetzung, die aus einem pharmazeutisch zulässigen Träger und einer therapeutischen Menge einer Verbindung gemäß einer der Ansprüche 1 bis 10 besteht.

12. Eine pharmazeutische Zusammensetzung, die aus einem pharmazeutisch zulässigen Träger und einer therapeutisch wirksamen Menge einer Verbindung gemäß einer der Ansprüche 1 bis 10 oder einer Mischung davon besteht, und die wirksam ist zur Behandlung oder Linderung unerwünschter Symptome bei Säugetieren, die durch den Bradykinin-B₁-Rezeptor vermittelt werden.

13. Eine pharmazeutische Zusammensetzung, die aus einem pharmazeutisch zulässigen Träger und einer therapeutisch wirksamen Menge einer Verbindung gemäß einer der Ansprüche 1 bis 10 oder einer Mischung davon besteht, und die wirksam ist zur Behandlung oder Linderung unerwünschter Symptome bei Säugetieren, die mit der Hochregulation des Bradykinin-B₁-Rezeptors nach Gewebeschädigung oder Inflammation assoziiert sind.

14. Eine Methode zur selektiven Inhibition des Bradykinin-B₁-Rezeptors über den Bradykinin-B₂-Rezeptor, in vitro, wobei die Methode die Verwendung einer Verbindung gemäß einer der Ansprüche 1 bis 10 beinhaltet.

15. Eine Methode zur Bestimmung der Konzentrationen von Bradykinin-B₁-Rezeptor-Agonisten in einer biologischen Probe, in vitro, wobei die Methode die Verbindung der besagten biologischen Probe mit einer Verbindung gemäß einer der Ansprüche 1 bis 10, bei einer vorbestimmten Konzentration, beinhaltet.

16. Eine Verbindung gemäß einer der Ansprüche 1 bis 10, zur Anwendung bei einer Methode zur Behandlung des menschlichen oder tierischen Körpers mit einer Therapie.

17. Eine Verbindung gemäß einer der Ansprüche 1 bis 10 zur Anwendung bei einer Methode zur Behandlung oder Linderung von unerwünschten Symptomen bei Säugetieren, die durch den Bradykinin-B₁-Rezeptor vermittelt werden.

18. Anwendung einer Verbindung gemäß einer der Ansprüche 1 bis 10 zur Herstellung eines Medikaments zur Behandlung oder Linderung von unerwünschten Symptomen bei Säugetieren, die durch den Bradykinin-B₁-Rezeptor vermittelt werden.

19. Eine Verbindung gemäß einer der Ansprüche 1 bis 10 zur Anwendung bei einer Methode zur Behandlung oder Linderung unerwünschter Symptome bei Säugetieren, die mit Gewebeschädigung oder Inflammation assoziiert sind.

20. Anwendung einer Verbindung gemäß einer der Ansprüche 1 bis 10 zur Herstellung eines Medikaments zur Behandlung oder Linderung von unerwünschten Symptomen bei Säugetieren, die mit Gewebeschädigung oder Inflammation assoziiert sind.

21. Eine Verbindung gemäß einer der Ansprüche 1 bis 10 zur Anwendung bei einer Methode zur Behandlung oder Linderung von unerwünschten Symptomen, die mit der Anwesenheit oder Sekretion von Bradykinin-B₁-Rezeptor-Agonisten bei Säugetieren assoziiert sind.

22. Anwendung einer Verbindung gemäß einer der Ansprüche 1 bis 10 zur Herstellung eines Medikaments zur Behandlung oder Linderung von unerwünschten Symptomen, die mit der Anwesenheit oder Sekretion von Bradykinin-B₁-Rezeptor-Agonisten bei Säugetieren assoziiert sind.

23. Eine Verbindung gemäß einer der Ansprüche 1 bis 10 zur Anwendung bei einer Methode zur Behandlung oder Linderung von Schmerzen, Entzündungen, septischem Schock oder dem Vernarbungsprozess bei Säugetieren, die durch den Bradykinin-B₁-Rezeptor in diesen Säugetieren vermittelt werden.

24. Eine Verbindung gemäß einer der Ansprüche 1 bis 10 zur Herstellung eines Medikaments zur Behandlung oder Linderung von Schmerzen, Entzündungen, septischem Schock oder dem Vernarbungsprozess bei Säugetieren, die durch den Bradykinin-B₁-Rezeptor in diesen Säugetieren vermittelt werden.

25. Eine Verbindung gemäß einer der Ansprüche 1 bis 10 zur Anwendung bei einer Methode zur Behandlung oder Linderung von unerwünschten Symptomen, die mit Verbrennungen, perioperativen Schmerzen, Migräne, Schock, Verletzung des Zentralnervensystems, Asthma, Rhinitis, Frühwehen, inflammatorischer Arthritis, inflammatorischer Darmerkrankung oder neuropathischen Schmerzen assoziiert sind.

26. Eine Verbindung gemäß einer der Ansprüche 1 bis 10 zur Herstellung eines Medikaments zur Behandlung oder Linderung von unerwünschten Symptomen, die mit Verbrennungen, perioperativen Schmerzen, Migräne, Schock, Verletzung des Zentralnervensystems, Asthma, Rhinitis, Frühwehen, inflammatorischer Arthritis, inflammatorischer Darmerkrankung oder neuropathischen Schmerzen assoziiert sind.

## Revendications

1. Composé choisi parmi les composés de formule (1) et de formule (11) et les sels pharmaceutiquement acceptables de ceux-ci : 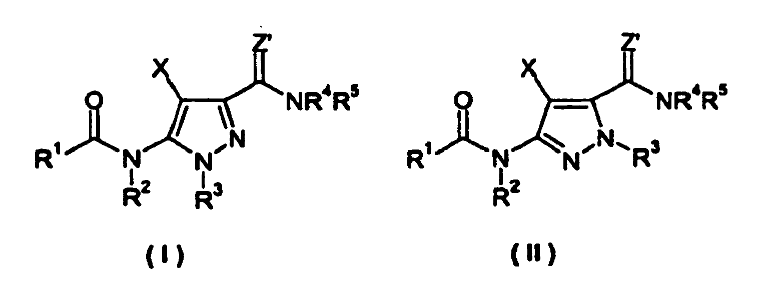 dans lequel :
Z' est choisi parmi : O, S et NH ;
R¹ est choisi parmi : un groupe 2-chlorophényle et 2-fluorophényle ;
R² est choisi parmi :
un atome d'hydrogène,
un groupe alkyle et alkyle substitué ;
R³ est choisi parmi ;
un atome d'hydrogène,
un groupe alkyle, alkyle substitué,
un groupe alcényle, alcényle substitué,
un groupe alcynyle, alcynyle substitué,
un groupe aryle, aryle substitué,
un groupe cycloalkyle, cycloalkyle substitué,
un groupe hétéroaryle, hétéroarylaryle substitué,
un groupe hétérocyclique et hétérocyclique substitué ;
R⁴ est choisi parmi ;
un groupe aryle, aryle substitué,
un groupe hétéroaryle et hétéroarylaryle substitué,
R⁵ est choisi parmi :
un atome d'hydrogène,
un groupe alkyle et alkyle substitué ;
X est choisi parmi :
un atome d'hydrogène,
un groupe alkyle, alkyle substitué,
un groupe alcoxy, alcoxy substitué,
un groupe aryle, aryle substitué,
un groupe carboxyle,
des groupes carboxylesters,
un groupe cyano,
un groupe halogéno,
un groupe hétéroaryle, hétéroarylaryle substitué,
un groupe hydroxy,
un groupe nitro,
un groupe amino, amino substitué,
un groupe acylamino, et
un groupe aminoacyle ;
à condition que le composé de formule (1) ne soit pas le phénylamide de l'acide 4-bromo-5-(2-chloro-benzoylamino)-1 H-pyrazole-3-carboxylique (A').

2. Composé selon la revendication 1, dans lequel R¹ est :
le 2-chlorophényle.

3. Composé selon la revendication 1 ou la revendication 2, dans lequel R² est choisi parmi :
un atome d'hydrogène,
un groupe méthyle,
un groupe éthyle,
un groupe isopropyle,
un groupe 2-méthoxyéth-1-yle,
un groupe pyrid-3-ylméthyle,
un groupe phényle,
un groupe benzyle,
un groupe t-butyle, et
un groupe t-butoxycarbonyl-méthyle.

4. Composé selon l'une quelconque des revendications 1 à 3, dans lequel R³ est choisi parmi :
un atome d'hydrogène,
un groupe alkyle en C₁-C₄,
un groupe aryle monocyclique, aryle monocyclique substitué,
un groupe hétéroaryle monocyclique et hétéroaryle monocyclique substitué.

5. Composé selon l'une quelconque des revendications 1 à 4, dans lequel R⁵ est choisi parmi :
un atome d'hydrogène,
un groupe méthyle,
un groupe éthyle,
un groupe isopropyle,
un groupe 2-méthoxyéthyle, et
un groupe pyrid-3-ylméthyle.

6. Composé selon l'une quelconque des revendications 1 à 5, dans lequel R⁴ est choisi parmi :
un groupe 4-(N,N-diéthylamino)phényle,
un groupe 4-(N,N-diméthylamino)phényle,
un groupe 2-méthylphényle,
un groupe phényle,
un groupe 1-naphtyle,
un groupe 4-méthylphényle,
un groupe 4-chlorophényle,
un groupe 3,4-dichlorophényle,
un groupe 4-méthoxyphényle,
un groupe pyridin-3-yle,
un groupe pyridin-4-yle,
un groupe 2-chlorophényle,
un groupe 4-méthoxy-3-hydroxyphényle,
un groupe 2-méthoxypyridin-5-yle,
un groupe 3,5-diméthoxyphényle,
un groupe pyrazin-2-yle,
un groupe 4-éthylphényle,
un groupe 4-(1-RorS)-1-méthylprop-1-yle),
un groupe 1-méthyl-1H-pyrazol-3-yle,
un groupe 9H-fluorèn-9-yle,
un groupe isoquinolin-1-yle,
un groupe isoquinolin-3-yle,
un groupe 4-phénylthiazol-2-yle,
un groupe 4-(4-pyridin-4-ylpipérazin-1-yl)phényle,
un groupe 4-[1,4'-bipéridin]-1'-ylphényle,
un groupe 1H-benzimidazol-2-yle,
un groupe benzothiazol-2-yle,
un groupe 4-(2-(4,5-dihydro-1 H-imidazol-2-yl)éthyl)phényle,
un groupe 4-(3-aminopropyl)phényle,
un groupe 4-(2-aminoéthyl)phényle,
un groupe 4-[1,4'-bipéridin]-1'-ylphényle,
un groupe 4-(2-(1,4,5,6-tétrahydropyrimidin-2-yl)éthyl)phényle,
un groupe 4-(4,5-dihydro-1H-imidazol-2-yl)phényle,
un groupe 4-fluoro-3-cyanophényle, et
un groupe 4-(2-cyanoéthyl)phényle.

7. Composé selon l'une quelconque des revendications 1 à 5, dans lequel R⁴ est choisi parmi :
un groupe 4-(4-pyridin-4-ylpipérazin-1-yl)phényle,
un groupe 4-(N,N-diéthylamino)phényle,
un groupe 4-(N,N-diméthylamino)phényle, et
un groupe 4-[1,4'-bipipéridin]-1'-yl-phényle.

8. Composé selon l'une quelconque des revendications 1 à 5, dans lequel R⁴ est choisi parmi :
un groupe 2-éthoxyphényle,
un groupe 3-(2-méthylthiazol-5-yl)-pyrazol-5-yle,
un groupe pyrazol-2-yle,
un groupe 4-aminophénylamino,
un groupe 4-(1H-imidazol-2-ylméthyl)-phénylamino,
un groupe 4-[2-(1H-imidazol-2-yl)-éthyl)-phénylamino,
un groupe 4-aminométhyl-phénylamino,
un groupe 4-(1H-imidazol-2-yl)-phénylamino,
un groupe 4-[N,N'-diéthylamidino]-phénylamino,
un groupe 4-[N,N'-diméthylamidino]-phénylamino,
un groupe 4-[N,N'-diphénylamidino]-phénylamino,
un groupe 4-(4,5-dihydro-1H-imidazol-2-ylméthyl)-phénylamino,
un groupe 4-(1H-benzimidazol-2-yl)-phénylamino,
un groupe 4-[N-(4,5-dihydro-1H-imidazol-2-yl)aminométhyl)-phénylamino,
un groupe 4-(1 H-benzimidazol-2-ylméthyl)-phénylamino,
un groupe 4-(1,4,5,6-tétrahydro-pyrimidin-2-yl)-phénylamino, et
un groupe 4-(1,4,5,6-tétrahydro-pyrimidin-2-ylméthyl)-phénylamino.

9. Composé selon l'une quelconque des revendications 1 à 8, dans lequel X est choisi parmi :
un atome d'hydrogène,
un atome de brome,
un atome de chlore,
un atome de fluor, et
un groupe méthyle.

10. Composé selon la revendication 1, choisi parmi les composés suivants et les sels pharmaceutiquement acceptables de ceux-ci ;
(1) le (4-diéthylaminophényl)amide de l'acide 4-bromo-5-(2-chlorobenzoylamino)-1H-pyrazole-3-carboxylique ;
(2) le (4-diméthylaminophényl)amide de l'acide 4-bromo-5-(2-chlorobenzoylamino)-1H-pyrazole-3-carboxylique ;
(3) l'o-tolylamide de l'acide 4-bromo-5-(2-chlorobenzoylamino)-1 H-pyrazole-3-carboxylique ;
(4) le phénylamide de l'acide 5-(2-chlorobenzoylamino)-4-méthyl-1 H-pyrazole-3-carboxylique ;
(5) le phénylamide de l'acide 5-(2-chlorobenzoylamino)-4-éthyl-1 H-pyrazole-3-carboxylique ;
(6) le phénylamide de l'acide 5-(2-chlorobenzoylamino)-4-propyl-1 H-pyrazole-3-carboxylique ;
(7) le phénylamide de l'acide 4-bromo-5-(2-chlorobenzoylamino)-1 H-pyrazole-3-carboxylique ;
(8) le méthylphénylamide de l'acide 4-bromo-5-(2-chlorobenzoylamino)-1H-pyrazole-3-carboxylique ;
(9) le naphtalène-1-ylamide de l'acide 4-bromo-5-(2-chlorobenzoylamino)-1H-pyrazole-3-carboxylique ;
(10) le p-tolylamide de l'acide 4-bromo-5-(2-chlorobenzoylamino)-1 H-pyrazole-3-carboxylique ;
(11) le (4-chlorophényl)amide de l'acide 4-bromo-5-(2-chlorobenzoylamino)-1H-pyrazole-3-carboxylique ;
(12) le (3,4-dichlorophényl)amide de l'acide 4-bromo-5-(2-chlorobenzoylamino)-1H-pyrazole-3-carboxylique ;
(13) le (4-méthoxyphényl)amide de l'acide 4-bromo-5-(2-chlorobenzoylamino)-1H-pyrazole-3-carboxylique ;
(14) le pyridine-3-ylamide de l'acide 4-bromo-5-(2-chlorobenzoylamino)-1H-pyrazole-3-carboxylique ;
(15) le pyridine-4-ylamide de l'acide 4-bromo-5-(2-chlorobenzoylamino)-1H-pyrazole-3-carboxylique ;
(16) le (2-chlorophényl)amide de l'acide 4-bromo-5-(2-chlorobenzoylamino)-1H-pyrazole-3-carboxylique ;
(17) le phénylamide de l'acide 4-chloro-5-(2-chlorobenzoylamino)-1 H-pyrazole-3-carboxylique ;
(18) le (3-hydroxy-4-méthoxyphényl)amide de l'acide 4-bromo-5-(2-chlorobenzoylamino)-1H-pyrazole-3-carboxylique ;
(19) le (6-méthoxypyridin-3-yl)amide de l'acide 4-bromo-5-(2-chlorobenzoylamino)-1H-pyrazole-3-carboxylique ;
(20) le (3,5-diméthoxyphényl)amide de l'acide 4-bromo-5-(2-chlorobenzoylamino)-1H-pyrazole-3-carboxylique ;
(21) le pyrazin-2-ylamide de l'acide 4-bromo-5-(2-chlorobenzoylamino)-1H-pyrazole-3-carboxylique ;
(22) le (4-éthylphényl)amide de l'acide 4-bromo-5-(2-chlorobenzoylamino)-2H-pyrazole-3-carboxylique ;
(23) le (4-sec-butylphényl)amide de l'acide 4-bromo-5-(2-chlorobenzoylamino)-2H-pyrazole-3-carboxylique ;
(24) le (1-méthyl-1H-pyrazol-3-yl)amide de l'acide 4-bromo-5-(2-chlorobenzoylamino)-1H-pyrazole-3-carboxylique ;
(25) le (9H-fluorèn-9-yl)amide de l'acide 4-bromo-5-(2-chlorobenzoylamino)-1H-pyrazole-3-carboxylique ;
(26) l'isoquinolin-1-ylamide de l'acide 4-bromo-5-(2-chlorobenzoylamino)-1H-pyrazole-3-carboxylique ;
(27) l'isoquinolin-3-ylamide de l'acide 4-bromo-5-(2-chlorobenzoylamino)-1H-pyrazole-3-carboxylique ;
(28) le (4-phénylthiazol-2-yl)amide de l'acide 4-bromo-5-(2-chlorobenzoylamino)-1H-pyrazole-3-carboxylique ;
(29) le [4-(4-pyridin-4-ylpipérazin-1-yl)phényl]amide de l'acide 4-bromo-5-(2-chlorobenzoylamino)-1H-pyrazole-3-carboxylique ;
(30) le [4-(1,4'-bipipéridin]-1'-ylphényl)amide de l'acide 4-bromo-5-(2-chlorobenzoylamino)-1H-pyrazole-3-carboxylique ;
(31) le (1 H-benzimidazol-2-yl)amide de l'acide 4-bromo-5-(2-chlorobenzoylamino)-1H-pyrazole-3-carboxylique ;
(32) le benzothiazol-2-ylamide de l'acide 4-bromo-5-(2-chlorobenzoylamino)-1H-pyrazole-3-carboxylique ;
(33) le (4-(2-(4,5-dihydro-1H-imidazol-2-yl)éthyl)phényl)amide de l'acide 4-bromo-5-(2-chlorobenzoylamino)-1 H-pyrazole-3-carboxylique ;
(34) le (4-(3-aminopropyl)phényl)amide de l'acide 4-bromo-5-(2-chlorobenzoylamino)-1H-pyrazole-3-carboxylique ;
(35) le (4-(2-aminoéthyl)phényl)amide de l'acide 4-bromo-5-(2-chlorobenzoylamino)-1H-pyrazole-3-carboxylique ;
(36) le (4-[1,4'-bipipéridin]-1'-ylphényl)amide de l'acide 4-bromo-5-(2-chlorobenzoylamino)-1H-pyrazole-3-carboxylique ;
(37) le (4-(2-(1,4,5,6-tétrahydropyrimidin-2-yl)éthyl)phényl)amide de l'acide 4-bromo-5-(2-chlorobenzoylamino)-1H-pyrazole-3-carboxylique ;
(38) le (4-(4,5-dihydro-1H-imidazol-2-yl)phényl)amide de l'acide 4-bromo-5-(2-chlorobenzoylamino)-1H-pyrazole-3-carboxylique ;
(39) le (4-fluoro-3-cyano-phényl)amide de l'acide 4-bromo-5-(2-chlorobenzoylamino)-1H-pyrazole-3-carboxylique ;
(40) le (4-[1,4'-bipipéridin]-1'-yl-phényl)amide de l'acide 4-bromo-5-(2-chlorobenzoylamino)-1-méthyl-pyrazole-3-carboxylique ;
(41) le [4-(4-pyridin-4-yl-pipérazin-1-yl)phényl]amide de l'acide 4-bromo-5-(2-chlorobenzoylamino)-1-méthyl-pyrazole-3-carboxylique ;
(42) le (4-(2-cyanoéthyl)phényl)amide de l'acide 4-méthyl-5-(2-fluorobenzoylamino)-1-t-butyl-pyrazole-3-carboxylique ;
(43) le (4-(2-(4,5-dihydro-1H-imidazol-2-yl)éthyl)phényl)amide de l'acide 4-méthyl-5-(2-fluorobenzoylamino)-1-t-butyl-pyrazole-3-carboxylique ;
(44) le (4-(2-(4,5-dihydro-1H-imidazol-2-yl)éthyl)phényl)amide de l'acide 4-méthyl-5-(2-fluorobenzoylamino)-1H-pyrazole-3-carboxylique ;
(45) le (4-(2-(4,5-dihydro-1H-imidazol-2-yl)éthyl)phényl)amide de l'acide 4-méthyl-5-(2-fluorobenzoylamino)-1-phényl-pyrazole-3-carboxylique ;
(46) le (4-(2-cyanoéthyl)phényl)amide de l'acide 4-méthyl-5-(2-fluorobenzoylamino)-1-phényl-pyrazole-3-carboxylique ;
(47) le (4-(3-aminopropyl)phényl)amide de l'acide 4-bromo-5-(2-chlorobenzoylamino)-1-méthyl-pyrazole-3-carboxylique ; et
(48) le (4-(2-(4,5-dihydro-1H-imidazol-2-yl)éthyl)phényl)amide de l'acide 4-bromo-5-(2-chlorobenzoylamino)-1-méthyl-pyrazole-3-carboxylique.

11. Composition pharmaceutique comprenant un véhicule pharmaceutiquement acceptable et une quantité efficace au plan thérapeutique d'un composé selon l'une quelconque des revendications 1 à 10.

12. Composition pharmaceutique comprenant un véhicule pharmaceutiquement acceptable et une quantité efficace au plan thérapeutique d'un composé selon l'une quelconque des revendications 1 à 10, ou un mélange de ceux-ci, efficace pour traiter ou atténuer des symptômes préjudiciables chez des mammifères facilités par un récepteur de la bradykinine B₁.

13. Composition pharmaceutique comprenant un véhicule pharmaceutiquement acceptable et une quantité efficace au plan thérapeutique d'un composé selon l'une quelconque des revendications 1 à 10, ou un mélange de ceux-ci, efficace pour traiter ou atténuer des symptômes préjudiciables chez des mammifères associés à la régulation d'un récepteur de la bradykinine B₁ après une lésion ou une inflammation tissulaire.

14. Procédé d'inhibition sélective d'un récepteur de la bradykinine B₁ par rapport à un récepteur de la bradykinine B₂, in vitro, procédé qui comprend l'utilisation d'un composé selon l'une quelconque des revendications 1 à 10.

15. Procédé de détermination de niveaux d'agonistes d'un récepteur de la bradykinine B₁ dans un échantillon biologique, in vitro, procédé qui comprend la mise en contact dudit échantillon biologique avec un composé selon l'une quelconque des revendications 1 à 10, dans une concentration prédéterminée.

16. Composé selon l'une quelconque des revendications 1 à 10, à utiliser dans un procédé de traitement d'un corps humain ou animal par une thérapie.

17. Composé selon l'une quelconque des revendications 1 à 10, à utiliser dans un procédé de traitement ou d'atténuation de symptômes préjudiciables chez des mammifères facilités par un récepteur de la bradykinine B₁.

18. Utilisation d'un composé selon l'une quelconque des revendications 1 à 10, dans la fabrication d'un médicament pour traiter ou atténuer des symptômes préjudiciables chez des mammifères facilités par un récepteur de la bradykinine B₁.

19. Composé selon l'une quelconque des revendications 1 à 10, à utiliser dans un procédé de traitement ou d'atténuation de symptômes préjudiciables chez des mammifères associés à une lésion ou à une inflammation tissulaire.

20. Utilisation d'un composé selon l'une quelconque des revendications 1 à 10, dans la fabrication d'un médicament pour traiter ou atténuer des symptômes préjudiciables chez des mammifères associés à une lésion ou à une inflammation tissulaire.

21. Composé selon l'une quelconque des revendications 1 à 10, à utiliser dans un procédé de traitement ou d'atténuation de symptômes préjudiciables associés à la présence ou à la sécrétion d'agonistes d'un récepteur de la bradykinine B₁, chez des mammifères.

22. Utilisation d'un composé selon l'une quelconque des revendications 1 à 10, dans la fabrication d'un médicament pour traiter ou atténuer des symptômes préjudiciables associés à la présence ou à la sécrétion d'agonistes d'un récepteur de la bradykinine B₁, chez des mammifères.

23. Composé selon l'une quelconque des revendications 1 à 10, à utiliser dans un procédé de traitement ou d'amélioration d'une douleur, d'une inflammation, d'un choc septique ou du processus de cicatrisation chez des mammifères facilité par un récepteur de la bradykinine B₁ chez ces mammifères.

24. Utilisation d'un composé selon l'une quelconque des revendications 1 à 10, dans la fabrication d'un médicament pour traiter ou améliorer une douleur, une inflammation, un choc septique ou le processus de cicatrisation chez des mammifères facilité par un récepteur de la bradykinine B₁ chez ces mammifères.

25. Composé selon l'une quelconque des revendications 1 à 10, à utiliser dans un procédé de traitement ou d'atténuation de symptômes préjudiciables associés à des brûlures, une douleur périopératoire, la migraine, un choc, une lésion du système nerveux central, l'asthme, la rhinite, le travail prématuré, l'arthrite inflammatoire, une infection intestinale inflammatoire ou une douleur neuropathique.

26. Utilisation d'un composé selon l'une quelconque des revendications 1 à 10, dans la fabrication d'un médicament pour traiter ou améliorer des symptômes préjudiciables associés à des brûlures, une douleur périopératoire, la migraine, un choc, une lésion du système nerveux central, l'asthme, la rhinite, le travail prématuré, l'arthrite inflammatoire, une infection intestinale inflammatoire ou une douleur neuropathique.
